# EUROPEAN PATENT SPECIFICATION

(11) **EP 0 637 237 B1**
(45) Date of publication and mention of the grant of the patent: **19.05.2004**
(21) Application number: 93907015.7
(22) Date of filing: 23.02.1993
(51) Int. Cl.: A61K 31/00, A61K 31/13, A61K 31/135, A61K 31/405, C12N 15/12, C12N 15/11, C12N 5/10, A01K 67/027

(54) **CALCIUM RECEPTOR-ACTIVE MOLECULES**
CALCIUMREZEPTORAKTIVE MOLEKÜLE
MOLECULES ACTIVES SUR LES RECEPTEURS DU CALCIUM

(43) Date of publication of application: 08.02.1995
(60) Divisional application: 03012225.3
(73) Proprietor: BRIGHAM AND WOMEN'S HOSPITAL, INC., Boston, MA 02115 (US); NPS PHARMACEUTICALS, INC., Salt Lake City Utah 84108 (US)
(72) Inventor: NEMETH, Edward, F., Salt Lake City, UT 84108 (US); BROWN, Edward, M., Milton, MA 02186 (US); HEBERT, Steven, C., Wellesley, MA 02187 (US); VAN WAGENEN, Bradford, C., Salt Lake City, UT 84102 (US); BALANDRIN, Manuel, F., Salt Lake City, UT 84121 (US); FULLER, Forrest, H., Salt Lake City, UT 84103 (US); DEL MAR, Eric, G., Salt Lake City, UT 84108 (US)
(74) Representative: Jaenichen, Hans-Rainer, Dr.
(86) International application number: PCT/US1993/001642
(87) International publication number: WO 1994/018959

(56) References cited:
- EP-A- 0 253 327
- EP-A- 0 395 357
- WO-A-91/00853
- WO-A-92/14709
- WO-A-93/04373
- GB-A- 2 113 089
- BIOSCIENCE REP. vol. 10, no. 6 , 1990 pages 493 - 507 M. ZAIDI '"Calcium receptors" on eukaryotic cells with special reference to the osteoclast.' cited in the application
- CELL CALCIUM vol. 11, no. 5 , 1990 pages 319 - 321 E.F. NEMETH ET AL. 'The role of extracellular calcium in the regulation of intracellular calcium and cell function.' cited in the application
- CELL CALCIUM vol. 11, no. 5 , 1990 pages 323 - 327 E.F. NEMETH 'Regulation of cytosolic calcium by extracellular divalent cations in C-cells and parathyroid cells.' cited in the application
- NORMAN, VANAMAN, MEANS , EDS. 'calcium-binding proteins in health and disease' 1987 , ACADEMIC PRESS , SAN DIEGO Evidence for the presence of a novel calcium-binding protein calcium receptor on the surface of parathyroid cells. Auth. E.F. Nemeth see page 36 - page 38

## Description

### Field of the Invention

This invention relates to the design, development, composition and use of novel calcimimetic molecules able to act in a manner analogous to extracellular calcium ions on cells, to calcilytic molecules which block the activity of extracellular calcium ions on cells, and to methods for their use and identification.

It also relates to a novel superfamily of inorganic-ion receptors which includes, among others, calcium receptors, nucleic acids encoding such receptors, cells, tissues and animals containing such nucleic acids, antibodies to receptors, assays utilizing receptors, and methods relating to all of the foregoing.

### Background of the Invention

The following description provides a summary of information relevant to the present invention. It is not an admission that any of the information provided herein is prior art to the presently claimed invention, nor that any of the publications specifically or implicitly referenced are prior art to that invention.

Certain cells in the body respond not only to chemical signals, but also to ions such as extracellular calcium ions (Ca²⁺). Changes in the concentration of extracellular Ca²⁺ (referred to herein as "[Ca²⁺]") alter the functional responses of these cells. One such specialized cell is the parathyroid cell which secretes parathyroid hormone (PTH). PTH is the principal endocrine factor regulating Ca²⁺ homeostasis in the blood and extracellular fluids.

PTH, by acting on bone and kidney cells, increases the level of Ca²⁺ in the blood. This increase in [Ca²⁺] then acts as a negative feedback signal,depressing PTH secretion. The reciprocal relationship between [Ca²⁺] and PTH secretion forms the essential mechanism maintaining bodily Ca²⁺ homeostasis.

Extracellular Ca²⁺ acts directly on the parathy roid cell to regulate PTH secretion. The existence of a parathyroid cell surface protein which detects changes in [Ca²⁺] has been suggested. This protein acts as a receptor for extracellular Ca²⁺ ("the Ca²⁺ receptor"), and is suggested to detect changes in [Ca²⁺] and to initiate a functional cellular response, PTH secretion. For example, the role of Ca²⁺ receptors and extracellular Ca²⁺ in the regulation of intracellular Ca²⁺ and cell function is reviewed in Nemeth et al., 11 Cell Calcium 319, 1990; the role of Ca²⁺ receptors in parafollicular and parathyroid cells is discussed in Nemeth, 11 Cell Calcium 323, 1990; and the role of Ca²⁺ receptors on bone osteoclasts is discussed by Zaidi, 10 Bioscience Reports 493, 1990.

Other cells in the body, specifically the osteoclast in bone, the juxtaglomerular, proximal tubule cells in the kidney, the keratinocyte in the epidermis, the parafollicular cell in the thyroid, intestinal cells the trophoblast in the placenta, have the capacity to sense changes in [Ca²⁺]. It has been suggested that cell surface Ca²⁺ receptors may also be present on these cells, imparting to them the ability to detect and to initiate or enable a response to changes in [Ca²⁺].

In parathyroid cells, osteoclasts, parafollicular cells (C-cells), keratinocytes, juxtaglomerular cells and trophoblasts, an increase in [Ca²⁺] evokes an increase in intracellular free Ca²⁺ concentration ("[Ca²⁺]ᵢ"). Such an increase may be caused by influx of extracellular Ca²⁺ or by mobilization of Ca²⁺ from intracellular organelles. Changes in [Ca²⁺]ᵢ are readily monitored and quantitated using fluorimetric indicators such as fura-2 or indo-1 (Molecular Probes, Eugene, OR). Measurement of [Ca²⁺]ᵢ provides an assay to assess the ability of molecules to act as agonists or antagonists at the Ca²⁺ receptor.

In parathyroid cells, increases in the concentration of extracellular Ca²⁺ evoke rapid and transient increases in [Ca²⁺]ᵢ which are followed by lower yet sustained increases in [Ca²⁺]ᵢ. The transient increases in [Ca²⁺]ᵢ arise from the mobilization of intracellular Ca²⁺, whereas the lower, sustained increases result from the influx of extracellular Ca²⁺. The mobilization of intracellular Ca²⁺ is accompanied by increased formation of inositol-1,4,5-trisphosphate (IP₃) and diacylglycerol, two biochemical indicators which are associated with receptor-dependent mobilization of intracellular Ca²⁺ in various other cells.

In addition to Ca²⁺, various other di- and trivalent cations, such as Mg²⁺, Sr²⁺, Ba²⁺, La³⁺, and Gd³⁺ also cause the mobilization of intracellular Ca²⁺ in parathyroid cells. Mg²⁺ and La³⁺ also increase the forma tion of IP₃; all these inorganic cations depress the secretion of PTH. The postulated Ca²⁺ receptor on the parathyroid cell is therefore promiscuous because it detects a variety of extracellular di- and trivalent cations.

The ability of various compounds to mimic extracellular Ca²⁺ in vitro is discussed by Nemeth et al., (spermine and spermidine) in "Calcium-Binding Proteins in Health and Disease", 1987, Academic Press, Inc., pp. 33-35; Brown et al., (e.g., neomycin) 128 Endocrinology 3047, 1991; Chen et al., (diltiazem and its analog, TA-3090) 5 J. Bone and Mineral Res. 581, 1990; and Zaidi et al., (verapamil) 167 Biochem Biophys Res Comm 807, 1990.

Brown et al., 6 J. Bone and Mineral Res. 11, 1991 discuss the existing theories regarding the effects of Ca²⁺ ions on parathyroid cells, and propose that the results may be explained by both a receptor-like mechanism and a receptor-independent mechanism as follows:
Polyvalent cations [e.g., divalent and trivalent cations] exert a variety of effects on parathyroid function, such as inhibition of parathyroid hormone (PTH) secretion and cAMP accumulation, stimulation of the accumulation of inositol phosphates, and elevation of the cytosolic calcium concentration. These actions are thought to be mediated through a "receptor-like" mechanism. The inhibition of agonist-stimulated cAMP accumulation by divalent and trivalent cations, for example, is blocked following preincubation with pertussis toxin. Thus, the putative polyvalent cation receptor may be coupled to inhibition of adenylate cyclase by the inhibitory guanine nucleotide regulatory (G) protein, Gᵢ.
We recently showed that the polycationic antibiotic, neomycin, mimics the actions of di- and trivalent cations in several aspects of parathy roid function. To determine whether these actions were specific to this agent or represented a more generalized action of polycations, we tested the effects of the highly basic peptides, polyarginine and polylysine, as well as protamine on the same parameters in dispersed bovine parathyroid cells. The results demonstrate that the parathyroid cell responds to a variety of polycations as well as to polyvalent cations, potentially via similar biochemical pathways. These results are discussed in terms of the recently postulated, "receptor-independent" modulation of G proteins by polycations in other systems.
The Ca²⁺ receptor has been presumed to be analogous to other G protein-coupled receptors [e.g., a glycoprotein], but recent studies with other cell types have raised the possibility that polycations can modulate cell function by alternative or additional mechanisms. In mast cells, for example, a variety of amphipathic cations, including mastoparan, a peptide from wasp venom, 48/80, a synthetic polycation, and polylysine, enhance secretion by a pertussis toxin-sensitive mechanism, suggesting the involvement of a G protein. No classic cell surface receptor has been identified that could mediate the actions of these diverse agents. Furthermore, these same compounds have been shown to activate directly purified G proteins in solution or in artificial phospholipid vesicles. On the basis of these observations, it has been proposed that amphipathic cations activate G proteins and, in turn, mast cell secretion by a "receptor-independent" mechanism.
Polycations have also been shown to interact strongly with acidic phospholipids. Polylysines of varying chain lengths (20-1000 amino acids) bind to artificial phospholipid vesi cles with dissociation constants in the range of 0.5 nM to 1.5 µM. The binding affinity is directly related to the length of the polylysine chain, with polymers of 1000 amino acids having a K_{d} of 0.5 nM, shorter polymers having higher Kd values, and lysine not interacting to a significant extent. This relationship between potency and chain length is similar to that observed for the effects of polylysine 10,200, polylysine 3800, and lysine on parathyroid function.
It is possible that the binding of polycations to biomembranes produces some of their biologic actions. The permeabilization of the plasma membrane induced in some cell types by a variety of pore-forming agents, including polycations, has been postulated to be mediated by their interaction with a phosph atidylserine-like structure. In addition, the "receptor-independent" activation of purified G proteins by amphipathic cations is potentiated when these proteins are incorporated into phospholipid vesicles.
Calcium ions, in the millimolar concentration range, also produce marked changes in membrane structure. In some cases, calcium can either antagonize or potentiate the interaction of polycations with membrane lipids. These considerations raise the possibility that the actions of both polyvalent cations and polycations on parathyroid cells could involve a receptor-independent mechanism not requiring the presence of a classic, cell surface, G protein-coupled receptor. Further studies, however, are required to elucidate the molecular basis for Ca²⁺ sensing by this and other cell types. [Citations omitted.]

Shoback and Chen (6 (Supplement 1), J. Bone and Mineral Res. 1991, S135) and Racke et al. (6 (Supplement 1), J. Bone and Mineral Res. 1991, S118) describe experiments which are said to indicate that a Ca²⁺ receptor or Ca²⁺ sensor is present in parathyroid cells. Messenger RNA isolated from such cells can be expressed in oocytes and caused to provide those oocytes with a phenotype which might be explained by the presence of a Ca²⁺ receptor protein.

### Summary of the Invention

Applicant has demonstrated that Ca²⁺ receptor proteins enable certain specialized cells involved in bodily Ca²⁺ metabolism to detect and respond to changes in the concentration of extracellular Ca²⁺. Although these receptors share certain general characteristics, they can be selectively affected by different pharmacological agents. As detailed below, certain molecules are identified with selective activity on Ca²⁺ receptors at parathyroid cells, osteoclasts, and C-cells.

Ca²⁺ receptors constitute discrete molecular targets for a new class of molecules that mimic ("calcimimetics") or antagonize ("calcilytics") the actions of extracellular Ca²⁺. Such receptors are present on cell surfaces and have a low affinity for extracellular Ca²⁺ (apparent K_{d} generally greater than about 0.5 mM). Such receptors may include a free or bound effector mechanism, as defined by Cooper, Bloom and Roth, "The Biochemical Basis of Neuropharmacology", Ch. 4. Such receptors are thus distinct from intracellular Ca²⁺ receptors, e.g., calmodulin and the troponins. Calcimimetics, for example, act on Ca²⁺ receptors selectively to directly or indirectly depress the function of parathyroid cells or osteoclasts or to stimulate the function of C-cells. Calcimimetics and calcilytics of this invention allow novel therapies for hyperparathyroidism, osteoporosis and other Ca²⁺-related diseases. This application concerns in one aspect targeting Ca²⁺ receptors on each of these three cell types and other cell types that detect and respond to changes in [Ca²⁺].

Applicant is the first to demonstrate a Ca²⁺ receptor protein in parathyroid cells, and to pharmacologically differentiate such Ca²⁺ receptors in other cells, such as C-cells and osteoclasts. Applicant is also the first to describe methods by which molecules active at these Ca²⁺ receptors can be identified and used as lead molecules in the discovery, development, design, modification and/or construction of useful calcimimetics or calcilytics which are active at Ca²⁺ receptors. Such calcimimetics or calcilytics are useful in the treatment of various disease states characterized by abnormal levels of one or more components, e.g., polypeptides such as hormones, enzymes or growth factors, the expression and/or secretion of which is regulated or affected by activity at one or more Ca²⁺ receptors. Further, the identification of different Ca²⁺ receptors in different cell types, and the specific response of such receptors to different lead molecules allows design and construction of specific molecules active in treatment of specific diseases which can be affected by action at such specific Ca²⁺ receptors. For example, abnormal levels of parathyroid hormone secretion can be affected by such specific molecules without affecting the level of secretion of other Ca²⁺ regulated hormones and the like.

Identification of such lead molecules was impeded by the prior lack of a high-throughput screening system to discover active molecules, and the absence of a structural data base upon which to design effective drug candidates. These barriers are now removed by cloning the parathyroid cell Ca²⁺ receptor and functionally related receptors, and systematically examining the structural features of certain lead molecules that activate such cloned Ca²⁺ receptors and functionally related receptors. Cloning of the Ca²⁺ receptor also enables development of transfected cell lines suitable for high-throughput screening of natural product or molecule libraries and synthetic molecules. This, together with structure-activity studies discussed below, provides the technology necessary to develop novel calcimimetics and calcilytics.

Applicant enables such procedures in this application. A bovine parathyroid cell calcium receptor cDNA has been cloned and is deposited in the ATCC under accession number ATCC 75416. Using this clone, inorganic-ion receptors in other tissues and species homologs are easily obtained. For example, the human parathyroid cell Ca²⁺ receptor cDNA can be cloned by screening nucleic acid libraries or by screening for functional expression in Xenopus oocytes, and the structural features of organic molecules necessary for activity on the Ca²⁺ receptor can be determined through the testing of selected natural products or other molecule libraries and subsequent structure-activity studies.

Thus, in a first aspect, the invention features a nucleic acid molecule encoding a calcium receptor polypeptide selected from the group consisting of:
(a) nucleic acid molecules comprising the nucleotide sequence as depicted in SEQ ID NO. 1;
(b) nucleic acid molecules comprising the nucleic acid sequence of the cDNA insert contained in ATCC 75416; and
(c) nucleic acid molecules which are able to hybridize to a nucleic acid molecule complementary to a molecule of (a) or (b) and which encode a functional calcium receptor.

Such nucleic acid molecules and the encoded receptor polypeptides are useful for identifying a molecule which either mimics the activity of extracellular Ca²⁺ by evoking an increase in [Ca²⁺]ᵢ in a cell, or blocks an increase in [Ca²⁺]ᵢ elicited by extracellular Ca²⁺. The molecule has an EC₅₀ of less than or equal to 5 µM, and is not protamine.

By "mimic" is meant that the molecule has one or more of the specific actions of extracellular Ca²⁺ on an extracellular Ca²⁺ responsive cell. The term does not require that all of the biological functions of extracellular Ca²⁺ are mimicked, but rather that at least one such function is mimicked. In addition it does not require that the molecule bind to the same site on the Ca²⁺ receptor as does extracellular Ca²⁺ (see for example, the novel compound NPS 467 and its action in Example 20 below). By "block" is meant that one such action of Ca²⁺ is reduced or prevented by the molecule. The EC₅₀ can be determined in assays as described below, where the activity mimicked is measured and the concentration of molecule which mimics at half the maximum mimicking effect is the EC₅₀. Conversely, the IC₅₀ of a calcilytic is that amount which blocks half maximal activity. Preferably, such assays measure [Ca²⁺]ᵢ increases and are confirmed to be specific to a Ca²⁺ receptor by methods described below, or their equivalent.

In preferred embodiments, bioassays described herein demonstrate that the increase in [Ca²⁺]ᵢ in a cell is transient, having a duration of less than one minute, and the increase in [Ca²⁺]ᵢ is rapid, occurring within thirty seconds; and the molecule also (a) evokes a sus tained increase (greater than thirty seconds) in [Ca²⁺]ᵢ, (b) evokes an increase in inositol-1,4,5-trisphosphate and/or diacylglycerol levels, e.g., within less than 60 seconds, and (c) inhibits dopamine- or isoproterenol- stimulated cyclic AMP formation. In addition, the tran sient increase in [Ca²⁺]ᵢ is abolished by pretreatment of the cell for ten minutes with 10 mM sodium fluoride, or the transient increase is diminished by brief pretreatment (not more than ten minutes) of the cell with an activator of protein kinase C, e.g., phorbol myristate acetate (PMA), mezerein or (-)indolactam V.

In a parathyroid cell, those molecules which are active in all of the assays described above are particularly useful since they are specific in their actions to a Ca²⁺ receptor of such a cell. This is particularly true for the PMA pretreatment effect described above.

In a more preferred embodiment, the cell is a parathyroid cell, and the molecule inhibits parathyroid hormone secretion from the cell. Other preferred embodiments include molecules that elicit an increase in [Ca²⁺]ᵢ as detected, for example, as an increase in Cl⁻ current in a Xenopus oocyte injected with mRNA from a parathyroid cell, bone osteoclast, juxtaglomerular kidney cell, proximal tubule kidney cell, distal tubule kidney cell, cell of the thick ascending limb of Henle's loop and/or collecting duct, keratinocyte in the epidermis, parafollicular cell in the thyroid (C-cells), intestinal cell, trophoblast in the placenta, platelet, vascular smooth muscle cell, cardiac atrial cell, gastrin and glucagon secreting cells, kidney mesangial cell and mammary cell.

In other preferred embodiments, the molecule evokes the mobilization of intracellular Ca²⁺ to cause the increase in [Ca²⁺]ᵢ; the cell is a C-cell or an osteoclast and the molecule inhibits bone resorption in vivo; the cell is an osteoclast and the molecule inhibits bone resorption in vitro; or the cell is a C- cell and the molecule stimulates calcitonin secretion in vitro or in vivo; and most preferably the molecule is either a calcimimetic or calcilytic having an EC₅₀ or IC₅₀ at a Ca²⁺ receptor of less than or equal to 5 µM, and even more preferably less than or equal to 1 µM, 100 nmolar, 10 nmolar, or 1 nmolar. Such lower EC₅₀'s or IC₅₀'s are advantageous since they allow lower concentration of molecules to be used in vivo or in vitro for therapy or diagnosis. The discovery of molecules with such low EC₅₀'s and IC₅₀'s enables the design and synthesis of similarly potent and efficacious molecules.

By "calcimimetic" molecule is meant any molecule which has one or more activities of extracellular Ca²⁺, and preferably mimics the activity of Ca²⁺ at a Ca²⁺ receptor. For example, when used in reference to a parathyroid cell it is a molecule which, when tested on parathyroid cells in vitro, possesses one or more, and preferably all of the following characteristics as measured by techniques well known to those in the art:
1. The molecule causes a rapid (time to peak < 5 sec) and transient increase in [Ca²⁺]ᵢ that is refractory to inhibition by 1 µM La³⁺ or Gd³⁺. The increase in [Ca²⁺]ᵢ persists in the absence of extra cellular Ca²⁺ but is abolished by pretreatment with ionomycin (in the absence of extracellular Ca²⁺);
2. The molecule potentiates increases in [Ca²⁺]ᵢ elicited by submaximal concentrations of extracellular Ca²⁺;
3. The increase in [Ca²⁺]ᵢ elicited by extracellular Ca²⁺ is not inhibited by dihydropyridines;
4. The transient increase in [Ca²⁺]ᵢ caused by the molecule is abolished by pretreatment for 10 min. with 10 mM sodium fluoride;
5. The transient increase in [Ca²⁺]ᵢ caused' by the molecule is diminished by pretreatment with an activator of protein kinase C (PKC), such as phorbol myristate acetate (PMA), mezerein or (-)-indolactam V. The overall effect of the protein kinase C activator is to shift the concentration-response curve of the molecule to the right without affecting the maximal response;
6. The molecule causes a rapid (< 30 sec.) increase in the formation of inositol-1,4,5- trisphosphate and or diacylglycerol;
7. The molecule inhibits dopamine- or isopro terenol-stimulated cyclic AMP formation;
8. The molecule inhibits PTH secretion;
9. Pretreatment with pertussis toxin (100 ng/ml for > 4 hrs.) blocks the inhibitory effect of the molecule on cyclic AMP formation but does not effect increases in [Ca²⁺]ᵢ, inositol-1,4,5-trisphosphate, or diacylglycerol, nor decreases in PTH secretion;
10. The molecule elicits increases in [Ca²⁺]ᵢ as detected, for example, as an increase in Cl⁻ current in Xenopus oocytes injected with poly(A)⁺- enriched mRNA from bovine or human parathyroid cells but is without effect in Xenopus oocytes injected with water or rat brain or liver mRNA; and
11. Similarly, using a cloned receptor from parathyroid cells, the molecule will elicit a response in Xenopus oocytes injected with the specific cDNA, mRNA or synthetic sense RNA (cRNA) encoding the receptor.

By "calcilytic" molecule is meant any molecule which blocks one or more of the activities of extracellular Ca²⁺ on an extracellular Ca²⁺-sensing cell, preferably by acting as an antagonist at the Ca²⁺ receptor. For example, when used in reference to a parathyroid cell, it is a molecule which, when tested on parathyroid cells in vitro, possesses one or more, and preferably all of the following characteristics as measured by techniques well known to those in the art:
1. The molecule blocks, either partially or completely, the ability of increased concentrations of extracellular Ca²⁺ to:
   a) increase [Ca²⁺]ᵢ,
   b) mobilize intracellular Ca²⁺,
   c) increase the formation of inositol-1,4,5-trisphosphate,
   d) decrease dopamine- or isoproterenol-stimulated cyclic AMP formation, and
   e) inhibit PTH secretion;
2. At low [Ca²⁺], i.e., 0.5 mM, the molecule by itself does not change [Ca²⁺]ᵢ;
3. The molecule blocks increases in Cl⁻ current in Xenopus oocytes injected with poly(A)⁺- mRNA from bovine or human parathyroid cells elicited by extracellular Ca²⁺ or calcimimetic compounds but not in Xenopus oocytes injected with water or rat brain or liver mRNA;
4. Similarly, using a cloned receptor from parathyroid cells, the molecule will block a response in Xenopus oocytes injected with the specific cDNA, MRNA or cRNA encoding the Ca²⁺ receptor, elicited by extracellular Ca²⁺ or a calcimimetic compound.

Parallel definitions of useful calcimimetics and calcilytics at Ca²⁺ receptors on other cell types are evident from the examples provided below.

The Ca²⁺ receptor is able to detect and respond to certain inorganic polycations and polycationic organic molecules. For example, the parathyroid cell is unable to distinguish increases in extracellular Ca²⁺ concentration from the addition of these organic polycations, presumably because these organic molecules act just like extracellular Ca²⁺ at the Ca²⁺ receptor. The calcimimetic molecules described herein are particularly good agonists of the Ca²⁺ receptor and may be used as drugs that alter selected cellular functions, e.g., secretion of PTH from parathyroid cells. Unlike Ca²⁺ most of these molecules act only at one or more, but not all Ca²⁺ receptors, and thus provide an ability to specifically target one Ca²⁺ receptor.

The calcimimetics and calcilytics can be formu lated as pharmaceutical compositions which are useful for regulating the level of extracellular free Ca²⁺ in a patient and for mimicking the effect of extracellular Ca²⁺ on a cell selected from the group described above, by administering to the patient such a pharmaceutical composition. Prior to this invention, applicant was unaware of any such molecules acting on the Ca²⁺ receptor useful in treatment of diseases caused by irregularity in operation or regulation of a Ca²⁺ receptor or diseases in an animal having normal Ca²⁺ receptors but which can be treated by activating or deactivating such Ca²⁺ receptors.

It is the specificity of action of such molecules that is particularly advantageous in this invention since it allows specific in vivo and in vitro therapy and diagnosis and discovery of additional calcimimetic or calcilytic molecules.

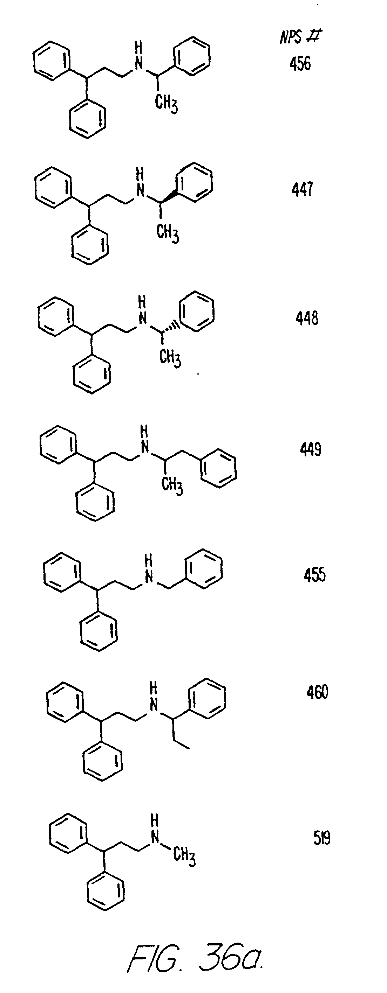

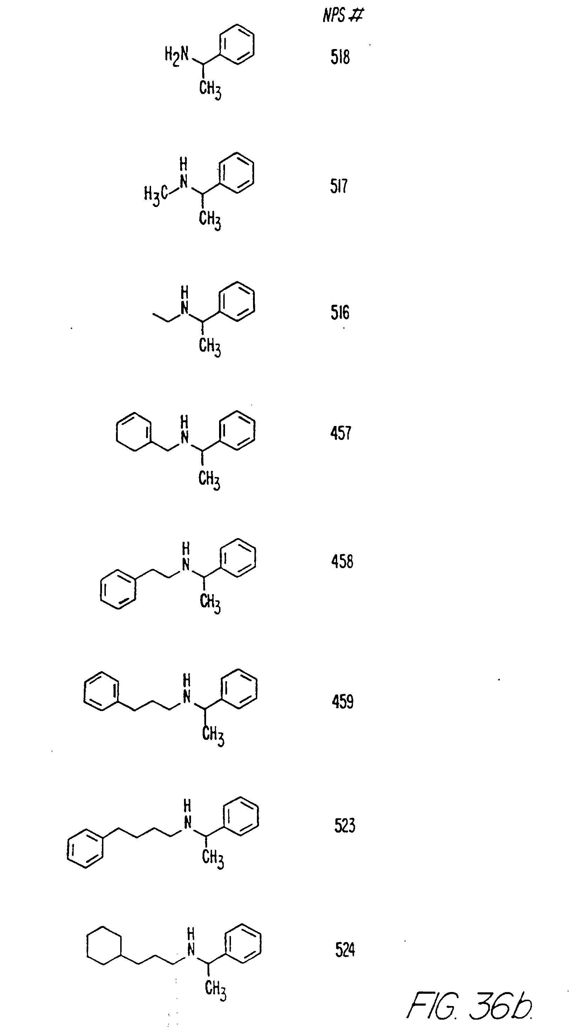

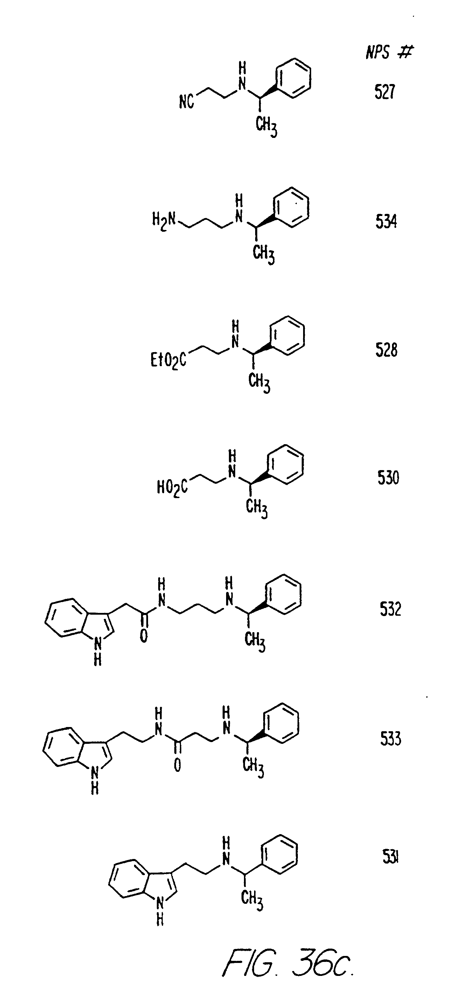

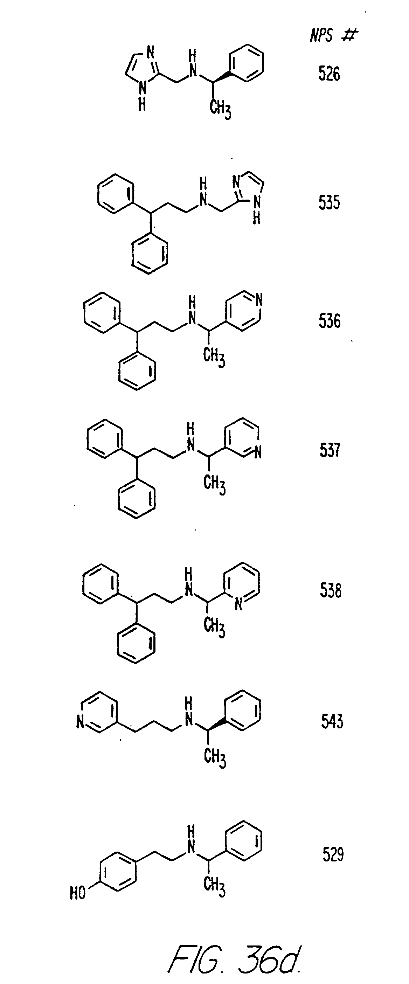

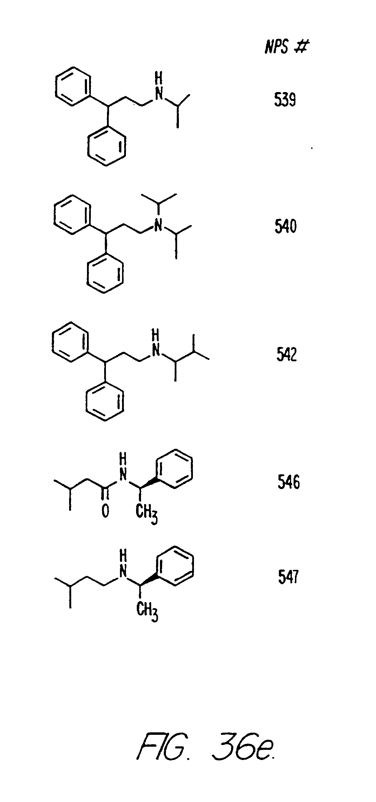

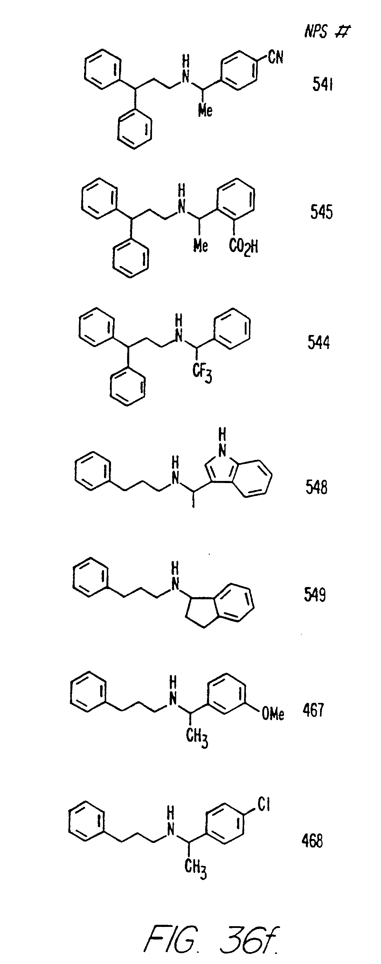

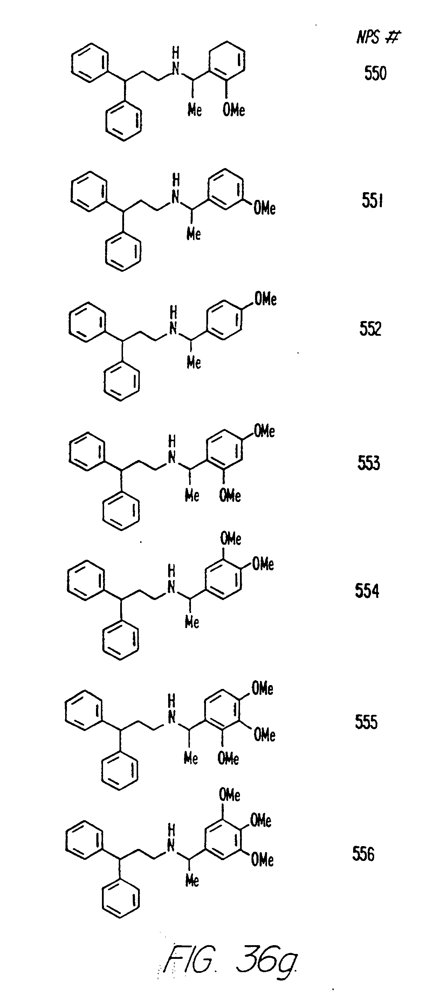

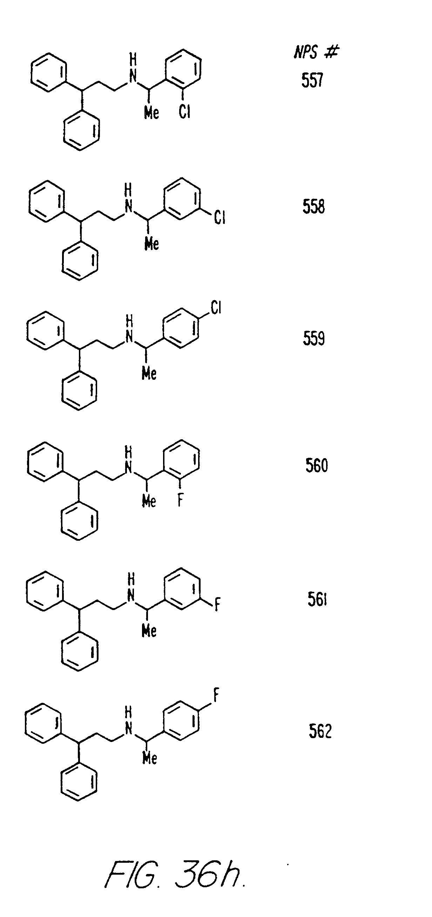

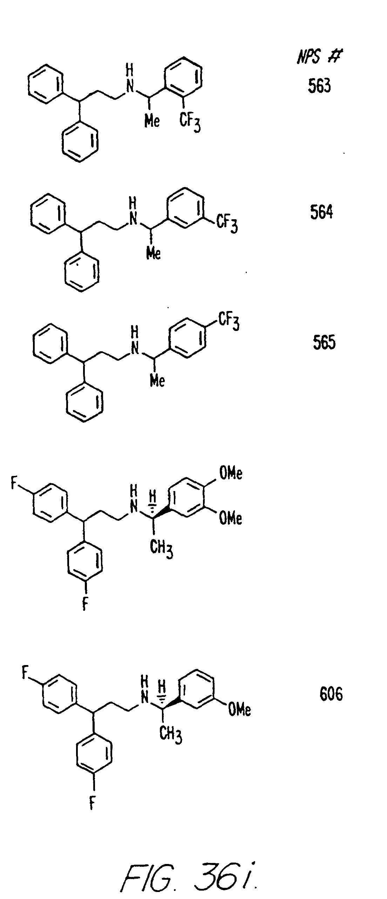

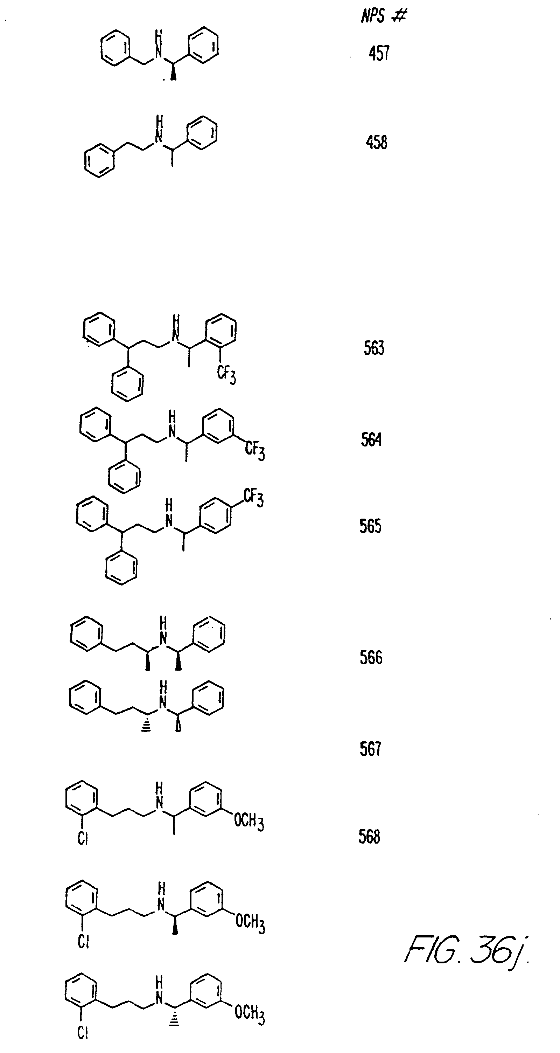

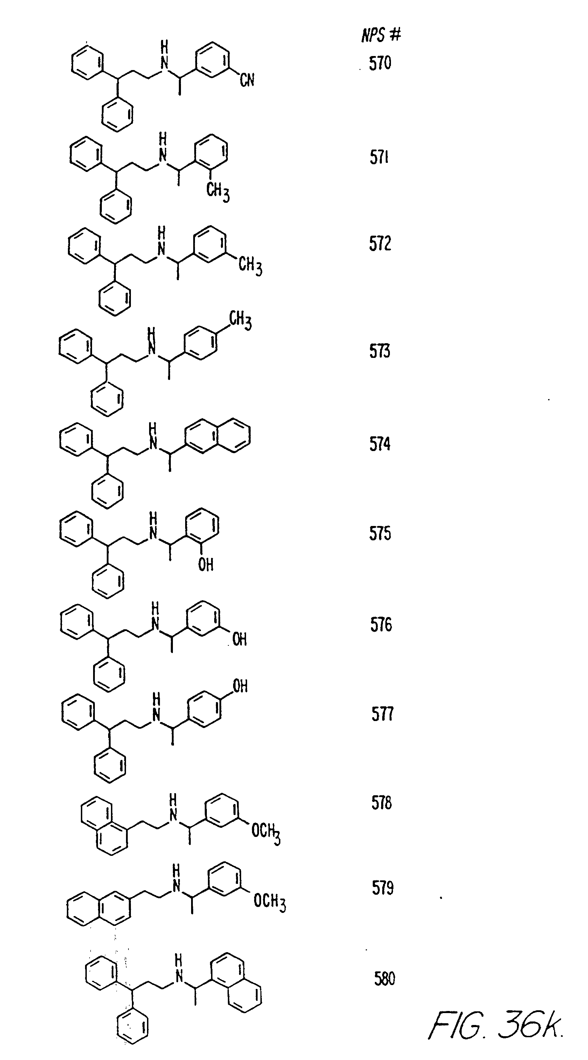

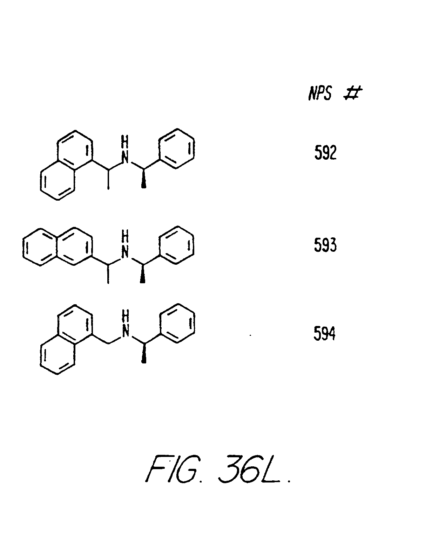

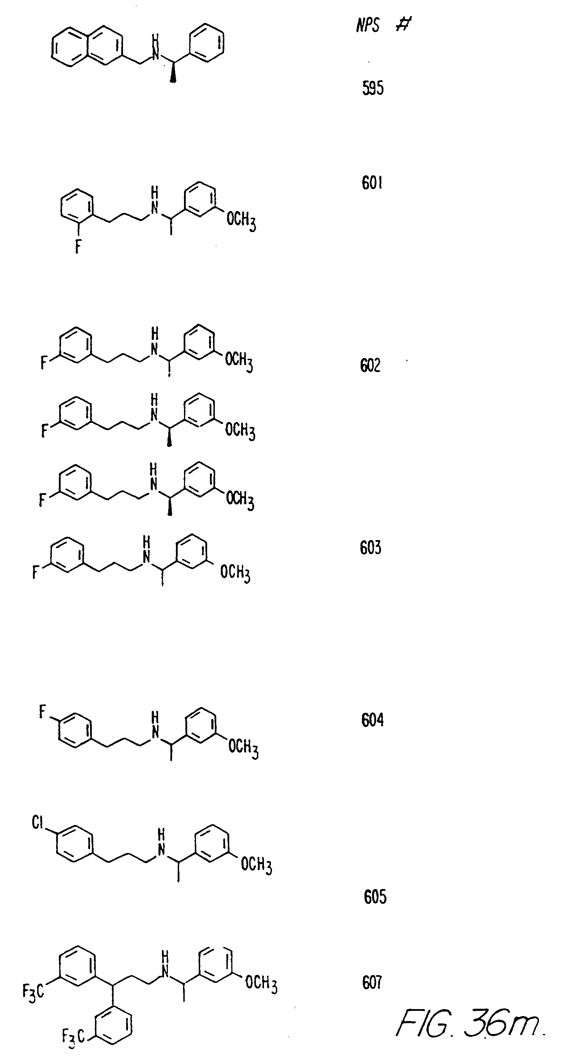

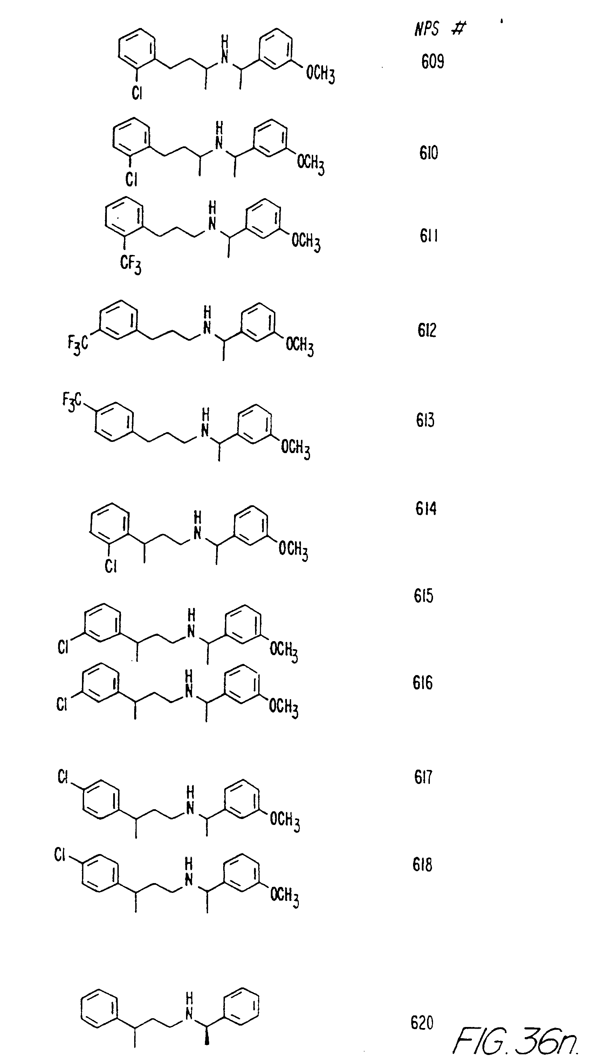

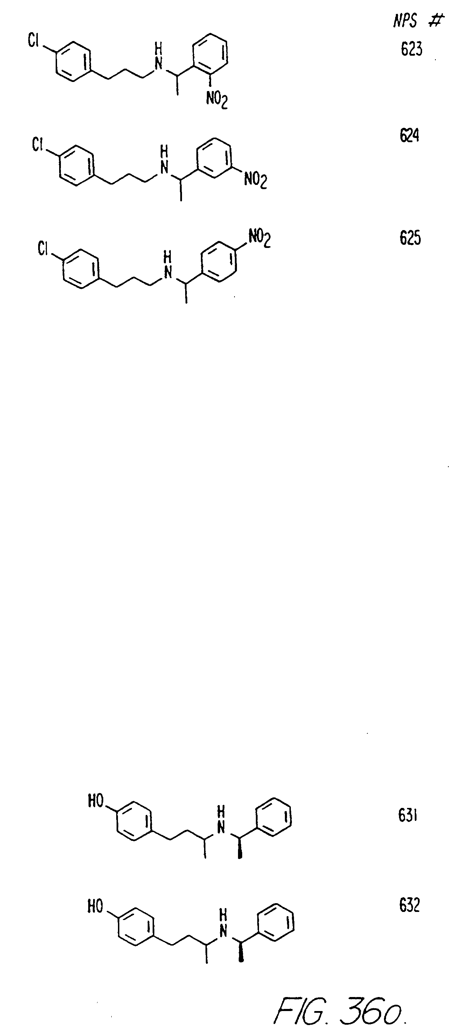

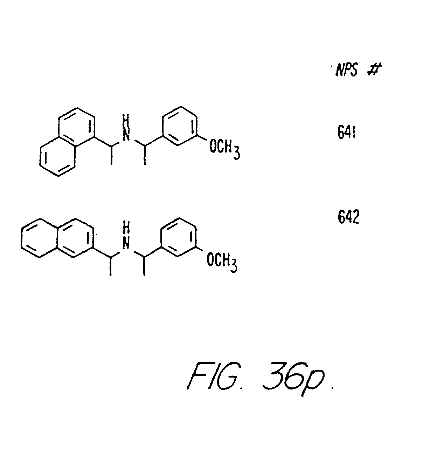

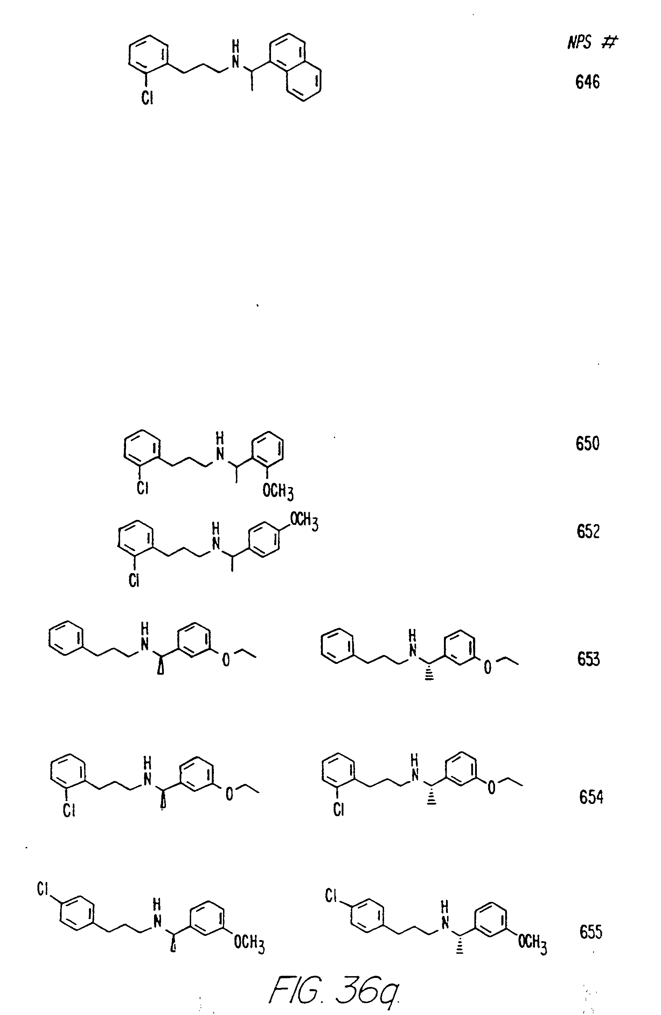

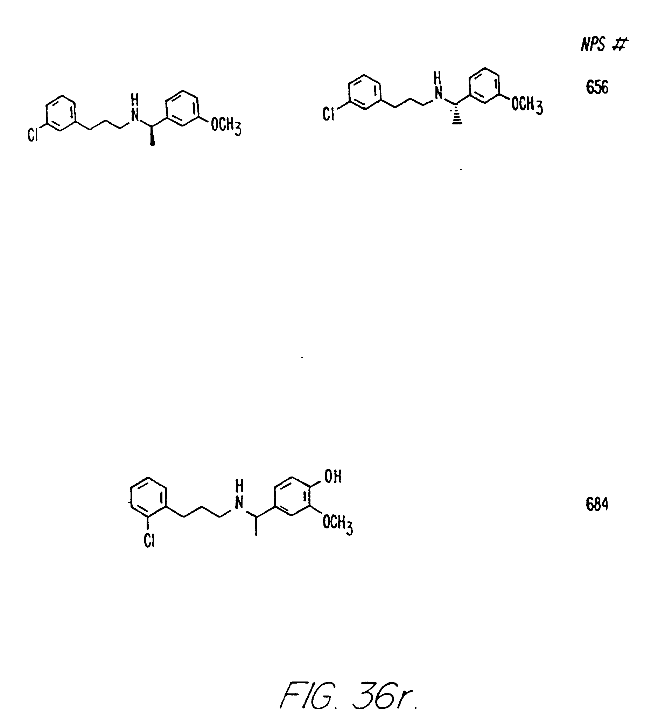

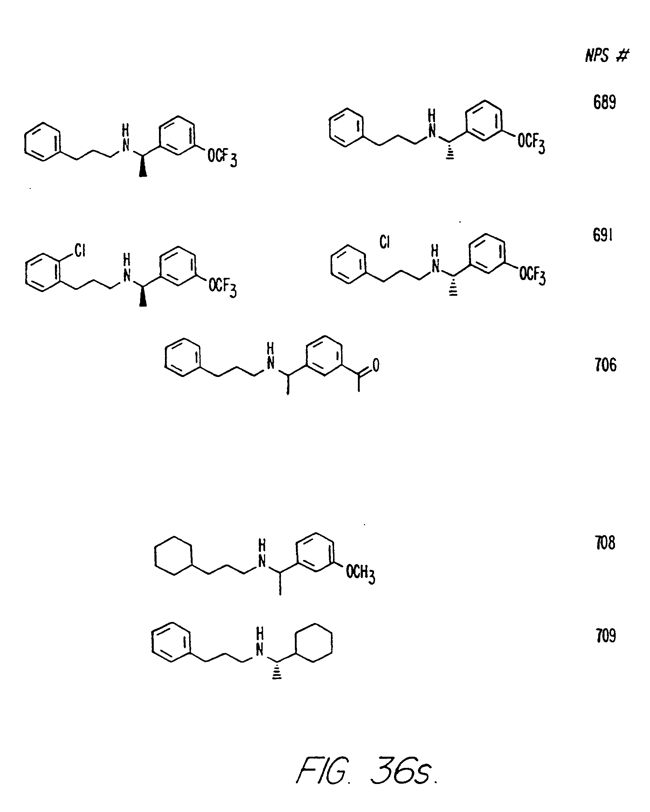

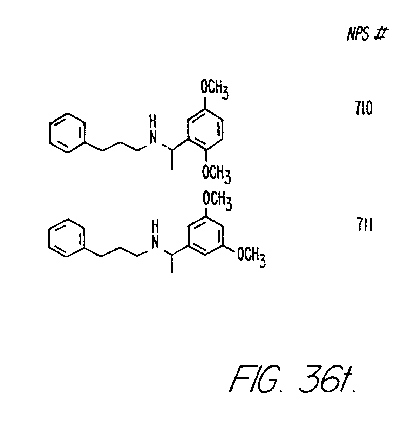

Thus,the invention features a recombinant Ca²⁺ receptor, a cell including a recombinant Ca²⁺. receptor and purified nucleic acid encoding a Ca²⁺ receptor. Described are also the biological activity and use of the molecule NPS 019, the novel compounds or compositions of matter of NPS 459, NPS 467, and NPS 568 (see Fig. 36) and a method for identifying a useful calcimimetic or calcilytic molecule by identifying a molecule which mimics or blocks one or more activities of Ca²⁺ at a first Ca²⁺ receptor but not at a second Ca²⁺ receptor, e.g., by use of a recombinant Ca²⁺ receptor.

In another aspect, a cell including a recombinant Ca²⁺ receptor(s) from kidney is employed as a test system to screen antibiotics (e.g. aminoglycoside antibiotics) for activity at calcium receptors which produces renal toxicity in humans.

By "recombinant" is meant to include any Ca²⁺ receptor produced by recombinant DNA techniques such that it is distinct from the naturally occurring Ca²⁺ receptor either in its location, purity or structure. Generally, such a receptor will be present in a cell in an amount different from those normally observed in nature.

By "purified" is meant that the antibody or nucleic acid is distinct from naturally occurring antibody or nucleic acid, being separated from antibody or nucleic acid with which it naturally occurs, e.g., in a vector system, such that it can be used to express recombinant Ca²⁺ receptor. Preferably, the antibody or nucleic acid is provided as a homogeneous preparation by standard techniques.

Such cloned receptors can be expressed in a desired cell, and isolated and crystallized to allow structure determination. Such a structure will allow design of useful molecules of this invention which can bind to the Ca²⁺ receptor. In addition, equivalent such receptors can be cloned using a first clone as a probe for clones in other cell, cDNA or genomic libraries.

Antibodies to the cloned receptor can be isolated and used as therapeutics in this invention, or as diagnostic tools for determining Ca²⁺ receptor numbers and/or locations and/or functional integrity to diagnose Ca²⁺-related diseases or conditions. Such antibodies can also be used in vivo by intravenous administration as calcimimetics or calcilytics.

Described are also calcimimetic or calcilytic molecules able to act as either selective agonists or antagonists respectively at a Ca²⁺ receptor of one or more but not all cells chosen from the group consisting of parathyroid cells, bone osteoclasts, juxtaglomerular kidney cells, proximal tubule kidney cells, distal tubule kidney cell, cell of the thick ascending limb of Henle's loop and/or collecting duct, keratinocyte in the epidermis, parafollicular cell in the thyroid (C-cells), intestinal cell, trophoblast in the placenta, platelet, vascular smooth muscle cell, cardiac atrial cell, gastrin and glucagon secreting cells, kidney mesangial cell and mammary cell. Such a composition may include any pharmaceutically acceptable carrier known to those in the art to provide a pharmaceutical composition.

The described nucleic acid molecules can be used to modulate the number of Ca²⁺ receptors in a patient by standard techniques, e.g, antisense and related technologies (e.g., ribozymes), as a therapeutic for a disease state.

The described nucleic acid molecules and encoded polypeptides can be used in methods for identifying molecules which affect the activity of a Ca²⁺ receptor using assays, as defined below, to detect calcimimetics and/or calcilytics. Further, molecules found to be effective to reduce or enhance expression of Ca²⁺ receptor at a transcriptional or translational level by use of the assays or antibodies or other techniques described below can be defined for therapeutic uses.

The foregoing summary has been largely in connection with the preferred embodiment which relates generally to calcium receptors. The disclosed receptor belongs to a novel superfamily of polypeptide receptor/sensor molecules. The cDNA clone, BoPCaR 1, disclosed herein and duly deposited, represents the first such clone described to encode a receptor/sensor which is a member of this superfamily. For purposes of the present invention, receptor/sensors belonging to this superfamily are called inorganic-ion receptors.

The novel superfamily of inorganic ion receptors includes a variety of such molecules which are related to each other by similarity of amino acid sequence, by structure and/or by function. In total, these attributes also distinguish members of this superfamily of receptors from all receptors/sensors currently known in the art. Members of this superfamily of receptors are primarily distinguished functionally by their surprising ability to detect and respond to changes in the levels of inorganic cations such as calcium, magnesium, potassium, sodium, or hydrogen ions and the like, and upon sensing such ions, to evoke changes in cellular functions. Such changes may involve changes in second messenger levels as occur for known G-protein coupled receptors, or changes in ionic transmembrane ion flux or the like. Other members of this superfamily also exist which sense inorganic anions such as phosphate or chloride ions. All such receptors are within the scope of the current invention as described herein.

Receptors belonging to the superfamily of inorganic-ion receptors may be activated by stimuli other than ligand binding. For example, some members of this superfamily of receptors are activated by physical forces such as stretch forces acting on membranes of cells expressing such receptors. All such receptors are within the scope of the present invention.

The inorganic-ion receptors can be used in the manner as described above in connection with the preferred receptor. Recombinant receptors expressed in a variety of tissue types including human tissue types can be used to screen (including high through-put screens) for drug discovery in methods known to those skilled in the art. Ionmimetics and ionlytics can be readily identified. The procedures can be adapted for the individual function of the receptor. For example, calcium activated chloride current may be employed with an inorganic-ion receptor which forms a calcium channel or which nutrilizes intracellular calcium. Ligand-gated ion channels are known in the art. Inorganic-ion receptors that couple to second messenger systems permit assays as described above, including measurement of cyclic AMP and inositol phosphates. Cells that have recombinant receptors coupled to readily detectable agents also may be,used such as G-protein coupling of receptors to pigment dispersion in melanophores as described in the literature. Thus, described are also methods for discovering agents that are inorganic-ion receptor mimetics or lytics, as well as such mimetics and lytics themselves, including those preferably selected from the mimetics and lytics described in detail above. Likewise, described are diagnosis and treatment of inorganic-ion receptor related diseases or conditions as described above in connection with the preferred embodiment. For example, diseases associated with elevated levels of an inorganic-ion receptor can be diagnosed based upon assays for such elevated levels. Therapeutic molecules can be identified by screening for agents that mimic the activity of the relevant native ion or that modify the effect or potency of the relevant native ion. Tissue specific expression as well as levels of expression can be evaluated and the like.

Thus, in this aspect of the invention, a novel superfamily of isolated inorganic-ion receptors is provided and unique fragments thereof also are provided. Preferably, the isolated receptors are human receptors, and most preferably the isolated receptor is a calcium receptor expressed in tissues or cells selected from the group consisting of: parathyroid, vascular, kidney, epidermis, thyroid, osteoclast, intestine, mammary, trophoblast, platelet, gastrin secreting, glucagon secreting, cardiac, and brain, and unique fragments thereof. The invention further features polypeptide fragments of the foregoing inorganic-ion receptors which have desirable activity. For example, the fragment may include just a binding site, or a site which binds to mimics, agonists or antagonists. Other useful fragments include those that have only the external portion, membrane-spanning portions, or intracellular portion of the receptor. In addition, these fragments are useful for forming chimeric receptors with fragments of other receptors, as described in greater detail below. The invention also features muteins or analogues and other derivatives of the isolated receptors. Thus, the invention embraces not only naturally occurring proteins, but derivatives thereof.

The invention also features nucleotide sequences encoding the foregoing inorganic-ion receptors and fragments and derivatives thereof. Such nucleotide sequences may be obtained through a variety of procedures, and the disclosure of the present invention allows one of ordinary skill in the art to obtain cDNA or genomic clones encoding such receptors. For example, hybridization probes may be made based upon the nucleotide sequence of BoPCaR 1. When genomic libraries for cDNA libraries from any tissue are screened at low stringency with such probes, hybridizing clones are obtained which encode other members of the superfamily. Additionally, antibodies can readily be prepared which bind to cloned or isolated inorganic-ion receptors. Such antibodies may also be used to isolate other receptors of the invention by expression-cloning techniques known to those of ordinary skill in the art. Targeted gene walking also may be employed to identify and clone members of the superfamily of inorganic-ion receptors. In addition, the clones may be obtained through expression cloning procedures as described above. These procedures can be adapted for the individual function of the inorganic-ion receptor of interest. For example, calcium activated chloride currents may also be employed for cloning of an inorganic-ion receptor which forms a calcium channel or which mobilizes intracellular calcium. Ligand-gated ion channels, as discussed above, are known in the art. For example, a serotonin gated ion channel has been cloned in this manner. However, the receptors of the present invention are distinct from other known ligand gated ion channels in their amino acid sequences and in that they sense inorganic ions such as calcium, magnesium, hydrogen ions, phosphate ions, and the like.

It will also be appreciated by those skilled in the art that each cloned receptor obtained in this manner provides new such DNA or antibody probes which themselves are used to identify still additional clones encoding inorganic-ion receptors. Furthermore, it will be appreciated that having obtained the sequence of more than one such receptor as outlined above, information is available pertaining to localized sequence conservation which is useful for obtaining still additional clones encoding other members of the superfamily. Such conserved sequences also may be derived from an analysis of the overall structure of BoPCaR 1, as it conventionally includes an extracellular domain, transmembrane domain and intracellular domain.

Thus, isolated nucleic acids encoding inorganic-ion receptors and unique fragments thereof are provided. The preferred receptor is a calcium receptor that is expressed in the tissues or cells selected from the group described above. Most preferably, the nucleic acid encodes a human inorganic-ion receptor or a unique fragment thereof. The invention further provides isolation of the endogenous regulatory elements controlling the expression of the foregoing inorganic-ion receptors, and agents that are capable of agonizing or antagonizing the activity of these regulatory elements also may be identified.

The invention further provides recombinant cells expressing the nucleic acids of the invention. In such cells, the nucleic acid may be under the control of its genomic regulatory elements, or may be under the control of exogenous regulatory elements including an exogenous promoter. By "exogenous" it is meant a promoter that is not normally coupled in vivo transcriptionally to the coding sequence for the inorganic-ion receptor.

Purified antibodies to the foregoing inorganic-ion receptors are provided as well as antibodies to allosteric sites of such receptors and idiotypes of such receptors.

Inorganic-ion receptor binding agents coupled to a toxin also are provided. Such agents may be antibodies and, as such, are immunotoxins. Such immunotoxins may be used for example in killing a cell expressing an inorganic-ion receptor in vitro or in vivo.

In addition, the disclosed nucleic acid molecules can be used to prepare transgenic, nonhuman mammals containing a transgene encoding an inorganic-ion receptor or a unique fragment thereof. Such transgenes may be useful in affecting or altering the expression of an inorganic-ion receptor or in inactivating the expression of an inorganic-ion receptor through, for example, homologous recombination. Other transgenes may be provided in such mammals, including those encoding antisense or a protein that is capable of altering the expression of a native inorganic-ion receptor gene. Such alteration may be up-regulation, down-regulation or complete inactivation.

Thus, methods are provided involving contacting a cell with an agent that binds to a cellular component for the purpose of affecting the expression of an inorganic-ion receptor. Such agents may bind to promoters, other regulatory agents acting on promoters, agents capable of binding to the receptor and nucleic acids encoding the receptor or a unique fragment thereof. Contact may be by extracellular administration, by providing a transgene encoding the agent or by any other suitable method depending upon the use to which the particular method is directed.

Other features and advantages of the invention will be apparent from the following description of the preferred embodiments thereof, and from the claims.

### Description of the Preferred Embodiments

The drawings will first briefly be described.

### Drawings

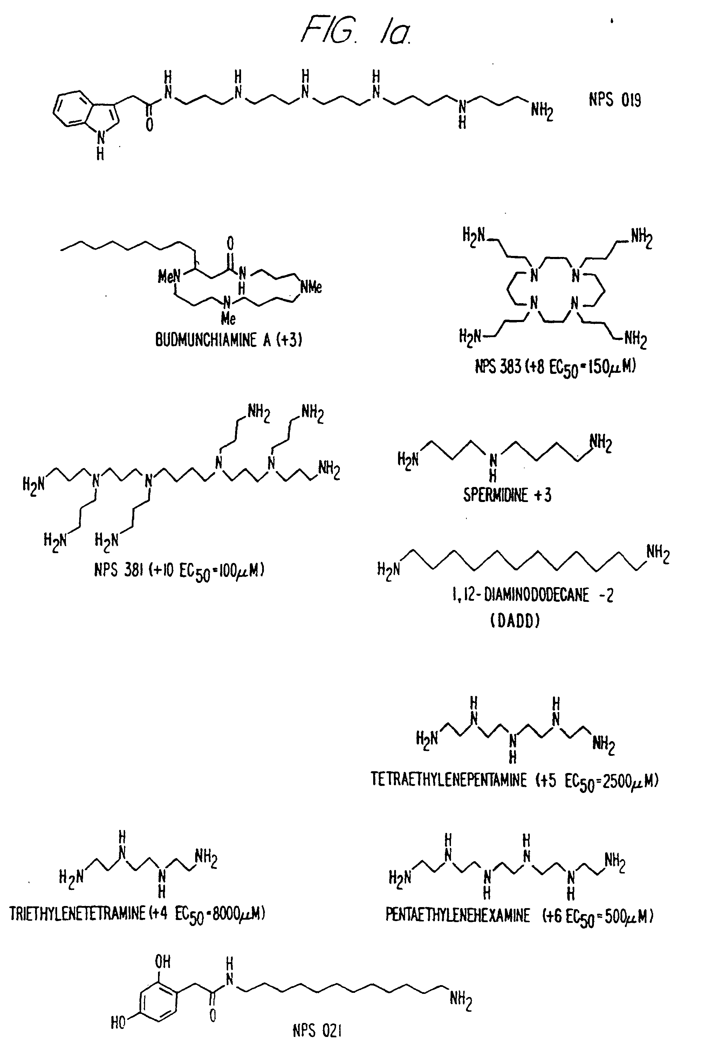

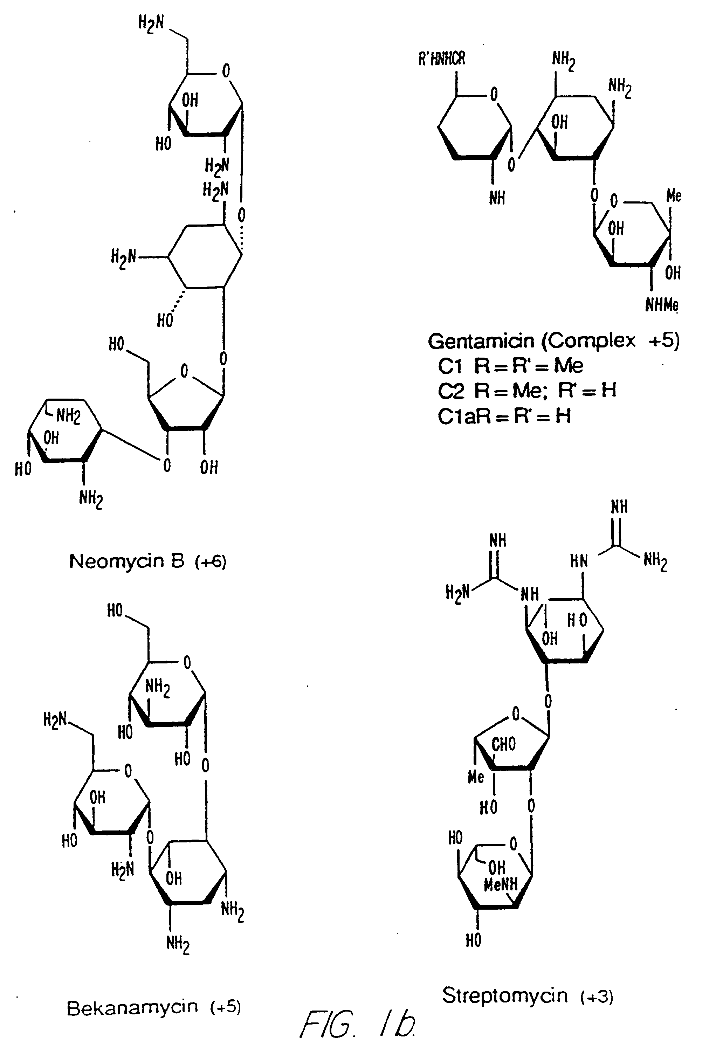

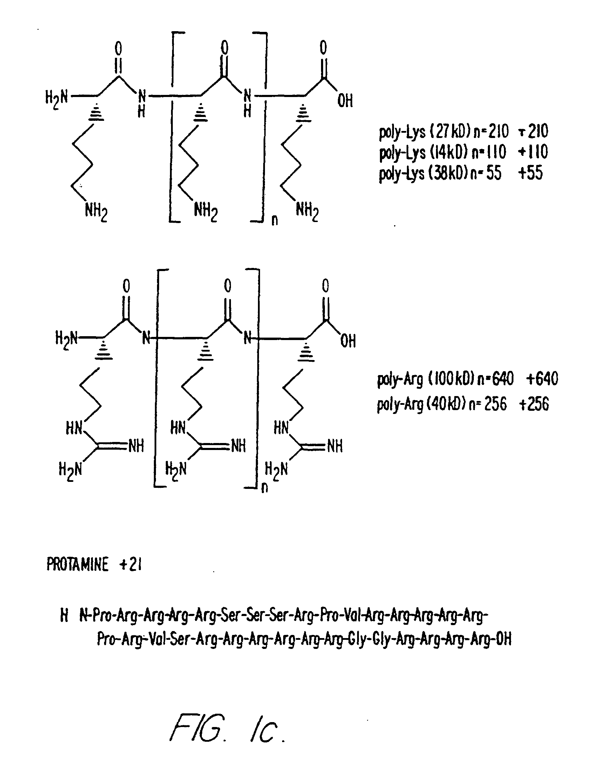

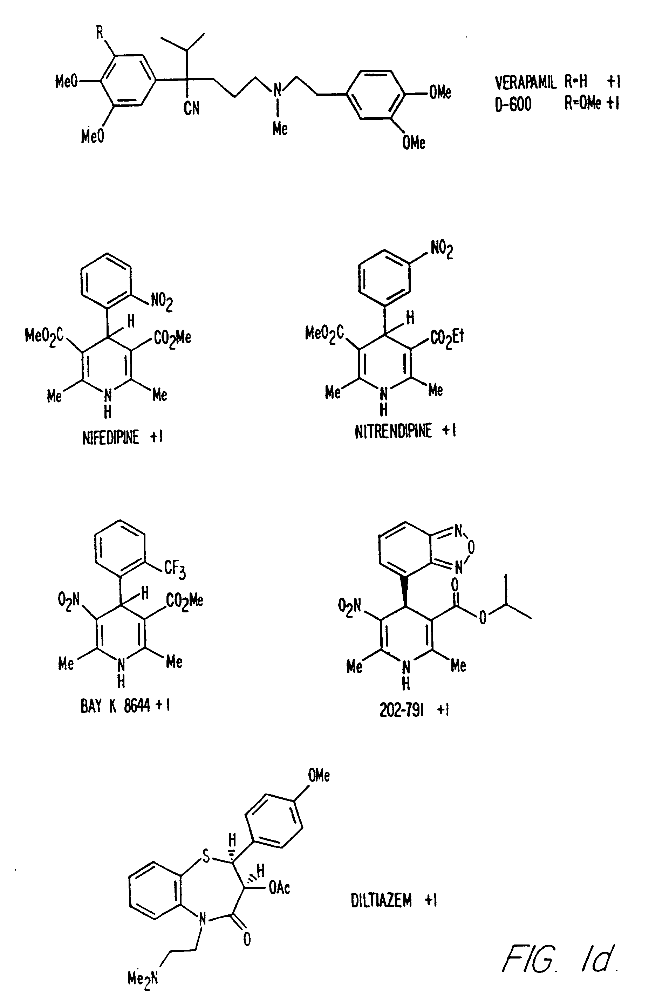

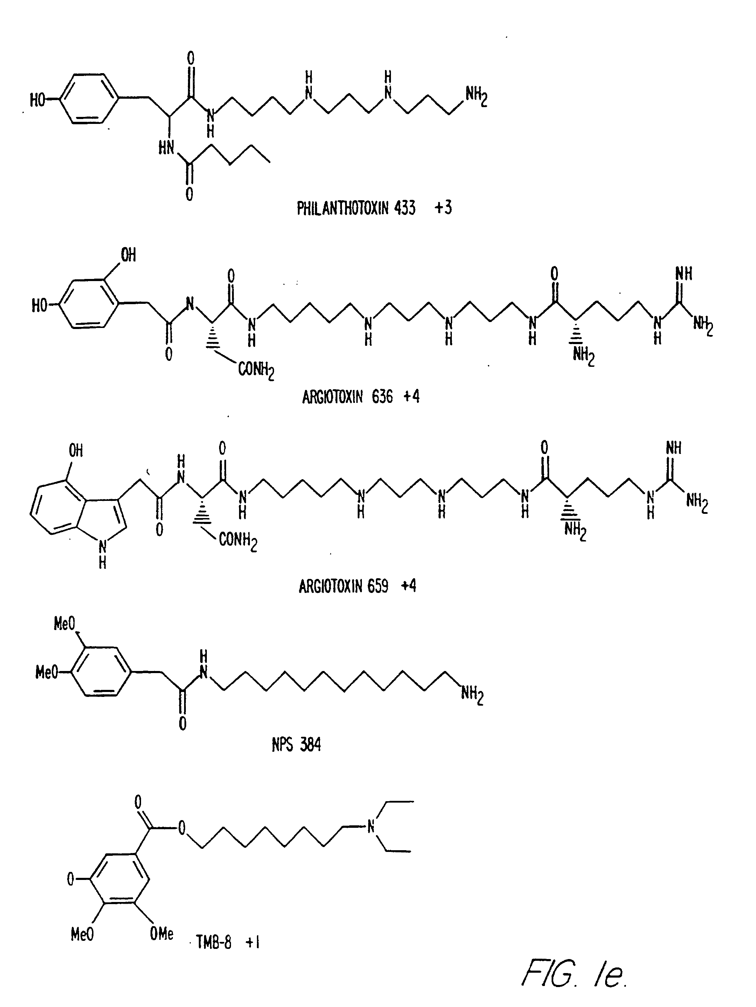

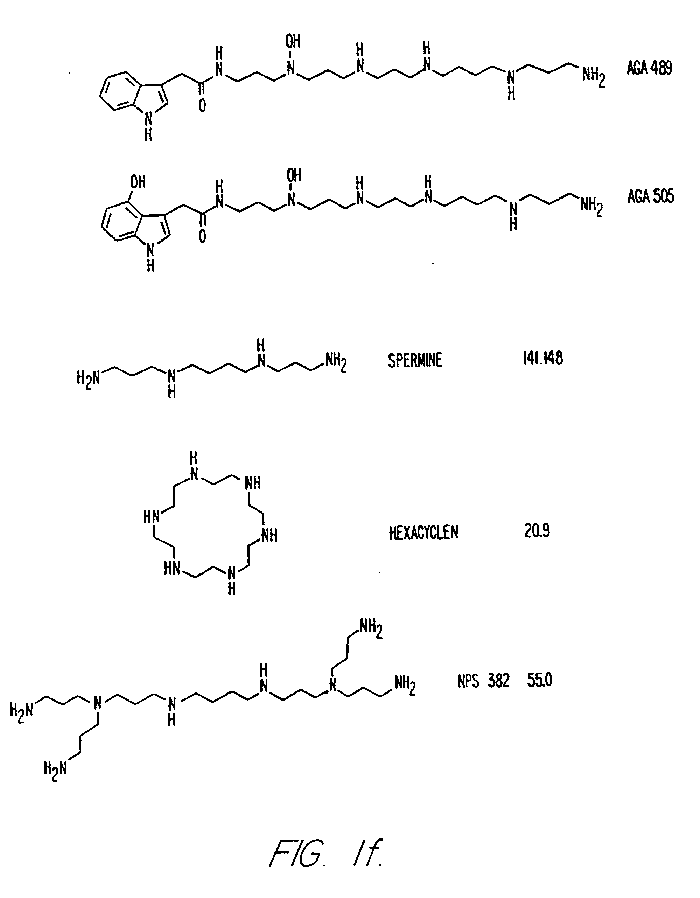

Fig. 1 depicts representative molecules useful in the invention.

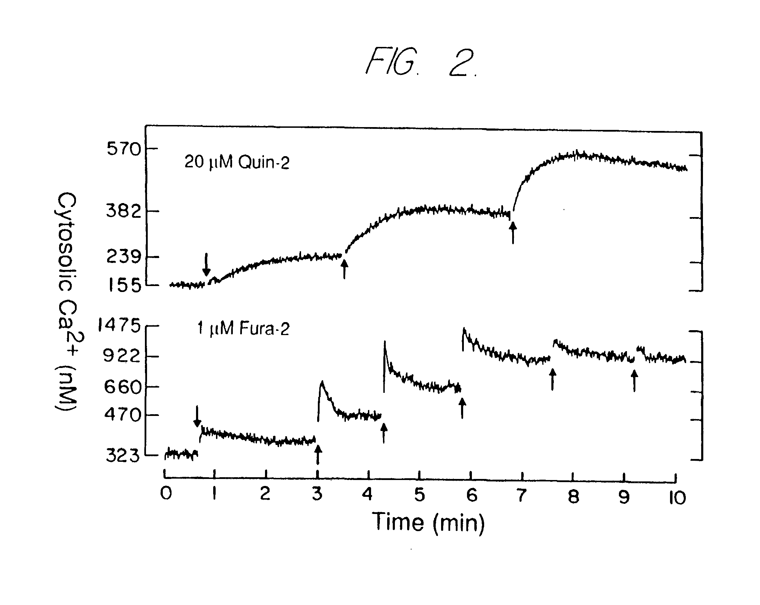

Fig. 2 is a graphical representation showing increases in [Ca²⁺]ᵢ induced by extracellular Ca²⁺ in quin-2- or fura-2-loaded bovine parathyroid cells. The initial [Ca²⁺] was 0.5 mM (using CaCl₂) and, at each of the arrows, was increased in 0.5 mM increments.

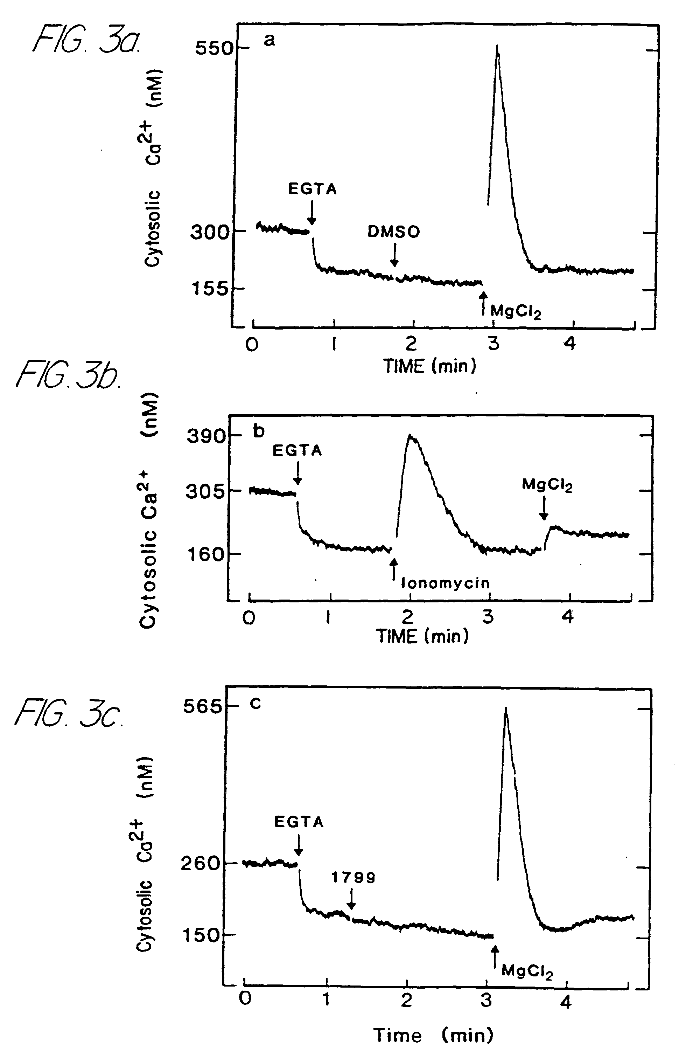

Fig. 3 is a graphical representation showing mobilization of [Ca²⁺]ᵢ in bovine parathyroid cells. The initial [Ca²⁺] was 0.5 mM and was decreased to < 1 µM by the addition of EGTA as indicated. (a) Extracellular Mg²⁺ (8 mM, final) elicits an increase in [Ca²⁺]ᵢ in the absence of extracellular Ca²⁺. (b) Pretreatment with ionomycin (1 µM) blocks the response to Mg²⁺. (c) Pretreatment with 5 µM molecule 1799 (a mitochondrial uncoupler) is without effect on the response to Mg²⁺.

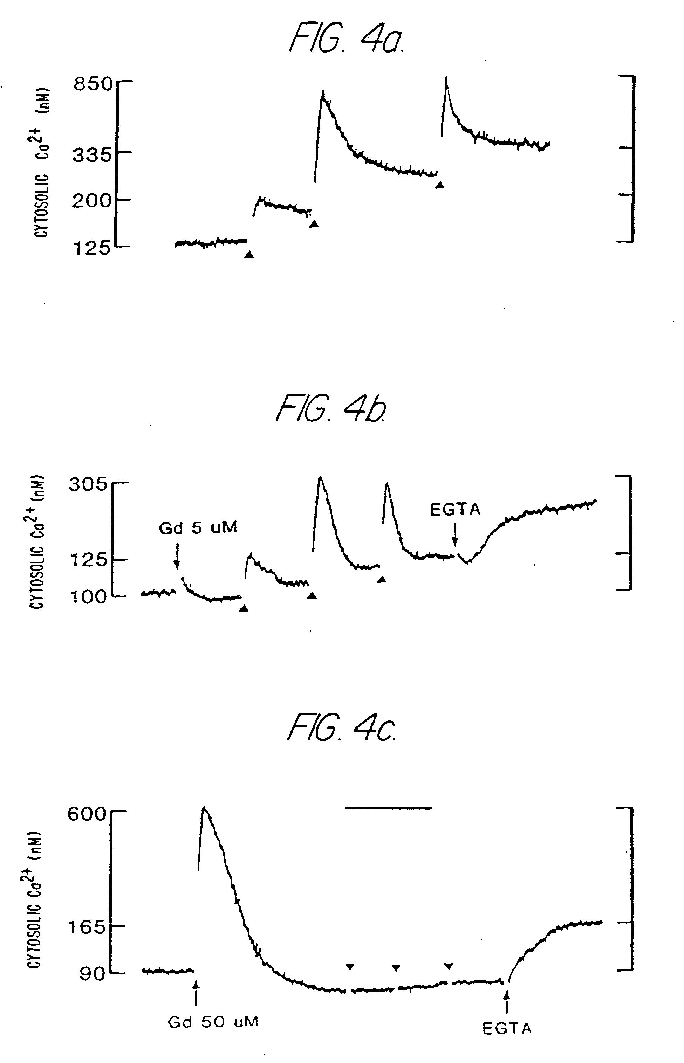

Fig. 4 is a graphical representation showing preferential inhibitory effects of a low concentration of Gd³⁺ on steady-state increases in [Ca²⁺]ᵢ and that a high concentration of Gd³⁺ elicits a transient increase in [Ca]ᵢ in bovine parathyroid cells. Top panel: Control. Initial concentration of extracellular Ca²⁺ was 0.5 mM and was increased by 0.5 mM at each of the arrowheads. Middle panel: Gd³⁺ (5 µM) blocks steady-state but not transient increases in [Ca²⁺]ᵢ elicited by extracellular Ca²⁺. Lower panel: Gd³⁺ (50 µM) elicits a transient increase in [Ca²⁺]ᵢ and abolishes both transient and sustained responses to extracellular Ca²⁺. In the middle and lower panels, just enough EGTA was added to chelate preferentially Gd³⁺: the block of Ca²⁺ influx is removed and [Ca²⁺]ᵢ rises promptly.

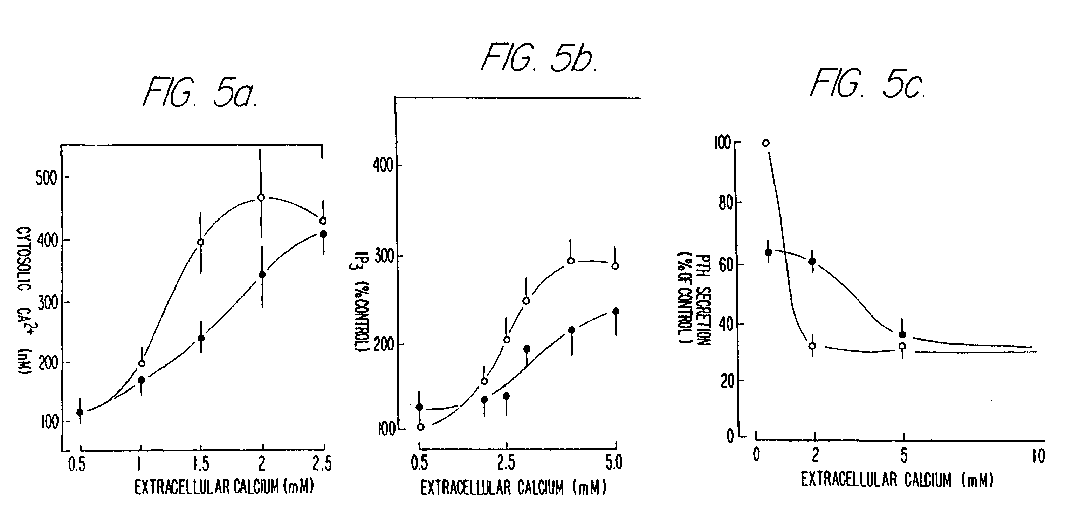

, Fig. 5 is a graphical representation showing that the effects of PMA on [Ca²⁺]ᵢ, IP₃ formation, and PTH secretion are overcome by increasing concentrations 'of extracellular Ca²⁺ in bovine parathyroid cells. For each variable, there is a shift to the right in the concentration-response curve for extracellular Ca²⁺. Note also that the concentration- response curves vary sigmoidally as [Ca²⁺] increases linearly.

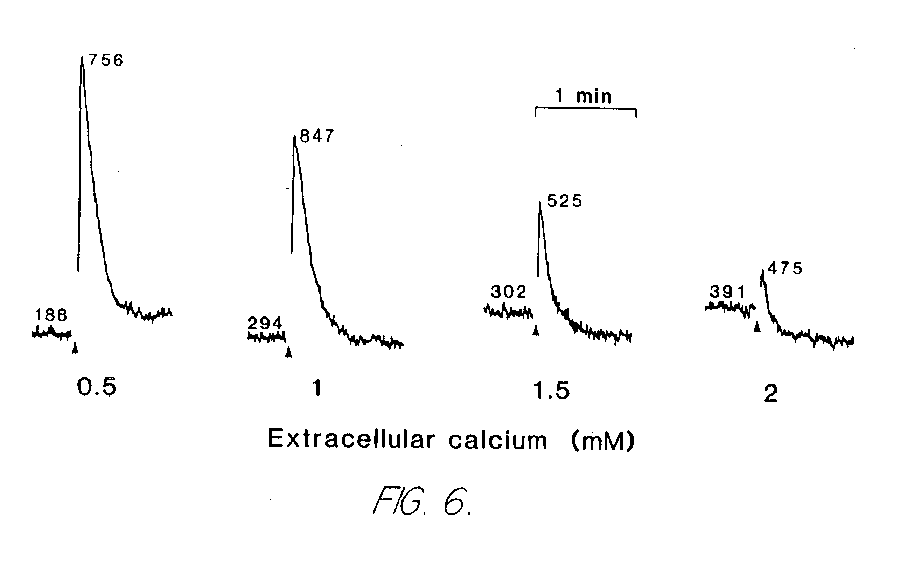

Fig. 6 is a graphical representation showing that increases in [Ca²⁺]ᵢ elicited by spermine are progressively depressed by increasing [Ca²⁺] in bovine parathyroid cells. Spermine (200 µM) was added at the time shown by arrowheads. In this and all subsequent figures, the numbers accompanying the traces are [Ca²⁺]ᵢ in nM.

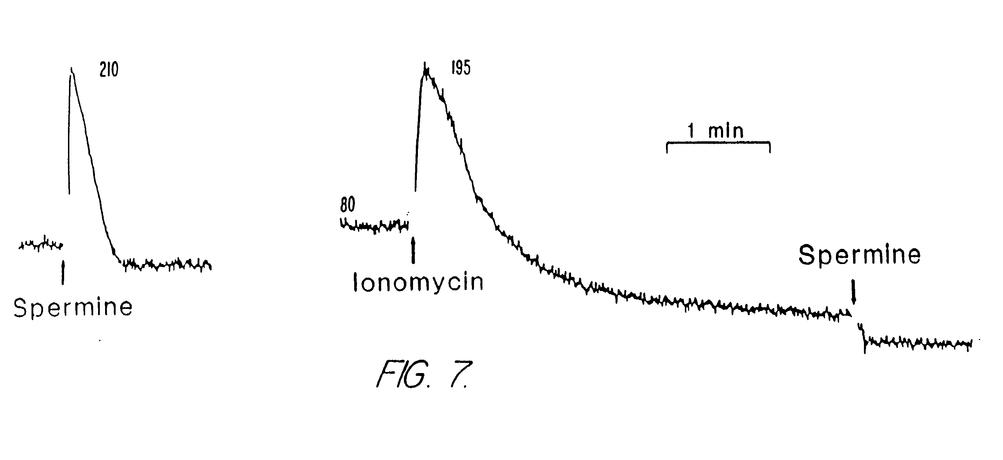

Fig. 7 is a graphical representation showing that spermine mobilizes intracellular Ca²⁺ in bovine parathyroid cells. EGTA was added to reduce [Ca²⁺] to <1 àM before the addition of spermine (200 µM) as indicated (left trace). Pretreatment with ionomycin (1 µM) blocks the response to spermine (right trace).

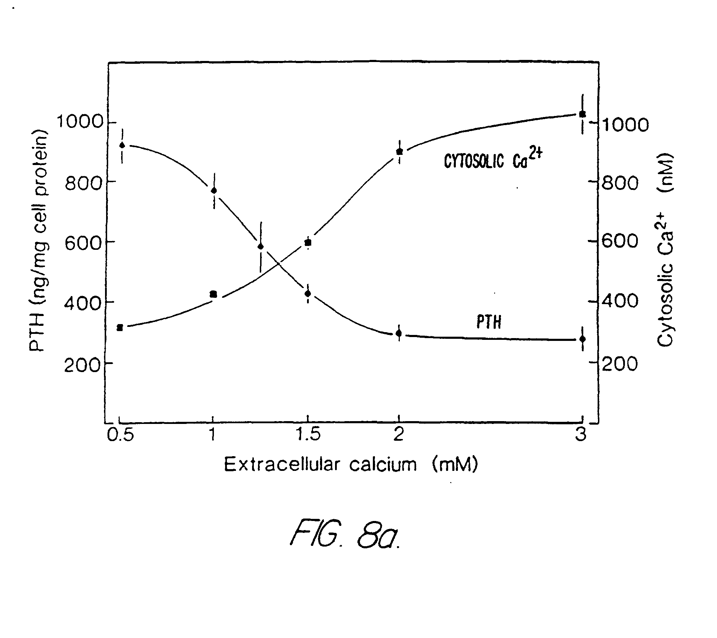

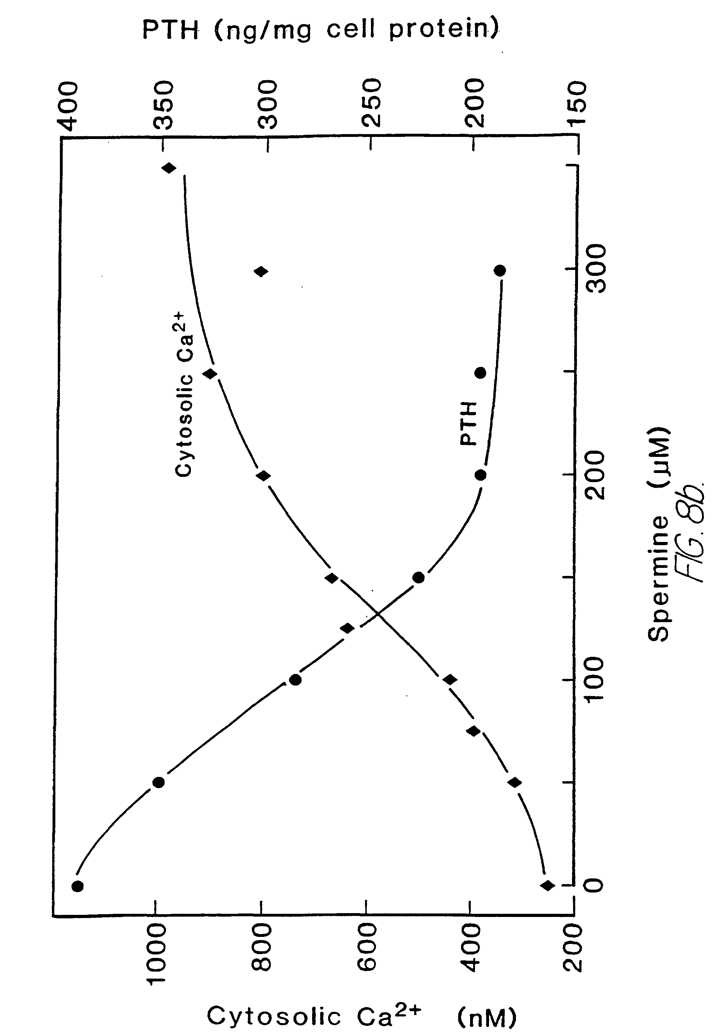

Figs. 8A and B are graphical representations showing that spermine increases [Ca²⁺]ᵢ and inhibits PTH secretion in bovine parathyroid cells similarly to extracellular Ca²⁺. The data points for the spermine dose-concentration response curves are the means of two experiments.

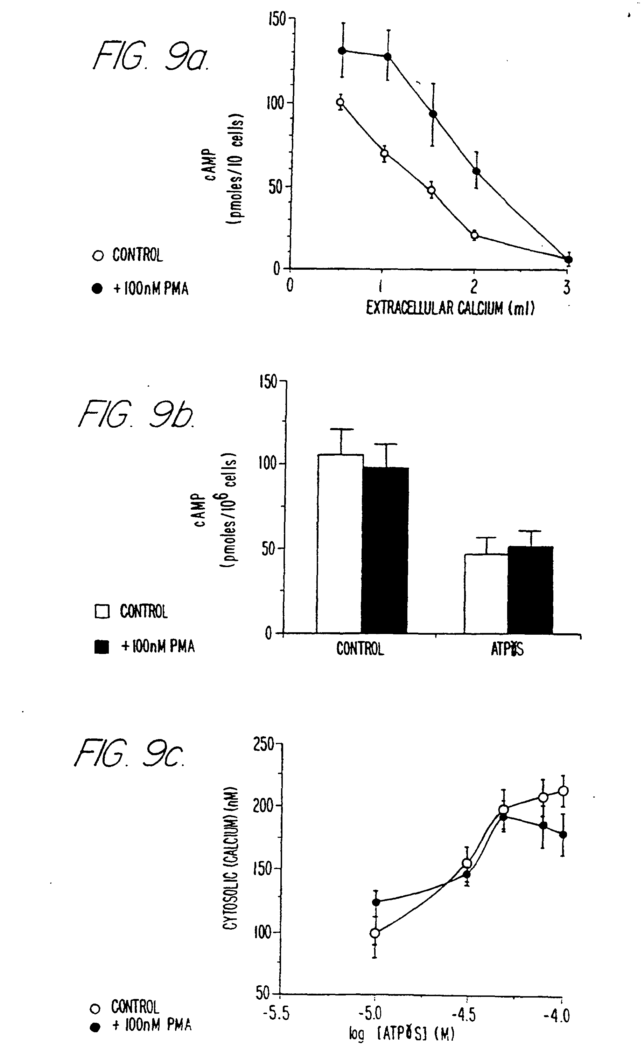

Fig. 9 is a graphical representation showing the contrasting effects of PMA on responses to extracellular Ca²⁺ and on responses to ATPγS in bovine parathyroid cells. Left panel: The concentration- response curve for extracellular Ca²⁺-induced inhibition of cyclic AMP formation is shifted to the right by PMA (100 nM). Middle panel: PMA does not affect the ability of ATPγS to increases [Ca²⁺]ᵢ. Note also that the concentration-response curve to ATPγS shows classical sigmoidal behavior as a function of the log concentration, in contrast to extracellular divalent cations.

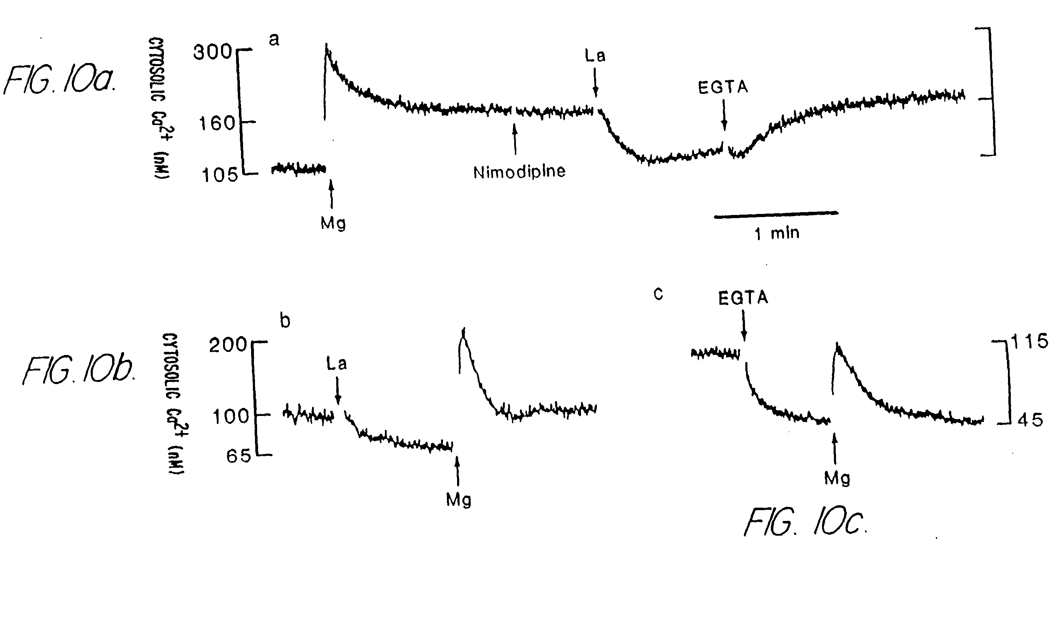

Fig. 10 is a graphical representation showing mobilization of intracellular Ca²⁺ in human parathyroid cells evoked by extracellular Mg²⁺. Cells were obtained from an adenoma and bathed in buffer containing 0.5 mM extracellular Ca²⁺. (a) Transient and sustained increases in [Ca²⁺]ᵢ elicited by extracellular Mg²⁺ (10 mM, final) shows that sustained increases are not affected by nimodipine (1 µM) but are depressed by La³⁺ (1 µM) and return promptly when La³⁺ is selectively chelated by a low concentration of EGTA. (b) La³⁺ (1 µM) blocks the sustained but not the transient increase in [Ca²⁺]ᵢ elicited by extracellular Mg²⁺. (c) Cytosolic Ca²⁺ transients elicited by extracellular Mg²⁺ persist in the absence of extracellular Ca²⁺.

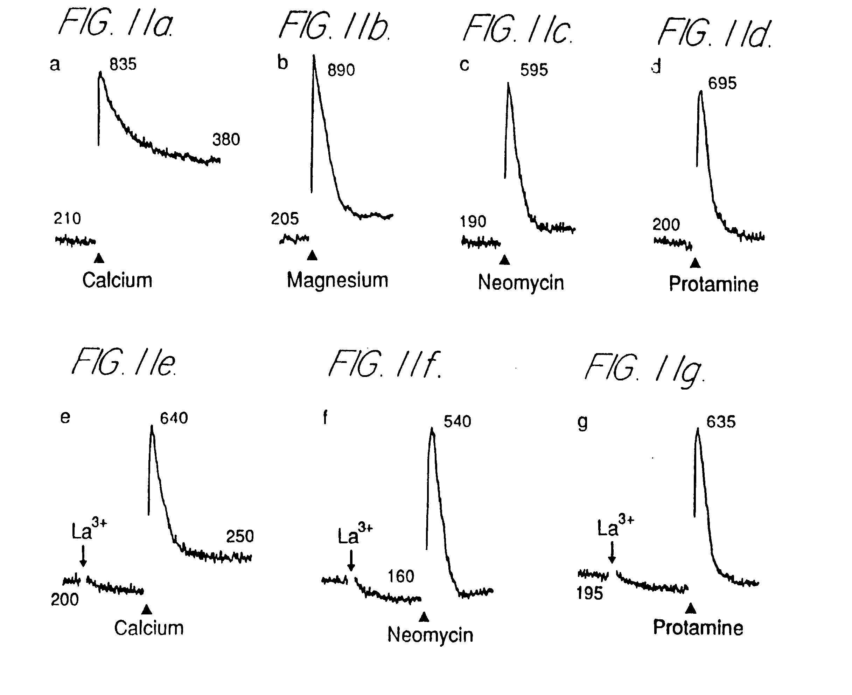

Fig. 11 is a graphical representation showing mobilization of intracellular Ca²⁺ evoked by neomycin or protamine in bovine parathyroid cells. In all traces, the initial [Ca²⁺] and [Mg²⁺] was 0.5 and 1 mM, respectively. In trace (a) and (b), the Ca²⁺ and Mg²⁺ concentrations were increased to 2 and 8 mM, from 0.5 and 1 mM respectively. In the other traces, (c) through (i), neomycin B (30 µM) or protamine (1 µg/ml) were added as indicated. La³⁺ (1 µM) EGTA (1 mM), or ionomycin (100 nM) were added as indicated. Each trace is representative of the pattern seen in 5 or more trials using at least 3 different cell preparations. Bar = 1 min.

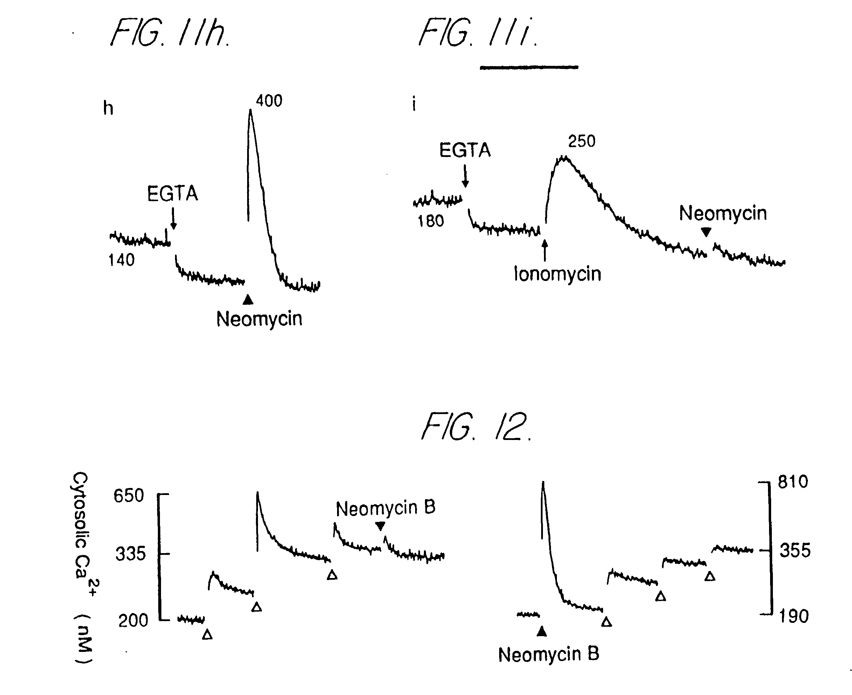

Fig. 12 is a graphical representation showing that neomycin B blocks transient but does not block steady-state increases in [Ca²⁺]ᵢ elicited by extracellular Ca²⁺ in bovine parathyroid cells. Left control: [Ca²⁺] was initially 0.5 mM and was increased in 0.5 mM increments at each of the open arrowheads before the addition of neomycin B (30 µM). Right: Neomycin B (30 µM) was added before [Ca²⁺]. Bar = 1 min.

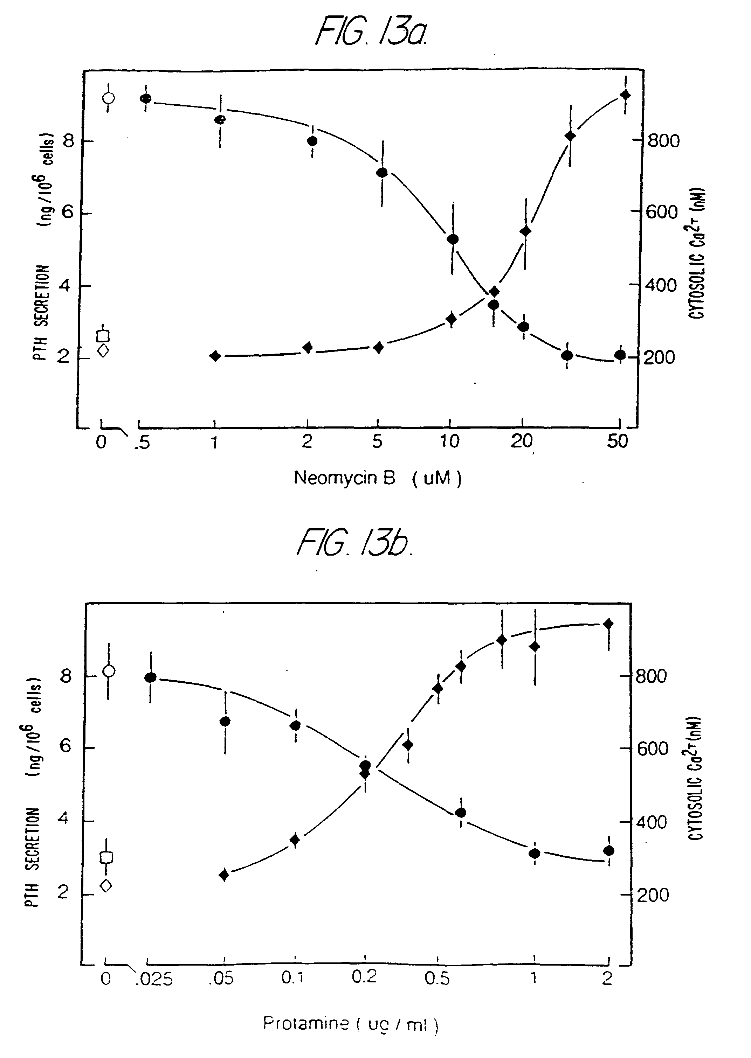

Fig. 13 is a graphical representation showing that neomycin B or protamine inhibit PTH secretion at concentrations which evoked increases in [Ca²⁺]ᵢ in bovine parathyroid cells. Cells were incubated with the indicated concentrations of organic polycation for 30 min. in the presence of 0.5 mM extracellular Ca²⁺. Open symbols: control responses for PTH secretion in the presence of 0.5 (circles) or 2 mM (diamonds) extracellular Ca²⁺. Values for [Ca²⁺]ᵢ are diamond symbols. Bovine cells were used in the experiments with protamine and human (adenoma) parathyroid cells were used in the experiments with neomycin B. Each point is the mean ± SEM of 3 experiments.

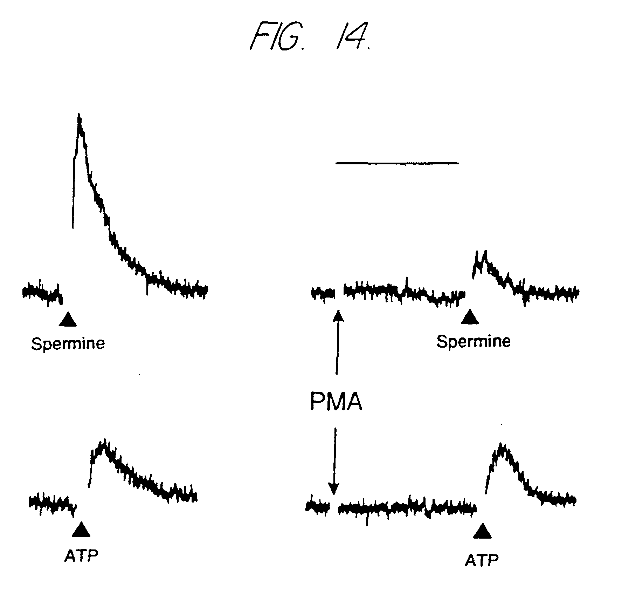

Fig. 14 is a graphical representation showing the preferential inhibitory effects of PMA on cytosolic Ca²⁺ transients elicited by spermine in bovine parathyroid cells. Initial [Ca²⁺] was 0.5 mM; spermine (200 µM) or ATP (50 µM) were added as indicated. Bar = 1 min.

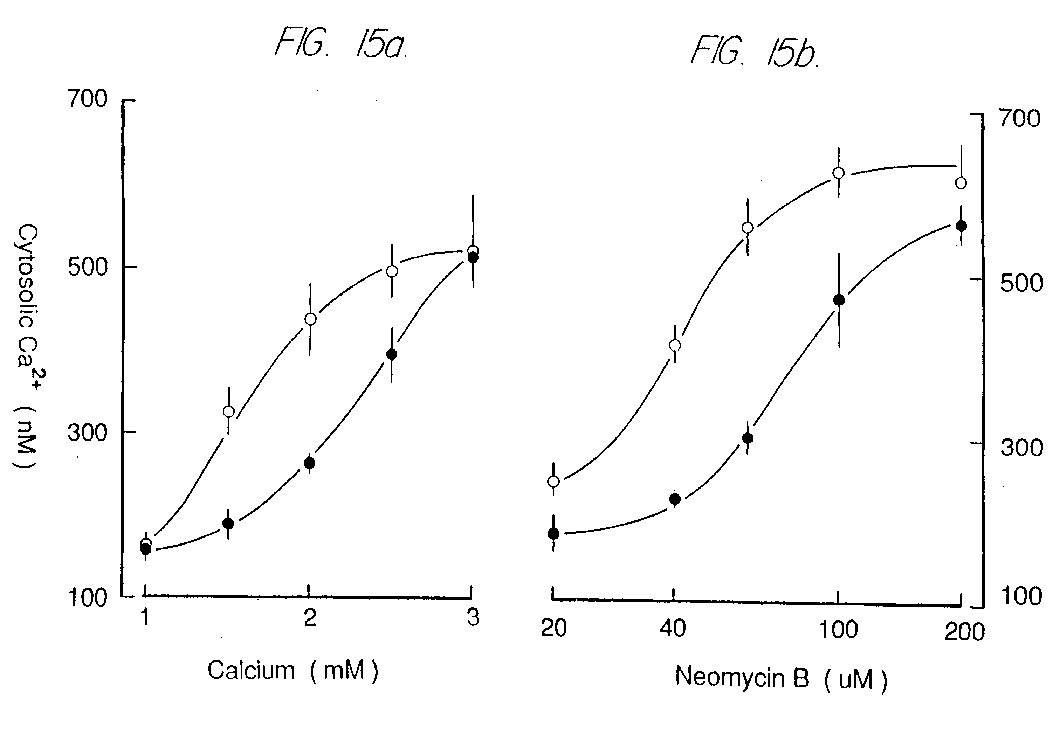

Fig. 15 is a graphical representation showing that PMA shifts to the right the concentration-response curves for extracellular Ca²⁺- and neomycin B-induced increases in [Ca²⁺]ᵢ in bovine parathyroid cells. Cells were pretreated with PMA for 1 min. before increasing [Ca²⁺] or before adding neomycin B as indicated. Each point is the mean ± SEM of 3 to 5 experiments.

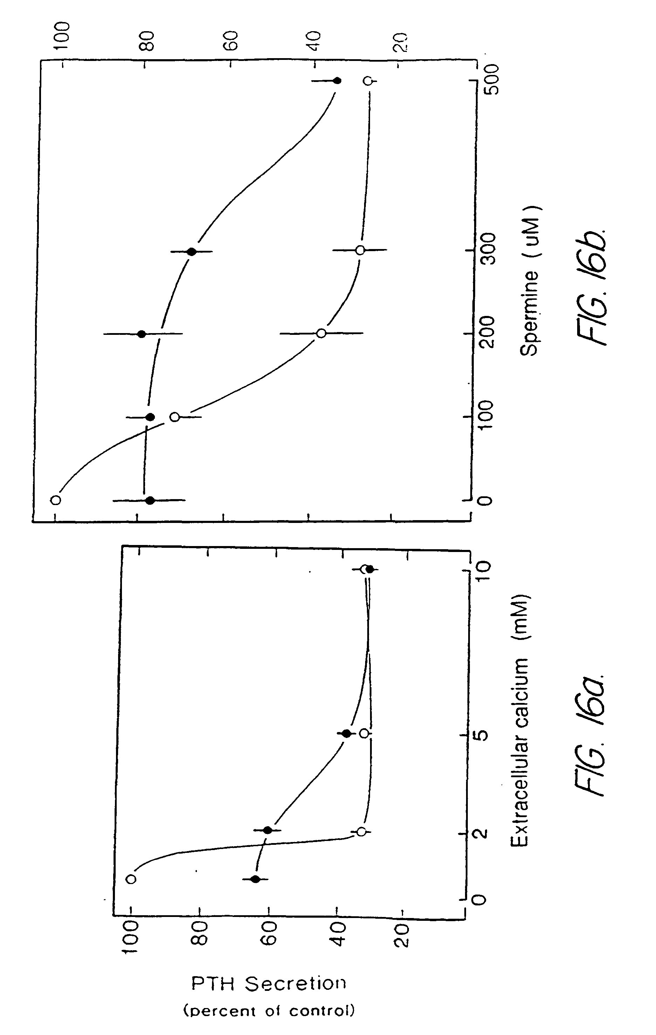

Fig. 16 is a graphical representation showing that PMA shifts to the right the concentration-response curves for extracellular Ca²⁺- and spermine-induced inhibition of PTH secretion in bovine parathyroid cells. Cells were incubated with the indicated [Ca²⁺] and spermine for 30 min. in the presence (closed circles) or absence (open circles) of 100 nM PMA. Each point is the mean ± SEM of 3 experiments.

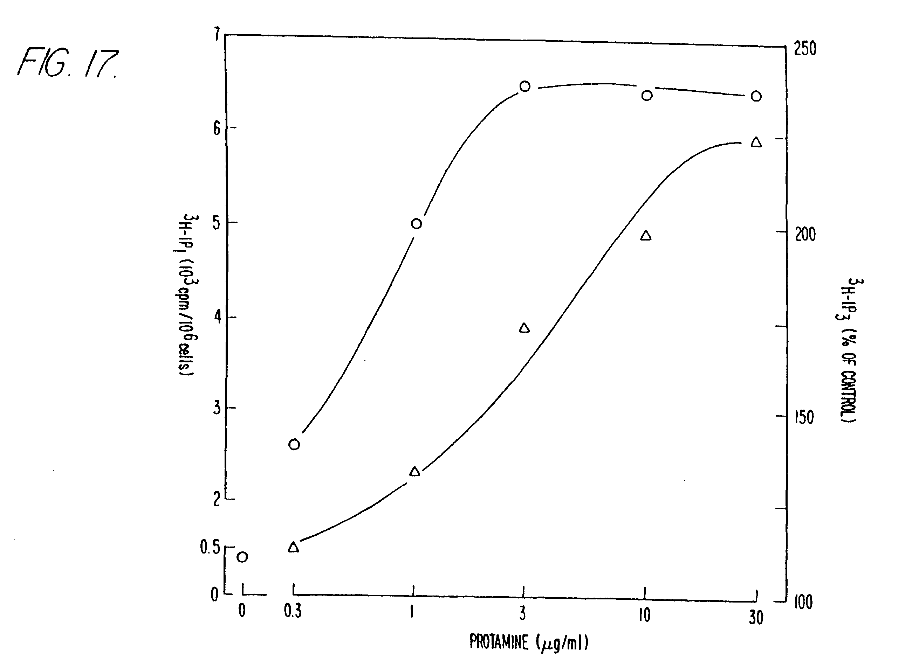

Fig. 17 is a graphical representation showing that protamine increases the formation of inositol phosphates in bovine parathyroid cells. Parathyroid cells were incubated overnight in culture media containing 4 µCi/ml ³H―myo―inositiol, washed, and incubated with the indicated concentration of protamine at 37°. After 30 sec. the reaction was terminated by the addition of CHCl₃:MeOH:HCl and IP₁ (circles) and IP₃ (triangles) separated by anion exchange chromatography. Each point is the mean of 2 experiments, each performed in triplicate.

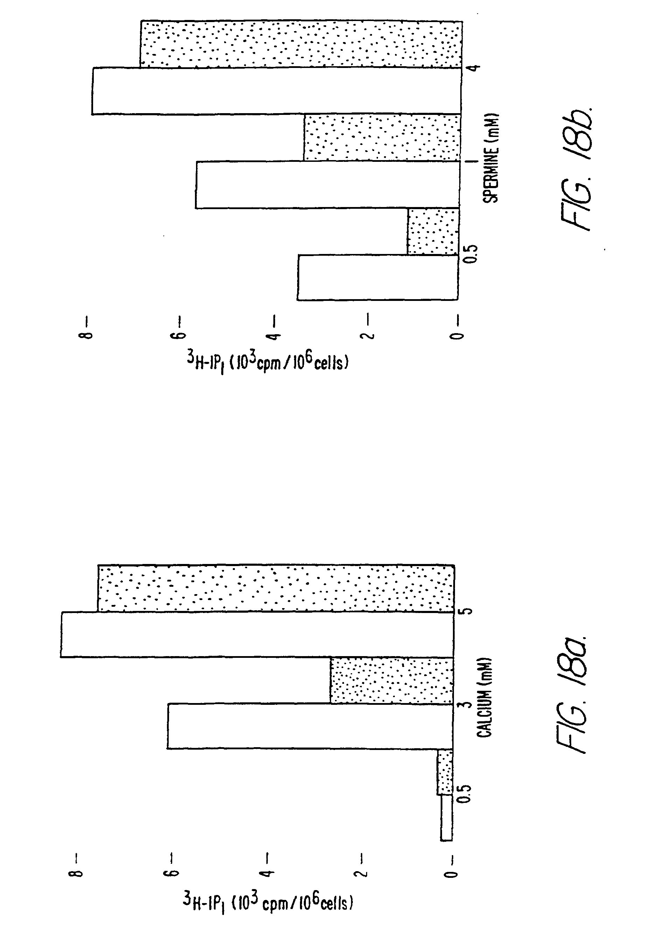

Fig. 18 is a graphical representation showing that PMA depresses the formation of IP₁ evoked by extracellular Ca²⁺ or spermine in bovine parathyroid cells. ³H-myo-inositol- labeled cells were exposed to the indicated [Ca²⁺] or spermine for 30 sec. before terminating the reaction and determining IP₁ by anion exchange chromatography. Hatched columns: Cells were pretreated with PMA (100 nM) for 5 min. before increasing [Ca²⁺] or adding spermine. Each value is the mean of 2 experiments, each performed in triplicate.

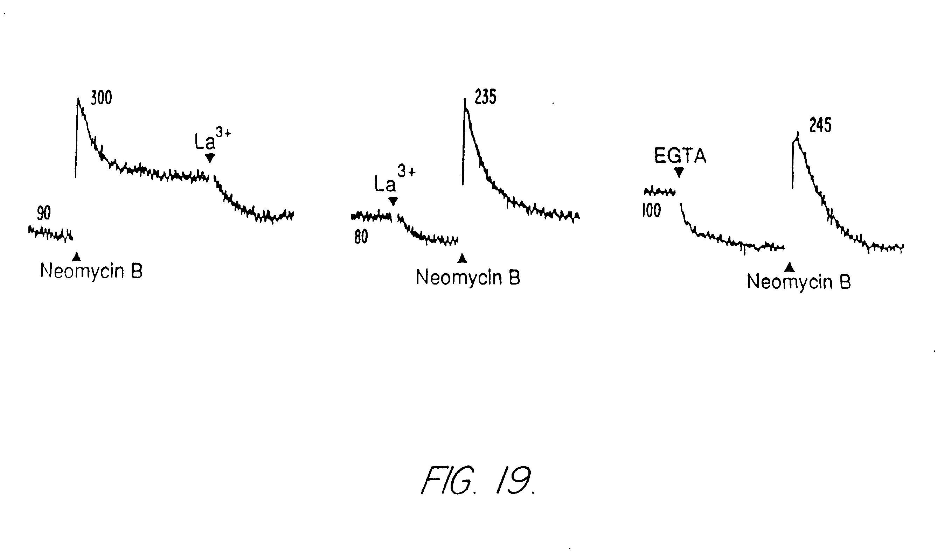

Fig. 19 is a graphical representation showing transient and sustained increases in [Ca²⁺]ᵢ elicited by neomycin B in human (adenoma) parathyroid cells. [Ca²⁺] was 0.5 mM. (a) The sustained increase in [Ca²⁺]ᵢ elicited by neomycin B (10 µM) was depressed by La³⁺. (b) The transient increase in [Ca²⁺]ᵢ evoked by neomycin B was unaffected by La³⁺. (c) Transient increases in [Ca²⁺]ᵢ persisted in the absence of extracellular Ca²⁺.

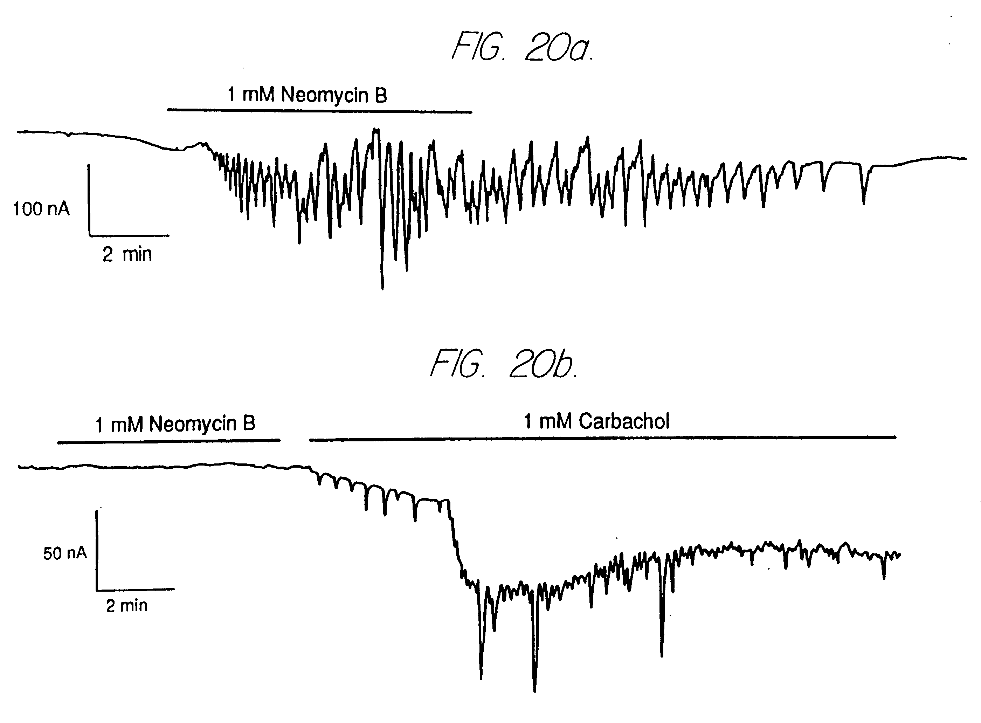

Fig. 20 is a graphical representation showing that neomycin B evokes oscillating increases the Cl⁻ current in Xenopus oocytes expressing the Ca²⁺ receptor. Upper trace from an oocyte three days after injection with human (hyperplastic) parathyroid cell poly(A)⁺-mRNA. Lower trace from an oocyte injected with water. Neomycin B failed to elicit a response in five water-injected oocytes and carbachol elicited a response in one, which is shown. In both traces, the holding potential was -76 mV.

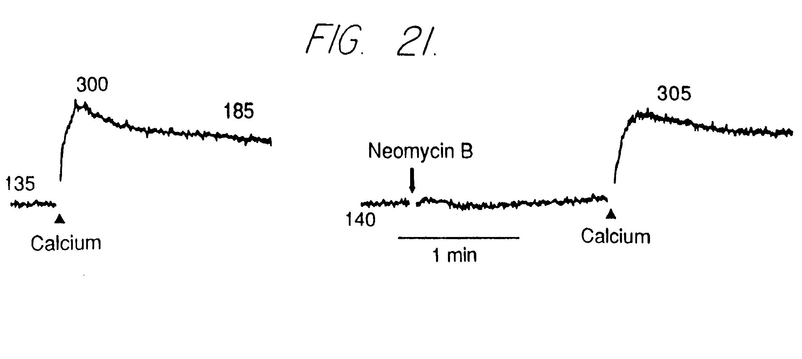

Fig. 21 is a graphical representation showing that neomycin B fails to affect basal or evoked increases in C-cells. Control, left trace: Fura-2- loaded rMTC 6-23 cells were initially bathed in buffer containing 1 mM Ca²⁺ before increasing [Ca²⁺] to 3 mM. Right trace: pretreatment with 5 mM neomycin B.

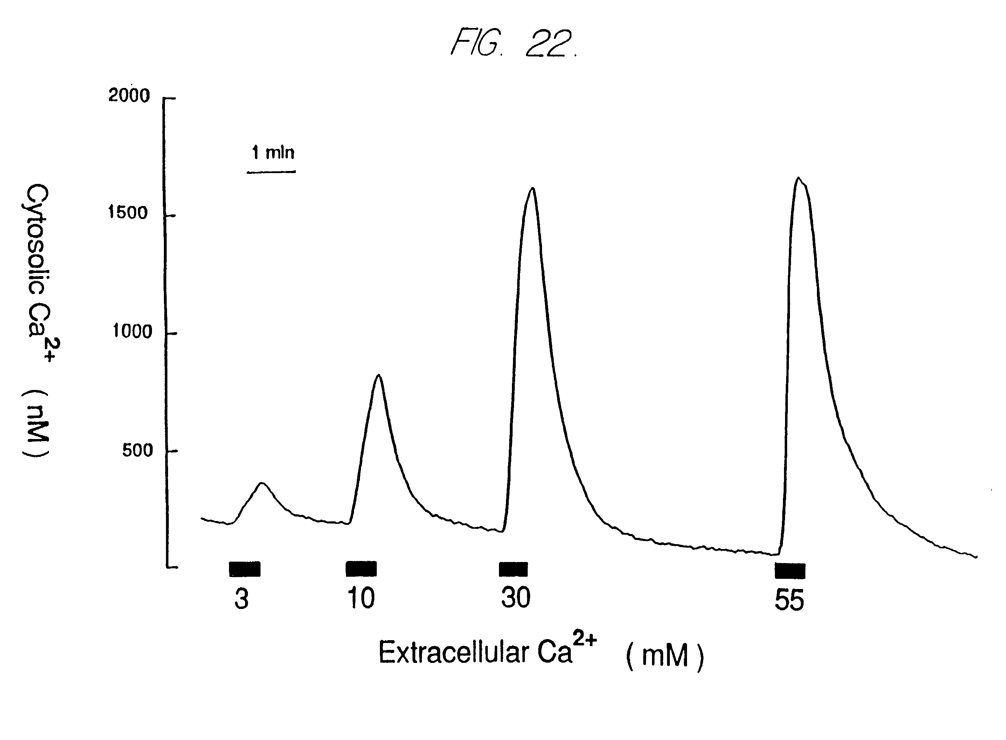

Fig. 22 is a graphical representation showing that extracellular Ca²⁺ evokes increases in [Ca²⁺]ᵢ in rat osteoclasts. Microfluorimetric recording in a single rat osteoclast loaded with indo-1 and superfused for the indicated times (bars) with buffer containing the indicated [Ca²⁺]. Normal buffer, superfused between the bars, contained 1 mM Ca²⁺.

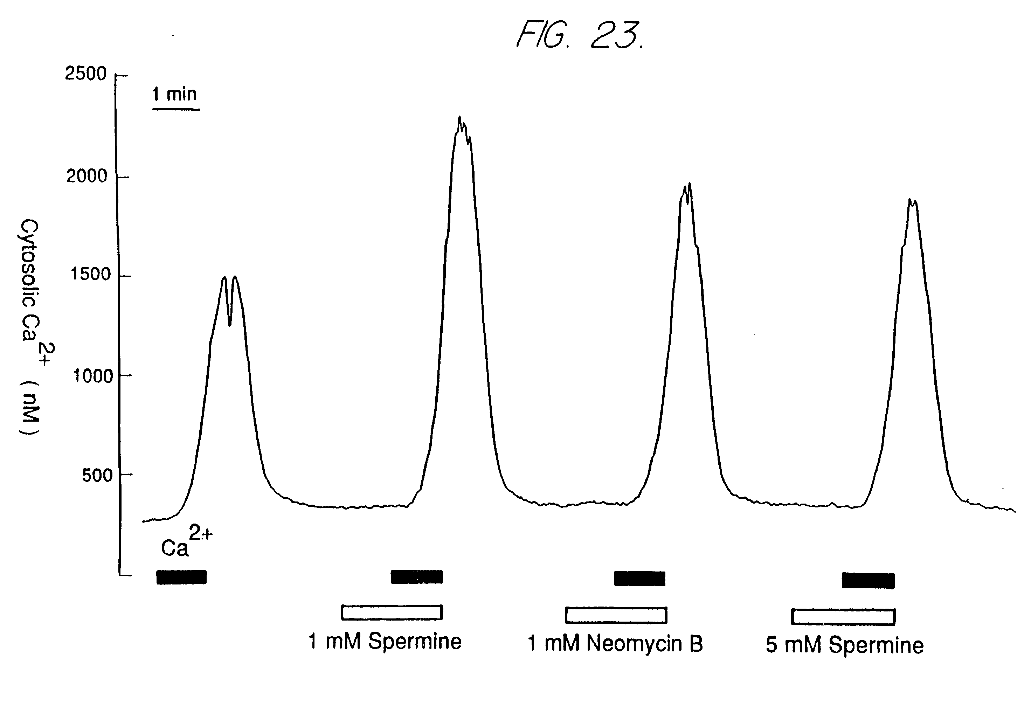

Fig. 23 is a graphical representation showing that spermine or neomycin B fail to evoke increases in [Ca²⁺]ᵢ in rat osteoclasts. An indo-1-loaded osteoclast was superfused with the indicated concentration of spermine or neomycin B (open bars) alone or together with 20 mM Ca²⁺ (solid bars).

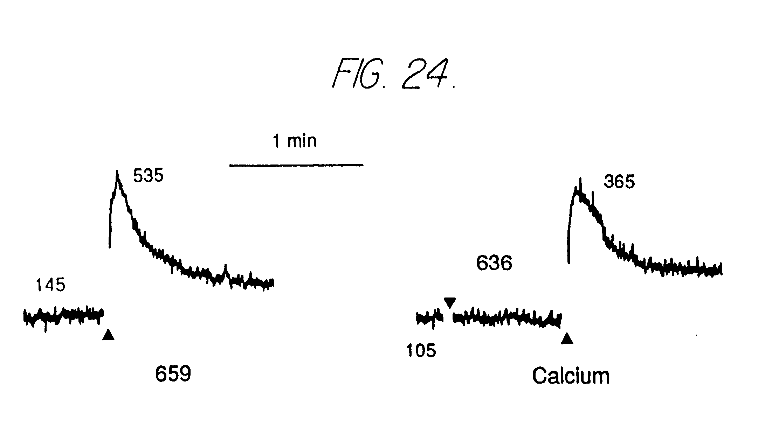

Fig. 24 is a graphical representation showing the differential effects of argiotoxin (shown as argiopine in the figure, structures also shown in Fig. 1) 659 and argiotoxin 636 on [Ca²⁺]ᵢ in bovine parathyroid cells. The initial [Ca²⁺] was 0.5 mM and was increased to 1.5 mM where indicated (right trace)N Where indicated, argiotoxin 659 (300 µM) or argiotoxin 636 (400 µM) was added.

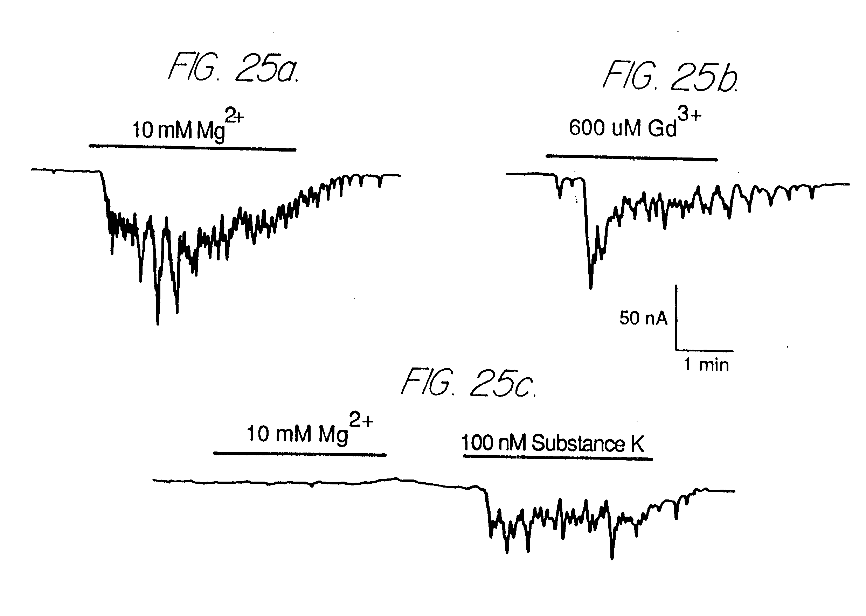

Fig. 25 is a graphical representation showing that extracellular Mg²⁺ or Gd³⁺ evoke oscillatory increases in Cl⁻ current in Xenopus oocytes injected with bovine parathyroid cell poly(A)⁺-mRNA. In trace (a), the concentration of extracellular Ca²⁺ was < 1 µM and in trace (b), 0.7 mM. Trace (c) shows that extracellular Mg²⁺ fails to elicit a response in an oocyte injected only with the mRNA for the substance K receptor, although superfusion with substance K evokes a response. Holding potential was -70 to -80 mV.

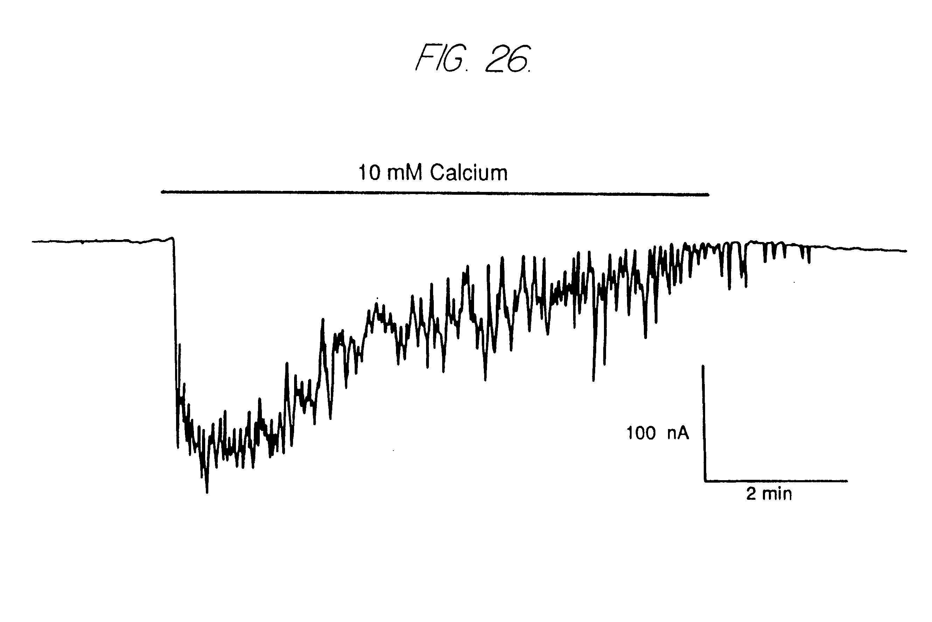

Fig. 26 is a graphical representation showing that extracellular Ca²⁺ elicits oscillatory increases in Cl⁻ current in Xenopus oocytes injected with human (hyperplastic) parathyroid tissue poly(A)⁺-mRNA. The oocyte was tested for responsivity to extracellular Ca²⁺ three days after injection of 50 ng poly(A)⁺-mRNA. Holding potential was -80 mV.

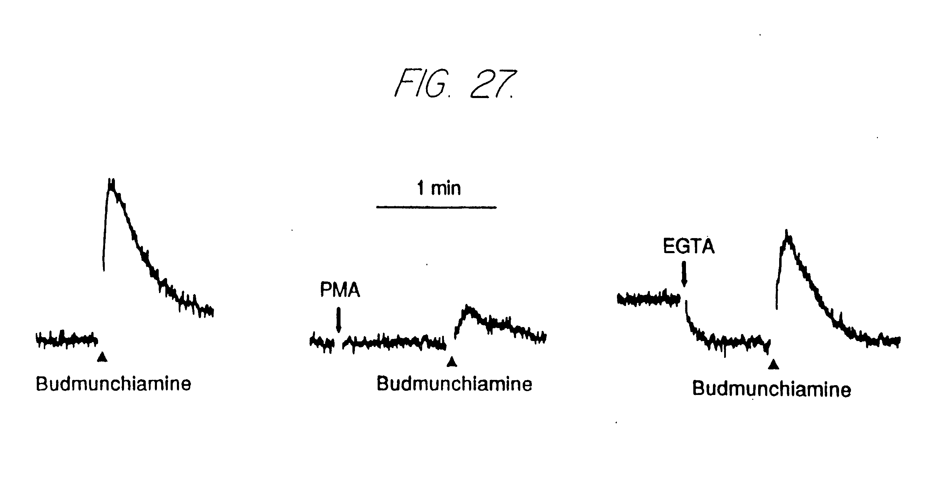

Fig. 27 is a graphical representation showing the mobilization of intracellular Ca²⁺ in bovine parathyroid cells elicited by budmunchiamine. Budmunchiamine (300 µM, structure also shown) was added where indicated.

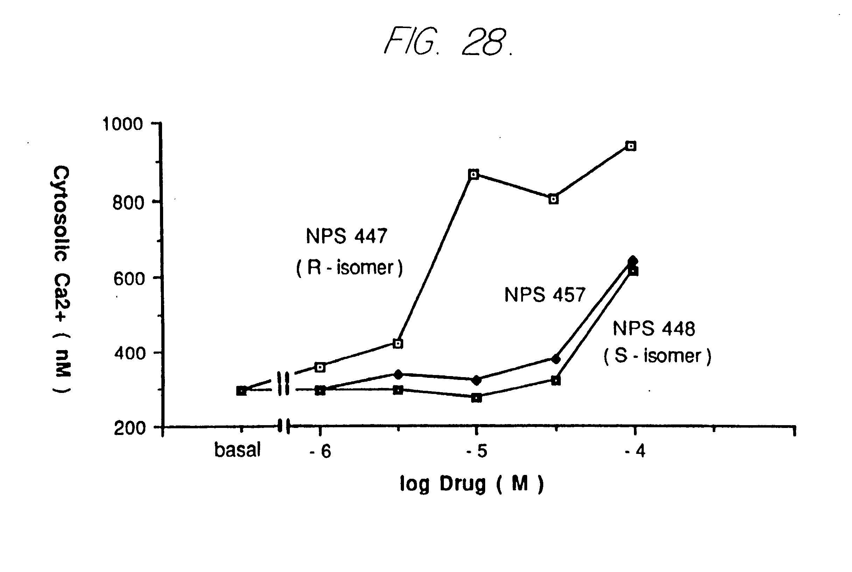

Fig. 28 is a graphical representation showing that the ability to mobilize intracellular Ca²⁺ in parathyroid cells is stereospecific. Bovine parathyroid cells loaded with fura-2 were initially suspended in buffer containing 0.5 mM extracellular Ca²⁺ before the addition of the indicated concentration of each molecule.

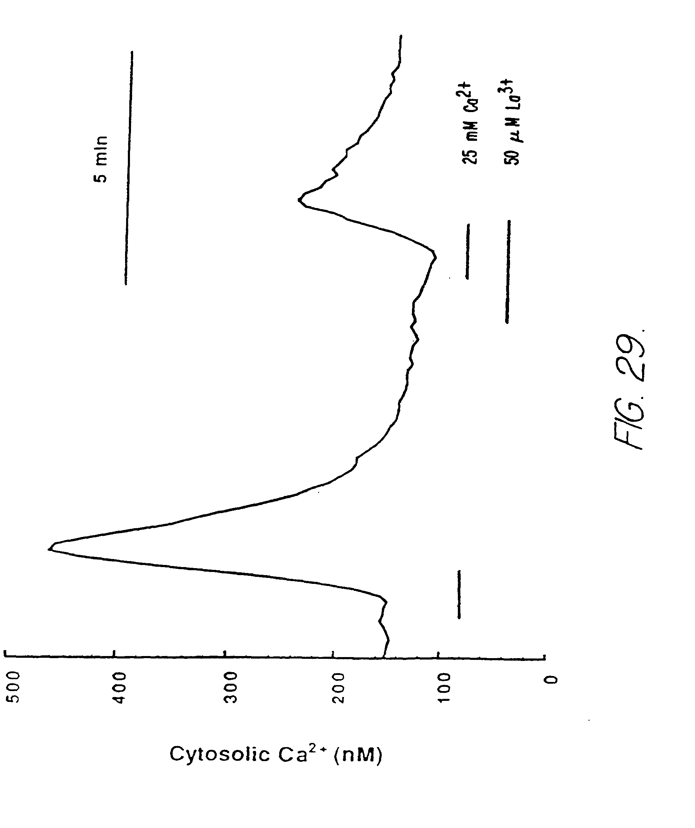

. Fig. 29 is a graphical representation showing effects of La³⁺ on [Ca²⁺]ᵢ in osteoclasts. A representative trace from a single rat osteoclast loaded with indo-1 is shown. At low concentrations, La³⁺ partially blocks increases in [Ca²⁺]ᵢ, elicited by extracellular Ca²⁺.

Figs. 30A and B are graphical representations showing the mobilization of intracellular Ca²⁺ elicited by extracellular Mn²⁺ in rat osteoclasts. Extracellular Mn²⁺ evokes concentration-dependent increases in [Ca²⁺]ᵢ (Fig. 30A) that persist in the absence of extracellular Ca²⁺ (Fig. 30B).

Figs. 31A and 31B are graphical representations showing mobilization of [Ca²⁺]ᵢ in rat osteoclasts elicited by a molecule termed NPS 449 (see Fig. 38). Isolated rat osteoclasts loaded with indo-1 were superfused with the indicated concentrations of NPS 449 in the presence (Fig. 31A) or absence (Fig. 31B) of 1 mM extracellular CaCl₂.

Fig. 32 is a graphical representation showing the mobilization of intracellular Ca²⁺ in C-cells evoked by NPS 019 (see Fig. 1). rMTC 6-23 cells were loaded with fura-2 and bathed in buffer containing 0.5 mM [Ca²⁺]. Where indicated, NPS 019 was added to a final concentration of 10 µM. Representative traces show that the transient increase in [Ca²⁺]ᵢ elicited by NPS 019 is refractory to inhibition by La³⁺ (middle trace) and persists in the absence of extracellular Ca²⁺ (right trace).

Fig. 33 is a graphical representation showing that NPS 456 (Fig. 36) evokes oscillatory increases in Cl⁻ current in Xenopus oocytes which have been injected with bovine parathyroid cell poly(A)⁺-mRNA.

Fig. 34 is a graphical representation showing that extracellular Ca²⁺ evokes oscillatory increases in Cl⁻ current in Xenopus oocytes which have been injected with human osteoclast mRNA. The oocyte was tested for responsivity to extracellular Ca²⁺ three days after injection of 50 ng of total poly(A)⁺ mRNA.

Fig. 35 is a graphical representation showing that the parathyroid cell Ca²⁺ receptor is encoded by mRNA in a size range of 2.5-3.5 kb. Bovine parathyroid cell poly(A)⁺ -mRNA was size fractionated on glycerol gradients and pooled into ten fractions. Each fraction was injected (50 ng/fraction) separately into Xenopus oocytes. After three days, the oocytes were examined for their ability to respond to neomycin B (10mM) with oscillatory increases in the Cl⁻ current.

Fig. 36 shows the chemical structures of molecules derived from diphenylpropyl-α-phenethylamine illustrating a family of molecules which were prepared and screened to find the useful molecules of the invention.

Fig. 37 is a graphical representation showing that NPS 021 is a calcilytic compound that blocks the effects of extracellular Ca²⁺ on [Ca²⁺]ᵢ in bovine parathyroid cells. Cells were initially bathed in buffer containing 0.5 mM CaCl₂ and, where indicated, the [Ca²⁺] was increased to a final of 2mM (left trace). The addition of NPS 021 (200 µM) caused no change in [Ca²⁺]ᵢ but inhibited the increase in [Ca²⁺]ᵢ elicited by extracellular Ca²⁺ (right trace).

Fig. 38 is a graph showing in vivo Ca²⁺ response to NPS R,S-467.

Fig. 39 is a graph showing in vivo PTH response to NPS R,S-467.

Fig. 40 is a graph showing in vivo serum Ca²⁺ response to 25 mg/kg NPS R,S-467.

Fig. 41 is a graph showing the in vivo response of [Ca²⁺]ᵢ in bovine parathyroid cells to different enantiomers of NPS 467.

Fig. 42 is a graph showing the in vivo response of serum Ca²⁺ in rats to different enantiomers of NPS 467.

Fig. 43a depicts a reaction scheme for the preparation of fendiline or fendiline analogs or derivatives depicted in Figure 36. Fig. 43b depicts a reaction scheme for the synthesis of NPS 467.

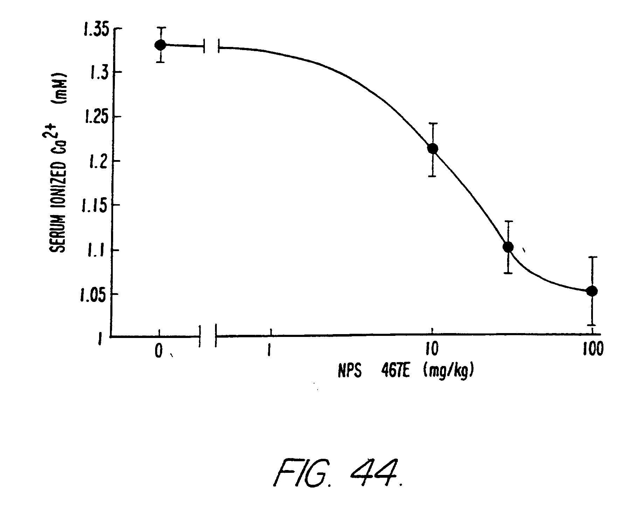

Fig. 44 depicts a dose-response curve showing that NPS 467 lowers serum ionized calcium in rats when administered orally.

Fig. 45 is a restriction map of the plasmid containing BoPCaR 1, deposited with the ATCC under accession number ATCC 75416.

Fig. 46 is a restriction map of BoPCaR 1.

Fig. 47 is a nucleotide sequence corresponding (about 90% accurate) to the 2.2 Kbfragment of BoPCaR 1 sequenced.

### Calcimimetic and Calcilytic Molecules

Calcimimetic and calcilytic molecules useful in the invention are generally described above. These molecules can be readily identified using screening procedures to define molecules which mimic or antagonize the activity of Ca²⁺ at Ca²⁺ receptors. Examples of such procedures are provided below. These examples are not limiting in the invention but merely illustrate methods which are readily used or adapted by those skilled in the art.

Generally, calcimimetic and calcilytic molecules are identified by screening molecules which are modelled after those described below (called lead molecules). As can be seen below there are several specific calcimimetics and calcilytics useful at various Ca²⁺ receptors. Derivative molecules are readily designed by standard procedures and tested in one of many protocols known to those skilled in the art. Many molecules may be screened easily to identify the most useful in this invention.

Organic cationic molecules which mimic or antagonize the actions of Ca²⁺ in other systems contain the requisite structure for activity on a Ca²⁺ receptor. Rational design of other useful molecules involves the study of a molecule known to be calcimimetic or calcilytic and then modifying the structure of the known molecule. For example, polyamines are potentially calcimimetic since spermine mimics the action of Ca²⁺ in several in vitro systems. Results show that spermine does indeed cause changes in [Ca²⁺]ᵢ and PTH secretion reminiscent of those elicited by extracellular di- and trivalent cations (see below). Conversely, Ga³⁺ antagonizes the effects of Gd³⁺ on the bovine parathyroid calcium receptor(s). The experiments outlined below are therefore aimed at demonstrating that this phenomenology, obtained with spermine, involves the same mechanisms used by extracellular Ca²⁺. To do this, the effects of spermine on a variety of physiological and biochemical parameters which characterize activation of the Ca²⁺ receptor were assessed. Those molecules having similar effects are useful in this invention and can be discovered by selecting or making molecules having a structure similar to spermine. Once another useful molecule is discovered this selection process can be readily repeated.

For clarity, below is provided a specific series of screening protocols to identify such useful molecules which are active at a parathyroid cell Ca²⁺ receptor, or which act as agonists or antagonists of the cellular response to changes in [Ca²⁺]. Equivalent assays can be used for molecules active at other Ca²⁺ receptors or other inorganic-ion receptors, or which otherwise mimic or antagonize cellular functions regulated by [Ca²⁺] or other ions. These assays exemplify the procedures which are useful to find molecules, including calcimimetic molecules, of this invention. Equivalent procedures can be used to find lytic molecules, including calcilytic molecules, by screening for those molecules most antagonistic to the actions of the ion, including extracellular Ca²⁺. In vitro assays can be used to characterize the selectivity, saturability, and reversibility of these mimetics and lytics by standard techniques.

### Screening Procedure

Generally, bovine parathyroid cells loaded with fura-2 are initially suspended in buffer containing 0.5 mM CaCl₂. The test substance is added to the cuvette in a small volume (5-15 µl) and any change in the fluorescence signal noted. Cumulative increases in the concentration of the test substance are made in the cuvette until some predetermined concentration is achieved or changes in fluorescence noted. If no changes in fluorescence are noted, the molecule is considered inactive and no further testing is performed. In initial studies, e.g., with polyamine-type molecules, molecules were tested at concentrations as high as 5 or 10 mM. As more potent molecules are now known (see below), the ceiling concentration is lowered. For example, newer molecules are tested at concentrations up to 500 µM or less. If no changes in fluorescence are noted at this concentration, the molecule can be considered inactive.

Molecules causing increases in [Ca²⁺]ᵢ are subjected to additional testing. The two essential characteristics of the molecule important for its consideration as a calcimimetic molecule are the mobilization of intracellular Ca²⁺ and sensitivity to PKC activators. Molecules causing the mobilization of intracellular Ca²⁺ in a PMA-sensitive manner have invariably been found to be calcimimetic molecules and to inhibit PTH secretion. Additional testing can, if needed, be performed to solidify this belief. Typically, all the various tests for calcimimetic or calcilytic activity (see above) are not performed. Rather, if a molecule causes the mobilization of intracellular Ca²⁺ in a PMA-sensitive manner, it is advanced to screening on human parathyroid cells. For example, measurements of [Ca²⁺]ᵢ are performed to determine the EC₅₀, and to measure the ability of the molecule to inhibit PTH secretion in human parathyroid cells which have been obtained from patients undergoing surgery for primary or secondary hyperparathyroidism. The lower the EC₅₀ or IC₅₀ the more potent the molecule as a calcimimetic or calcilytic.

Measuring [Ca²⁺]ᵢ with fura-2 provides a very rapid means of screening new organic molecules for activity. In a single afternoon, 10-15 molecules can be examined and their ability to mobilize intracellular Ca²⁺ (or not) assessed. The sensitivity of any observed increase in [Ca²⁺]ᵢ to depression by PMA can also be assessed. Moreover, a single cell preparation can provide data on [Ca²⁺]ᵢ, cyclic AMP levels, IP₃ and PTH secretion. A typical procedure is to load cells with fura-2 and then divide the cell suspension in two; most of the cells are used for measurement of [Ca²⁺]ᵢ and the remainder are incubated with molecules to assess their effects on cyclic AMP and PTH secretion. Because of the sensitivity of the radioimmunoassays for cyclic AMP and PTH, both variables can be determined in a single incubation tube containing 0.3 ml cell suspension (about 500,000 cells). Measurements of inositol phosphates are a time-consuming aspect of the screening. However, ion-exchange columns eluted with chloride (rather than formate) provide a very rapid means of screening for IP₃ formation since rotary evaporation (which takes around 30 hrs) is not required. This method allows processing of nearly 100 samples in a single afternoon. Those molecules that prove interesting, as assessed by measurements of [Ca²⁺]ᵢ, cyclic AMP, IP₃, and PTH are then subjected to a more rigorous analysis by examining formation of various inositol phosphates and assessing their isomeric form by HPLC.

Interesting molecules detected in these protocols are then assessed for specificity, e.g., by examining their effects on [Ca²⁺]ᵢ in calcitonin-secreting C-cells using, e.g., the rat MTC 6-23 cell line.

The following is illustrative of methods useful in these screening procedures. Examples of typical results for various test calcimimetic or calcilytic molecules are provided in Figs. 2-34.

### Parathyroid Cell Preparation

Parathyroid glands were obtained from freshly slaughtered calves (12-15 weeks old) at a local abattoir and transported to the laboratory in ice-cold parathyroid cell buffer (PCB) which contains (mM): NaCl, 126; KCl, 4; MgCl₂, 1; Na-HEPES, 20; pH 7.4; glucose, 5.6, and variable amounts of CaCl₂, e.g., 1.25 mM. Human parathyroid glands, obtained from patients undergoing surgical removal of parathyroid tissue for primary or uremic hyperparathyroidism (HPT), were treated similarly to bovine tissue. Glands were trimmed of excess fat and connective tissue and then minced with a fine scissors into approximate cubes of 2-3 mm. Dissociated parathyroid cells were prepared by collagenase digestion. Dissociated cells were then purified by centrifugation in Percoll buffer. The resultant parathyroid cell preparation was essentially devoid of red blood cells, adipocytes, and capillary tissue as assessed by phase contrast microscopy and Sudan black B staining. Dissociated and purified parathyroid cells were present as small clusters containing 5 to 20 cells. Cellular viability, as indexed by exclusion of trypan blue or ethidium bromide, was routinely 95%.

Although cells can be used for experimental purposes at this point, physiological responses (suppressibility of PTH secretion and resting levels of [Ca²⁺]ᵢ) are better after culturing the cells overnight. Primary culture also has the advantage that cells can be labeled with isotopes to near isotopic equilibrium, as is necessary for studies involving measurements of inositol phosphate metabolism (see below). After purification on Percoll gradients, cells were washed several times in a 1:1 mixture of Ham's F12-Dulbecco's modified Eagle's medium (GIBCO) supplemented with 50 µg/ml streptomycin, 100 U/ml penicillin, 5 µg/ml gentamicin and ITS⁺. ITS⁺ is a premixed solution containing insulin, transferrin, selenium, and bovine serum albumin (BSA)-linolenic acid (Collaborative Research, Bedford, MA). The cells were then transferred to plastic flasks (75 or 150 cm²; Falcon) and incubated at 37°C in a humid atmosphere of 5% CO₂. No serum is added to these overnight cultures, since its presence allows the cells to attach to the plastic, undergo proliferation, and dedifferentiate. Cells cultured under the above conditions were readily removed from the flasks by decanting, and show the same viability as freshly prepared cells.

### Measurement of Cytosolic Ca²⁺

Purified parathyroid cells were resuspended in 1.25 mM CaCl₂-2% BSA-PCB containing 1 µM fura-2-acetoxymethylester and incubated at 37°C for 20 min. The cells were then pelleted, resuspended in the same buffer lacking the ester, and incubated a further 15 min at 37°C. The cells were subsequently washed twice with PCB containing 0.5 mM CaCl₂ and 0.5% BSA and maintained at room temperature (about 20°C). Immediately before use, the cells were diluted five-fold with prewarmed 0.5 mM CaCl₂-PCB to obtain a final BSA concentration of 0.1%. The concentration of cells in the cuvette used for fluorescence recording was 1-2 x 10⁶/ml.

The fluorescence of indicator-loaded cells was measured at 37°C in a spectrofluorimeter (Biomedical Instrumentation Group, University of Pennsylvania, Philadelphia, PA) equipped with a thermostated cuvette holder-and magnetic stirrer using excitation and emission wavelengths of 340 and 510 nm, respectively. This fluorescence indicates the level of cytosolic Ca²⁺. Fluorescence signals were calibrated using digitonin (50 µg/ml, final) to obtain maximum fluorescence (Fₘₐₓ), and EGTA (10 mM, pH 8.3, final) to obtain minimal fluorescence (Fₘᵢₙ), and a dissociation constant of 224 nM. Leakage of dye is dependent on temperature and most occurs within the first 2 min after warming the cells in the cuvette; dye leakage increases only very slowly thereafter. To correct the calibration for dye leakage, cells were placed in the cuvette and stirred at 37°C for 2-3 min. The cell suspension was then removed, the cells pelleted, and the supernatant returned to a clean cuvette. The supernatant was then treated with digitonin and EGTA as above to obtain as estimate of dye leakage, which is typically 10-15% of the total Ca²⁺-dependent fluorescent signal. This estimate was subtracted from the apparent Fₘᵢₙ.

### Measurement of PTH Secretion

In most experiments, cells loaded with fura-2 were used in studies of PTH secretion. Loading parathyroid cells with fura-2 does not change their secretory response to extracellular Ca²⁺. Cells were suspended in PCB containing 0.5 mM CaCl₂ and 0.1% BSA. Incubations were performed in plastic tubes (Falcon 2058) containing 0.3 ml of the cell suspension with or without small volumes of CaCl₂ and/or organic polycations. After incubation at 37°C for various times (typically 30 min), the tubes were placed on ice and the cells pelleted at 2°C. Samples of the supernatant were brought to pH 4.5 with acetic acid and stored at -70°C. This protocol was used for both bovine and human parathyroid cells.

For bovine cells, the amount of PTH in sample supernatants was determined by a homologous radioimmunoassay using GW-1 antibody or its equivalent at a final dilution of 1/45,000. ¹²⁵I-PTH (65-84; INCSTAR, Stillwater, MN) was used as tracer and fractions separated by dextran-activated charcoal. Counting of samples and data reduction were performed on a Packard Cobra 5005 gamma counter.

For human cells, a commercially available radioimmunoassay kit (INS-PTH; Nichols Institute, Los Angeles, CA) which recognizes intact and N-terminal human PTH was used because GW-1 antibody recognizes human PTH poorly.

### Measurement of cyclic AMP

Cells were incubated as above for PTH secretion studies and at the end of incubation, a 0.15 ml sample was taken and transferred to 0.85 ml hot (70°C) water and heated at this temperature for 5-10 min. The tubes were subsequently frozen and thawed several times and the cellular debris sedimented by centrifugation. Portions of the supernatant were acetylated and cyclic AMP concentrations determined by radioimmunoassay.

### Measurement of Inositol Phosphate Formation

Membrane phospholipids were labeled by incubating parathyroid cells with 4 µCi/ml ³H-myo-inositol for 20-24 hrs. Cells were then washed and resuspended in PCB containing 0.5 mM CaCl₂ and 0.1% BSA. Incubations were performed in microfuge tubes in the absence or presence of various concentrations of organic polycation.for different times. Reactions were terminated by the addition of 1 ml chloroform/methanol/12 N HCl (200:100:1; v/v/v). Phytic acid hydrolysate (200 µl; 25 pg phosphate/tube) water was then added. The tubes were centrifuged and 600 µl of the aqueous phase was diluted into 10 ml water.

Inositol phosphates were separated by ion-exchange chromatography using AG1-X8 in either the chloride-or formate-form. When only IP₃ levels were to be determined, the chloride-form was used, whereas the formate form was used to resolve the major inositol phosphates (IP₃, IP₂, and IP₁). For determination of just IP₃, the diluted sample was applied to the chloride-form column and the column washed with 10 ml 30 mM HCl followed by 6 ml 90 mM HCl and the IP₃ eluted with 3 ml 500 mM HCl. The last eluate was diluted and counted. For determination of all major inositol phosphates, the diluted sample was applied to the formate-form column and IP₁, IP₂, and IP₃ eluted sequentially by increasing concentrations of formate buffer. The eluted samples from the formate columns were rotary evaporated, the residues brought up in cocktail, and counted.

The isomeric forms of IP₃ were evaluated by HPLC. The reactions were terminated by the addition of 1 ml 0.45 M perchloric acid and stored on ice for 10 min. Following centrifugation, the supernatant was adjusted to pH 7-8 with NaHCO₃. The extract was then applied to a Partisil SAX anion-exchange column and eluted with a linear gradient of ammonium formate. The various fractions were then desalted with Dowex followed by rotary evaporation prior to liquid scintillation counting in a Packard Tri-carb 1500 LSC.

For all inositol phosphate separation methods, appropriate controls using authentic standards were used to determine if organic polycations interfered with the separation. If so, the samples were treated with cation-exchange resin to remove the offending molecule prior to separation of inositol phosphates.

### Measurement of Cytosolic Ca²⁺ in C-cells

Neoplastic C-cells derived from a rat medullary thyroid carcinoma (rMTC 6-23) obtained from American Type Culture Collection (ATCC No. 1607) were cultured as monolayers in Dulbecco's Modified Eagle's medium (DMEM) plus 15% horse serum in the absence of antibiotics. For measurements of [Ca²⁺]ᵢ, the cells were harvested with 0.02% EDTA/0.05% trypsin, washed twice with PCB containing 1.25 mM CaCl₂ and 0.5% BSA, and loaded with fura-2 as described above for parathyroid cells. Measurements of [Ca²⁺]ᵢ were performed as described above with appropriate corrections for dye leakage.

### Measurement of [Ca²⁺]ᵢ in Rat Osteoclasts

Osteoclasts were obtained from 1-2 day old Sprague-Dawley rats using aseptic conditions. The rat pups were sacrificed by decapitation, the hind legs removed, and the femora rapidly freed of soft tissue and placed in prewarmed F-12/DMEM media (DMEM containing 10% fetal calf serum and antibiotics (penicillin-streptomycin-gentamicin; 100 U/ml-100 µg/ml-100 µg/ml)). The bones from two pups were cut lengthwise and placed in 1 ml culture medium. Bone cells were obtained by gentle trituration of the bone fragments with a plastic pipet and diluted with culture medium. The bone fragments were allowed to settle and equal portions (about 1 ml) of the medium transferred to a 6-well culture plate containing 25 mm glass coverslips. The cells were allowed to settle for 1 hr at 37°C in a humidified 5% CO₂-air atmosphere. The coverslips were then washed 3 times with fresh media to remove nonadherent cells. Measurements of [Ca²⁺]ᵢ in osteoclasts were performed within 6-8 hrs of removing nonadherent cells.

Cells attached to the coverslip were loaded with indo-1 by incubation with 5 µM indo-1 acetoxymethylester /0.01% Pluronic F28 for 30 min at 37°C in F-12/DMEM lacking serum and containing instead 0.5% BSA. The coverslips were subsequently washed and incubated an additional 15 min at 37°C in F-12/DMEM lacking ester before being transferred to a superfusion chamber mounted on the stage of a Nikon Diaphot inverted microscope equipped for microfluorimetry. Osteoclasts were easily identified by their large size and presence of multiple nuclei. The cells were superfused with buffer (typically PCB containing 0.1% BSA and 1 mM Ca²⁺) at 1 ml/min with or without test substance. The fluorescence emitted by excitation at 340 nm was directed through the video port of the microscope onto a 440 nm dichroic mirror and fluorescence intensity at 495 and 405 nm collected by photomultiplier tubes. The outputs from the photomultiplier tubes were amplified, digitized, and stored in an 80386 PC. Ratios of fluorescence intensity were used to estimate [Ca²⁺]ᵢ.

### Oocyte Expression

In additional studies, Xenopus oocytes injected with mRNA from bovine or human parathyroid cells were used in screening protocols, and Cl⁻ current measured as an indirect means of monitoring increases in [Ca²⁺]ᵢ. The following is an example to test the effect of neomycin.

Oocytes were injected with poly(A)⁺-enriched mRNA from human parathyroid tissue (hyperplastic glands from a case of secondary HPT). After 3 days, the oocytes were tested for their response to neomycin. Neomycin B evoked oscillatory increases in the Cl⁻ current which ceased upon superfusion with drug-free saline (see Fig. 20). Responses to neomycin B were observed at concentrations between 100 µM and 10 mM. To ensure that the response evoked by neomycin B was contingent upon injection of parathyroid mRNA, the effect of neomycin B on currents in water-injected oocytes was determined. In each of five oocytes examined, neomycin B (10 mM) failed to cause any change in the current. About 40% of oocytes are known to respond to carbachol, an effect mediated by an endogenous muscarinic receptor. In five oocytes examined, one showed inward currents in response to carbachol, and this is shown in the lower trace of Fig. 20. Thus, in cells expressing a muscarinic receptor coupled to increases in [Ca²⁺]ᵢ and Cl⁻ current, neomycin B fails to evoke a response. This shows that the response to neomycin B depends on expression of a specific protein encoded by parathyroid cell mRNA. It suggests quite strongly that in intact cells, neomycin B acts directly on the Ca²⁺ receptor to alter parathyroid cell function.

### Drug Design From Lead Molecules

Certain organic molecules mimic or antagonize the action of extracellular Ca²⁺ by acting at the Ca²⁺ receptor as shown herein. The molecules tested, however, are not necessarily suitable as drug candidates, but they serve to demonstrate that the hypothesis underlying Ca²⁺ receptor-based therapies is correct. These molecules can be used to determine the structural features that enable them to act on the Ca²⁺ receptor, and thus to select molecules useful in this invention.

An example of one such analytical procedure follows: This example is detailed in the examples below, but is used here to demonstrate the rationale that can be used to design useful molecules of this invention from lead molecules discussed herein. Those in the art will recognize the analytic steps defined in the example and that analogous analysis can be conducted on other lead molecules until the most desired calcimimetic or calcilytic is defined.

Other examples are also provided below. Together the data presented demonstrate that useful lead molecules will have aromatic groups which are preferably substituted at one or more positions, and may have branched or linear substituted or unsubstituted alkyl groups as desired. In addition, it is important to choose molecules of correct stereospecificity to ensure higher affinity for the desired Ca²⁺ receptor. These data thus point those in the art to appropriate lead molecules which can be derivatised to find optimum desired molecules of this invention, much as described below.

Although structurally diverse, molecules that are tested may have common features that can be studied. In this example, the correlation between net positive charge and potency in mobilizing intracellular Ca²⁺ was tested. Protamine (+21; EC₅₀ = 40 nM) was more effective than neomycin B (+6; EC₅₀ = 20 µM in human parathyroid cells and 40 µM in bovine parathyroid cells) which was more effective than spermine (+4; EC₅₀ = 150 µM) in causing the mobilization of [Ca²⁺]ᵢ in parathyroid cells. These results raise the question of whether positive charge alone determines potency, or if there are other structural features that contribute to activity on the Ca²⁺ receptor. This is important to determine at the outset because it profoundly impacts on the view that the Ca²⁺ receptor can be targeted with effective and specific therapeutic molecules. Thus, a variety of other organic polycations related to neomycin B and spermine can be studied to determine the relationship between the net positive charge of a molecule and its potency to mobilize intracellular Ca²⁺.

The first series of molecules studied were the aminoglycosides. The molecules were examined on bovine parathyroid cells and their EC₅₀'s for the mobilization of intracellular Ca²⁺ determined. For the aminoglycosides, the rank order of potency for eliciting cytosolic Ca²⁺ transients was neomycin B (EC₅₀ = 20 or 40 µM) > gentamicin (150 µM) > bekanamycin (200 µM) > streptomycin (600 µM). Kanamycin and lincomycin were without effect when tested at a concentration of 500 µM. The net positive charge on these aminoglycosides at pH 7.3 is neomycin B (+6) > gentamicin (+5) = bekanamycin (+5) > kanamycin (average +4.5) > streptomycin (+3) > lincomycin (+1). Within the aminoglycoside series, then, there is some correlation between net positive charge but it is not absolute, and kanamycin, which would be predicted to be more potent than streptomycin, is without activity.

Testing of various polyamines revealed additional and more marked discrepancies between net positive charge and potency. Three structural classes of polyamines were examined: (1) straight chain, (2) branched chain, and (3) cyclic. The structures of the polyamines tested are provided in Fig. 1. Amongst the straight chain polyamines, spermine (+4; EC₅₀ = 150 µM) was more potent than pentaethylenehexamine (+6; EC₅₀ = 500 µM) and tetraethylenepentamine (+5; EC₅₀ = 2.5 mM) even though the latter molecules have a greater net positive charge.

We synthesized some branched chain polyamines that have different numbers of secondary and primary amino groups and thus vary in net positive charge. Two of these molecules, NPS 381 and NPS 382, were examined for effects on [Ca²⁺]ᵢ in bovine parathyroid cells. NPS 382 (+8; EC₅₀ = 50 µM) was about twice as potent as NPS 381 (+10; EC₅₀ = 100 µM) even though it contains two fewer positive charges.

A similar discrepancy between positive charge and potency was noted in experiments with cyclic polyamines. For example, hexacyclen (+6; EC₅₀ = 20 µM) was more potent than NPS 383 (+8; EC₅₀ = 150 µM). The results obtained with these polyamines show that positive charge is not the sole factor contributing to potency.

Additional studies provided insights into the structural features of molecules that impart activity on the parathyroid cell Ca²⁺ receptor. One of the structurally important features is the intramolecular distance between the nitrogens (which carry the positive charge). Thus, spermine is 50-fold more potent than triethylenetetramine (EC₅₀ = 8 mM) in evoking increases in [Ca²⁺]ᵢ in bovine parathyroid cells yet both molecules carry a net positive charge of +4. The only difference in structure between these two polyamines is the number of methylenes separating the nitrogens: in spermine it is 3-4-3 whereas in triethylenetetramine it is 2-2-2. This seemingly minor change in the spacing between nitrogens has profound implications for potency and suggests that the conformational relationships of nitrogens within the molecule are critical. Supporting this are results obtained with hexacyclen and pentaethylenehexamine. The former molecule is simply the cyclic analog of the latter and contains the same number of methylenes between all nitrogens, yet the presence of the ring structure increases potency 25-fold. These results indicate that positive charge per se is not the critical factor determining the activity of an organic molecule on the Ca²⁺ receptor.

Another series of experiments reveals the importance of aromatic groups in determining activity on the Ca²⁺ receptor. The results were obtained with two arylalkylamines isolated from the venom of the spider Argiope lobata. These molecules, argiotoxin 636 and argiotoxin 659, have identical polycationic portions linked to different aromatic groups (Fig. 24). Argiotoxin 659 evoked transient increases in [Ca²⁺]ᵢ in bovine parathyroid cells when tested at concentrations of 100 to 300 µM. In contrast, argiotoxin 636 was without effect when tested at similar concentrations (Fig. 24). The only difference in structure between these two arylalkylamines is in the aromatic portion of the molecules: argiotoxin 659 contains a 4-hydroxyindole moiety whereas argiotoxin 636 contains a 2,4-dihydroxyphenyl group. The net positive charge on these two arylalkylamines is the same (+4), so their different potencies must result from the different aromatic groups. This shows that net positive charge alone does not determine potency. The real importance of these findings, however, is the discovery that aromatic groups contribute significantly to the ability of molecules to activate the Ca²⁺ receptor.

Agatoxin 489 (NPS 017) and Agatoxin 505 (NPS 015) both cause the mobilization of intracellular Ca²⁺ in parathyroid cells with EC₅₀'s of 6 and 22 µM, respectively. The only difference in the structure of these molecules is a hydroxyl group on the indole moiety (Fig. 1). This shows that substitutions on the aromatic region of the molecule can influence potency. This indicates that further lead molecules to be studied will include those molecules having substituted aromatic moieties.

The structural features to be varied systemati cally from lead molecules described herein include (1) net positive charge, (2) number of methylenes separating nitrogens, and (3) cyclic versions of, e.g., polyamines, with and without changes in methylene spacing and net positive charge. In addition systematic variations in the structure and location of aromatic groups can be examined, e.g., in a variety of arylalkylamines isolated from the venoms of wasps and spiders; and synthetic molecules can be prepared by the coupling of commercially available aromatic moieties to the argiotoxin polyamine moiety. The argiotoxin polyamine moiety can be readily coupled to any aromatic moiety containing a carboxylic acid. Thus, it is simple to systematically screen the hydroxy and methoxy derivatives of phenylacetic acid and benzoic acid as well as the hydroxyindoleacetic acid series. Analogs containing heteroaromatic functionalities can also be prepared and assessed for activity.

Comparisons of potency and efficacy among such molecules will reveal the optimal structure and location of the aromatic group at a constant positive charge.

One of the structural variations on the polyamine motif that seems to increase potency is the presence of the cyclic version of the straight chain- parent molecule. Budmunchiamine A, isolated from the plant Albizia amara, is a cyclic derivative of spermine (Fig. 1). The addition of budmunchiamine A to bovine parathyroid cells caused a rapid and transient increase in [Ca²⁺]ᵢ that persisted in the absence of extracellular Ca²⁺ and was blunted by pretreatment with PMA. It therefore causes the mobilization of intracellular Ca²⁺ in parathyroid cells, probably by acting on the Ca²⁺ receptor. It is about equipotent with spermine (EC₅₀ about 200 µM) yet carries one less positive charge (+3) than does spermine.

The results obtained with budmunchiamine A demonstrate the predictive power of the structure-activity studies and the novel structural information to be gained by testing natural products. Thus, screening of natural products, selected rationally on the basis of the structural information is readily performed e.g., molecules can be selected on the basis of well-established chemotaxonomic principles using appropriate data bases, such as Napralert. For example, macrocyclic polyamine alkaloids derived from papilionoid legumes related to Albizia, such as Pithecolobium and other plant-derived molecules can be screened.

Fig. 36 provides a second example of a series of molecules which were screened to determine useful molecules of this invention. These molecules were generally derived from fendiline and tested to determine their respective EC₅₀'s. Moreover, testing of related molecules, such as NPS 447 and NPS 448 reveals stereospecific effects of molecule structure. The most active compounds tested to date are the novel compounds designated NPS 467 and NPS 568 which have EC₅₀ values of less than 5 µM. Those in the art, by reviewing this series of molecules, can determine other suitable derivatives which can be tested in the invention.

These examples demonstrate the general design and screening process useful in this invention, and indicate that additional compound and natural product libraries can be screened as desired by those in the art to determine other useful lead molecules or novel molecules of this invention.

As discussed above, examples of molecules useful as calcimimetics include branched or cyclic polyamines, positively charged polyamino acids, and arylalkylamines. In addition, other positively charged organic molecules, including naturally occurring molecules and their analogs, are useful calcimimetics. These naturally occurring molecules and their analogs preferably have positive charge-to-mass ratios that correlate with those ratios for the molecules exemplified herein. (Examples include material isolated from marine species, arthropod venoms, terrestrial plants and fermentation broths derived from bacteria and fungi.) It is contemplated that one group of preferred naturally occurring molecules and analogs useful as calcimimetics will have a ratio of positive charge: molecular weight (in daltons) from about 1:40 to 1:200, preferably from about 1:40 to 1:100. More specific examples of such molecules are provided below.

### Polyamines

The polyamines useful as calcimimetics in this invention may be either branched or cyclic. Branched or cyclic polyamines potentially have higher calcimimetic activity than their straight-chain analogs. That is, branched or cyclic polyamines tend to have a lower EC₅₀ than their corresponding linear polyamines with the same effective charge at physiological pH (see Table 1).

**Table 1**

| Molecule | Net (+) Charge | EC₅₀(µM) |
|---|---|---|
| Neomycin | +6 | 20 or 40 |
| Hexacyclen | +6 | 20 |
| NPS 382 | +8 | 50 |
| NPS 381 | +10 | 100 |
| NPS 383 | +8 | 150 |
| Gentamicin | +5 | 150 |
| Spermine | +4 | 150 |
| Bekanamycin | +5 | 200 |
| Argiotoxin-659 | +4 | 300 |
| Pentaethylenehexamine (PEHA) | +6 | 500 |
| Streptomycin | +3 | 600 |
| Spermidine | +3 | 2000 |
| Tetraethylenepentamine (TEPA) | +5 | 2500 |
| 1,12-diaminododecane (DADD) | +2 | 3000 |
| Triethylenetramine (TETA) | +4 | 8000 |

"Branched polyamines" as used herein refers to a chain molecule consisting of short alkyl bridges or alkyl groups joined together by amino linkages, and also containing points at which the chain branches. These "branch points" can be located at either a carbon atom or a nitrogen atom, preferably at a nitrogen atom. A nitrogen atom branch point is typically a tertiary amine but it may also be quaternary. A branched polyamine may have 1 to 20 branch points, preferably 1 to 10 branch points.

Generally, the alkyl bridges and alkyl branches in a branched polyamine are from 1 to 50 carbon atoms in length, preferably from 2 to 6 carbon atoms. The alkyl branches may also be interrupted by one or more heteroatoms (nitrogen, oxygen or sulfur) or substituted with functional groups such as: halo, including fluoro, chloro, bromo, or iodo; hydroxy; nitro; acyloxy (R'COO-), acylamido (R'CONH-), or alkoxy (-OR'), where R' may contain from 1 to 4 carbon atoms. The alkyl branches may also be substituted with groups that are positively charged at physiological pH, such as amino or guanido. These functional substituents may add or change physical properties such as solubility to increase activity, delivery or bioavailability of the molecules.

The branched polyamines may have three or more chain and branch termination points. These termination points may be methyl groups or amino groups, preferably amino groups.

One preferred group of molecules is the group of branched polyamines having the formula:

H₂N-(CH₂)ⱼ-(NRᵢ-(CH₂)ⱼ)ₖ-NH₂

where k is an integer from 1 to 10, each j is the same or different and is an integer from 2 to 20, and each Rᵢ is the same or different and is selected from the group consisting of hydrogen and -(CH₂)ⱼ-NH₂, where j is as defined above, and at least one Rᵢ is not hydrogen.

Particularly preferred branched polyamines of this invention are the molecules N¹,N¹,N⁵,N¹⁰,N¹⁴,N¹⁴-hexakis-(3- aminopropyl) spermine and N¹,N¹,N⁵,N¹⁴,N¹⁴-tetrakis-(3-aminopropyl)spermine referred to as NPS 381 and NPS 382, respectively, in Figure 1.

"Cyclic polyamines" as used herein refer to heterocycles containing two or more heteroatoms (nitrogen, oxygen or sulfur), at least two of which are nitrogen atoms. The heterocycles are generally from about 6 to about 20 atoms in circumference, preferably from about 10 to about 18 atoms in circumference. The nitrogen heteroatoms are separated by 2 to 10 carbon atoms. The heterocycles may also be substituted at the nitrogen sites with aminoalkyl or aminoaryl groups (NH₂R-), wherein R is aminoaryl or a lower alkyl of 2 to 6 carbon atoms.

Particularly preferred cyclic polyamines of this invention are shown in Figure 1 as hexacyclen (1,4,7,10,13,16-hexaaza-cyclooctadecane) and NPS 383.

### Polyamino Acids

The polyamino acids useful in this invention may contain two or more positively charged amino acid residues at physiological pH. These positively charged amino acids include histidine, lysine and arginine. These polypeptides will vary in length from 2 to 800 amino acids in length, more preferably from 20 to 300 amino acids in length. These polypeptides may consist of a single repeating amino acid residue, or may have the variety of a naturally occurring protein or enzyme.

The amino acid residues comprising the polyamino acids may be any of the twenty naturally occurring amino acids, or other alternative residues. Alternative residues include, for example, the ositions of this invention may also contain components derivatized with a molecule or ion which acts as a label. A wide variety of labeling moieties can be used, including radioisotopes, chromophores, and fluorescent labels. Radioisotope labeling in particular can be readily detected in vivo. Radioisotopes may be coupled by coordination as cations in the porphyrin system. Useful cations include technetium, and indium. In the compositions, the positively charged molecule can be linked to or associated with a label.

### Methods of Synthesis

Strategies for the syntheses and the modification of polyamines involve the use of a variety of amine protecting groups (phthalimido, BOC, CBZ, benzyl, and nitrile) which can be removed selectively to construct functionalized molecules. The synthetic methods involved are modelled after those used to construct argiopines 636 and 659 and other arylalkylamines derived from spider venoms.

Chain extensions of 2-4 methylenes were typically accomplished by alkylation with the corresponding N-(bromoalkyl)phthalimide. A 1:1.2 mixture of amine to the bromoalkylphthalimide was refluxed in acetonitrile in the presence of 50% KF on Celite. Chain extensions were also accomplished by alkylation of a given amine with acrylonitrile or ethylacrylate. Reaction progress was monitored by TLC and intermediates purified on silica gel using combinations of dichloromethane, methanol, and isopropylamine. Final products were purified by cation exchange (HEMA-SB) and RP-HPLC (Vydac C-18). Purity and structure verification are accomplished by ¹H- and ¹³C-NMR and high-resolution mass spectrometry (EI, CI and/or FAB).

BOC protecting groups were added by the treatment of an amine (1° or 2°) with di-tert-butyl dicarbonate in dichloromethane in the presence of a catalytic amount of dimethylaminopyridine. Benzyl protecting groups were applied in one of two ways: (1) condensation of a 1° amine with benzaldehyde followed by sodium borohydride reduction or (2) alkylation of a 2° amine with benzylbromide in the presence of KF. Amide linkages and cyclizations were typically performed by the reaction of an amine (1° or 2°) with the N-hydroxysuccinimide ester of a given acid. This was accomplished directly (in the case of cyclizations) by treatment of the "amino acid" with dicyclohexylcarbodiimide under dilute conditions.

Deprotections of the phthalimido functionality were accomplished by reduction with hydrazine in refluxing methanol.. Deprotections of the BOC functionality were accomplished in anhydrous TFA. Deprotection of benzyl, nitrile, and CBZ protecting functionalities was accomplished by reduction in glacial acetic acid under 55 psi hydrogen in the presence of a catalytic amount of palladium hydroxide on carbon. Nitrile functionalities (in the presence of benzyl and CBZ groups) were selectively reduced under hydrogen in the presence of sponge Raney nickel.

Specifically, branched polyamines are typically prepared from simple diaminoalkanes of the formula NH₂-(CH₂)ₙ-NH₂, or simple polyamines such as spermidine or spermine. One of the two primary (terminal) amines is protected or "masked" with a protecting group such as BOC (t-butyloxycarbonyl), phthalimido, benzyl, 2- ethylnitrile (the Michael condensation production product of an amine and acrylonitrile), or amide. A typical reaction is the addition of a BOC protecting group by treatment with di-t-butyl-dicarbonate (BOC anhydride): 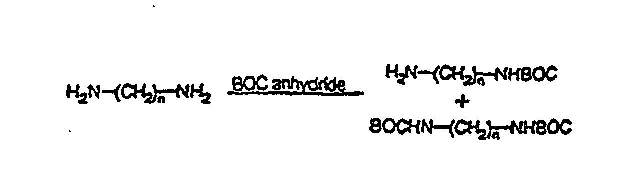 The monoprotected product is separated from the unprotected and diprotected products by simple chromatographic or distillation techniques.

The remaining free amine in the monoprotected product is then selectively alkylated (or acylated) with an alkylating (or acylating) agent. To ensure monoalkylation, the free amine- is partially protected by condensation with benzaldehyde followed by sodium borohydride reduction to form the N-benzyl derivative: 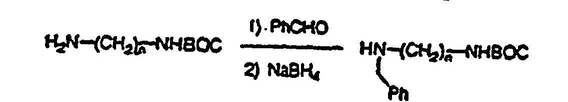 The N-benzyl derivative is then reacted with the alkylating agent. A typical alkylating agent is in an N-(bromoalkyl)phthalimide, which reacts as follows: 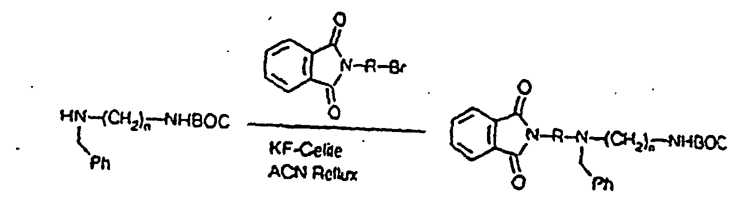 For example, N-(bromobutyl)phthalimide is used to extend or branch the chain with four methylene units. Alternatively, reaction with acrylonitrile followed by reduction of the cyano group will extend the chain by three methylenes and an amino group.

The protecting groups of the resulting chain-extended molecule can then be selectively cleaved to yield a new free amine. For example, trifluoroacetic acid is used to remove a BOC group; catalytic hydrogenation is used to reduce a nitrile functionality and remove a benzyl group; and hydrazine is used to remove phthalimido groups as follows:

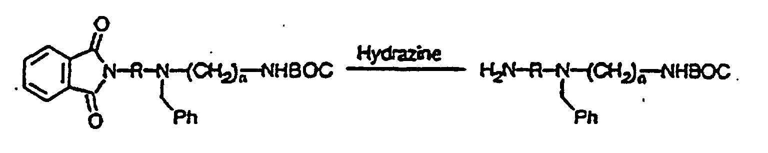

The new free amine may be alkylated (or acylated) further as above to increase the length of the polyamine. This process is repeated until the desired chain length and number of branches is obtained. In the final step, deprotection of the product results in the desired polyamine. However, further modifications may be effected at the protected end prior to deprotection in the following manner:

For example, prior to BOC-deprotection, the polyamine is acylated with the N-hydroxysuccinimide ester of 3,4-dimethoxyphenylacetic acid to yield a diprotected polyamine: 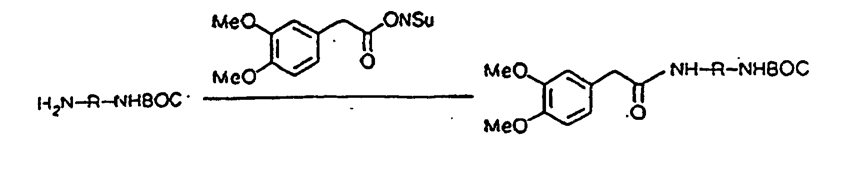 This ultimately will yield an arylalkyl polyamine. The BOC group can then be selectively removed with trifluoroacetic acid to expose the other amino terminus which can be extended as above.

Certain branched polyamines may be formed by simultaneously alkylating or acylating the free primary and secondary amines in a polyamine formed as above. For example, treatment of spermine with excess acrylonitrile followed by catalytic reduction yields the following:

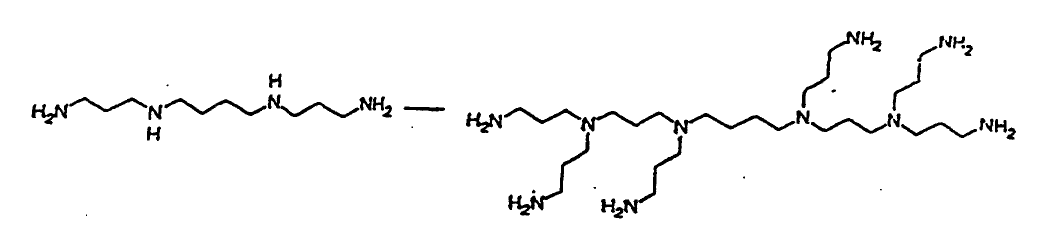

Cyclic polyamines may be prepared as above beginning with starting materials such as hexacylen (Aldrich Chem.).

The polyamino acids within the scope of the present invention can be made by recombinant techniques known in the art, or may be synthesized using standard solid-phase techniques known in the art. Solid-phase synthesis is commenced from the carboxy-terminal end of the peptide using an µ-amino protected amino acid. BOC protective groups can be used for all amino groups even through other protective groups are suitable. For example, BOC-lys-OH can be esterified to chloromethylated polystyrene resin supports. The polystyrene resin support is preferably a copolymer of styrene with about 0.5 to 2% divinylbenzene as a cross-linking agent which causes the polystyrene polymer to be completely insoluble in certain organic solvents. See Stewart et al., Solid-Phase Peptide Synthesis (1969), W.H. Freeman Co., San Francisco; and Merrifield, J. Am. Chem. Soc. (1963) 85:2149-2154. These and other methods of peptide synthesis are also exemplified by U.S. Patent Nos. 3,862,925; 3,842,067; 3,972,859; and 4,105,602.

The polypeptide synthesis may use manual techniques or automatically employing, for example, an Applied Biosystems 403A Peptide Synthesizer (Foster City, California) or a Biosearch SAM II automatic peptide synthesizer (Biosearch, Inc., San Rafael, California), following the instructions provided in the instruction manual supplied by the manufacturer.

The arylalkylamines of the invention are natural products isolated by known techniques, or synthesized as described in Jasys et al., Tetrahedron Lett. (1988) 29:6223-6226, and Nason et al., Tetrahedron Lett. (1989) 30:2337-2340.

One general protocol for preparation of fendiline (or fendiline analogs shown in Fig. 36) is as follows. In a 10 ml round bottom flask equipped with a magnetic stir bar and rubber septum, 1.0 mmole 3,3'- bisphenylpropylamine (or primary alkyl amine) in 2 ml ethanol was treated with 1.1 mmole phenol and 1.0 mmole acetophenone (or substituted acetophenone). To this was added 2.0 mmoles MgSO₄ and 1.0 mmole NaCNBH₃. This was stirred under a nitrogen atmosphere at room temperature (about 20°C) for 24 hrs. The reaction was poured into 50 ml ether and washed 3 times with 1 N NaOH and once with brine. The ether layer was dried with anhydrous K₂CO₃ and reduced in vacuo. The product was then purified by column chromatography or HPLC incorporating osilica stationary phase with combinations of CH₂Cl₂- Methanol- isopropylamine (typically 3% Methanol and 0.1% isopropylamine in methylene chloride).

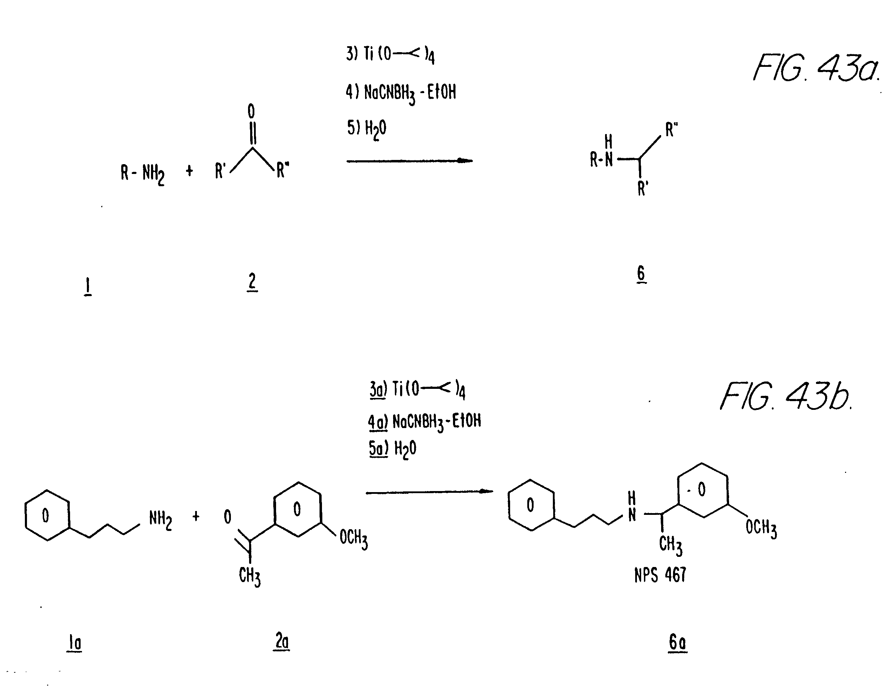

A preferred procedure for preparing fendiline or fendiline analogs (such as those depicted in Figure 36) uses titanium(IV) isopropoxide and was modified from methods described in J. Org. Chem. 55:2552 (1990). For the synthesis of NPS 544, titanium tetrachloride (method described in Tetrahedron Letters 31:5547 (1990)) was used in place of titanium(IV) isopropoxide. The reaction scheme is depicted in Figure 43a. In Figure 43a, R,R' and R" depict hydrocarbyl groups. According to one embodiment, in a 4 ml vial, 1 mmole of amine (1) (typically a primary amine) and 1 mmole ketone or aldehyde (2) (generally acetophenone) are mixed, then treated with 1.25 mmoles titanium(IV) isopropoxide (3) and allowed to stand with occasional stirring at room temperature for about 30 minutes. Alternatively, a secondary amine may be used in place of (1). Note: some reactions will give heavy precipitates or solids which are warmed/heated (to their melting point) to allow for stirring/mixing several times over the course of the reaction. The reaction mixture is treated with 1 ml ethanol containing 1 mmole sodium cyanoborohydride (4) and the resulting mixture is then allowed to stand at room temperature with occasional stirring for about 16 hours. After this time the reaction is quenched by the addition of about 500 µl water. The reaction mixture is then diluted to about 4 ml total volume with ethyl ether and then centrifuged. The upper organic phase is removed and reduced on a rotavapor. The resulting product, (6), is partially purified by chromatography through a short column of silica (or alternatively by using preparative TLC on silica) using combination of dichloromethane:methanol:isopropylamine (typically 95:5:0.1), prior to purification by HPLC (normal phase using silica with dichloromethane:methanol: isopropylamine or reversed phase, C-18 with 0.1% TFA with acetonitrile or methanol).

If appropriate or desired, chiral resolution may be accomplished using methods such as those described in Example 21:

### Formulation and Administration

As demonstrated herein, the molecules of the invention may be used to: (a) mimic or antagonize one or more of the effect of an extracellular ion, including extracellular Ca²⁺; (b) affect the extracellular free Ca²⁺ level in an individual; and (c) treat diseases such as hyperparathyroidism, osteoporosis and hypertension. In general, diseases or conditions involving inorganic-ion receptors now can be studied, diagnosed and/or beneficially treated. While the molecules have generally been shown to have an effect on parathyroid cells, they may also modulate the Ca²⁺ receptors on other cells, including bone osteoclasts, juxtaglomerular kidney cells, proximal tubule kidney cells, distal tubule kidney cell, cell of the thick ascending limb of Henle's loop and/or collecting duct, keratinocyte in the epidermis, parafollicular cell in the thyroid (C-cells), intestinal cell, trophoblast in the placenta, platelet, vascular smooth muscle cell, cardiac atrial cell, gastrin and glucagon secreting cells, kidney mesangial cell and mammary cell.

While these molecules will typically be used in therapy for human patients, they may be used to treat similar or identical diseases in other warm-blooded animal species such as other primates, farm animals such as swine, cattle, and poultry; and sports animals and pets such as horses, dogs and cats.

In therapeutic and/or diagnostic applications, the molecules of the invention can be formulated for a variety of modes of administration, including systemic and topical or localized administration. Techniques and formulations generally may be found in Remington's Pharmaceutical Sciences, Mack Publishing Co., Easton, PA.

For systemic administration, oral administration is preferred. Alternatively, injection may be used, e.g., intramuscular, intravenous, intraperitoneal, and subcutaneous. For injection, the molecules of the invention are formulated in liquid solutions, preferably in physiologically compatible buffers such as Hank's solution or Ringer's solution. In addition, the molecules may be formulated in solid form and redissolved or suspended immediately prior to use. Lyophilized forms are also included.

Systemic administration can also be by transmucosal or transdermal means, or the molecules can be administrated orally. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays, for example, or using suppositories. For oral administration, the molecules are formulated into conventional oral administration forms such as capsules, tablets, and tonics.

For topical administration, the molecules of the invention are formulated into ointments, salves, gels, or creams, as is generally known in the art.

As shown in the examples below, the amounts of various compounds of this invention which must be administered can be determined by standard procedures. Generally it is an amount between about 1 and 50 mg/kg animal to be treated.

### Recombinant Receptors

Natural product screening has traditionally provided the lead structures for the development of diverse therapeutic molecules. However, high-throughput screening of natural product libraries or other molecule libraries for activity on the Ca²⁺ receptor has not previously been possible. To achieve this capability, it is best to clone the Ca²⁺ receptor cDNA and then create transfected cell lines suitable for high-throughput screening. The structure of the receptor can additionally be used to gain insight into the molecular geometry of the ligand binding site(s), and such information used to augment a rational drug design program as discussed above. Limited structure-activity studies and testing of selected natural product molecules will provide the initial structural data base necessary to guide rational natural product screening and drug design.

The discovery of a superfamily of genes permits the same advantages in connection with all inorganic-ion receptors. Although the following discussion will refer often to methodology for cloning a calcium receptor, it will be understood by those of ordinary skill in the art that the methodology generally is applicable to the cloning of all inorganic-ion receptors.

These recombinant receptors allow for the first time screening mimics and lytics, including calcimimetics and calcilytics. For example, by binding assays with the receptors. This is particularly useful in screening with the human receptors in order to identify therapeutically useful compounds.

The bovine and human parathyroid cell Ca²⁺ receptor cDNA can be cloned by functional expression in Xenopus oocytes. It is possible to monitor an increase in intracellular Ca²⁺ in Xenopus oocytes indirectly by measuring current through the endogenous Ca²⁺-activated Cl⁻ channel. The amplification of the response afforded by this signal transduction pathway enables the detection of receptor proteins encoded by mRNA at very low levels. This allows the detection of receptor- specific cDNA clones without the need for high affinity ligands, specific antisera, or protein or nucleic acid sequence information. An example of such a procedure follows.

Adult female Xenopus laevis were obtained from Xenopus I (Ann Arbor, MI) and maintained according to standard procedures. Lobes of ovary were excised from hypothermically-anesthetized toads. Clusters of oocytes were transferred into modified Barth's saline (MBS). Individual oocytes were obtained by incubation in MBS containing 2 mg/ml collagenase (Sigma, Type 1A) for 2h at 21°C and stage V-VI oocytes were selected for injection.

Glass capillary tubes (1 mm diameter) were pulled to a fine tip and manually broken to achieve a tip diameter of about 15 µM. A droplet of mRNA (1 ng/nl in diethylpyrocarbonate (DEPC)-treated water) was placed onto parafilm and drawn into the capillary tube by suction. The capillary tube was then connected to a picospritzer (WPI Instruments) and the volume of the air-pulsed droplets adjusted to deliver 50 ng of mRNA (typically 50 nl). A 35 mm culture dish with a patch of nylon stocking fixed to the bottom was used to secure the oocytes during injection of mRNA into the vegetal pole. The injected oocytes were placed into a 35 mm culture dish containing MBS, 100 µg/ml penicillin and 100 µg/ml streptomycin and incubated at 18°C for 3 days.

Following incubation, an oocyte was placed into a 100 µl plastic chamber and superfused with MBS at a flow rate of 0.5 ml/min using a peristaltic pump. Test molecules or inorganic polycations were added by rapidly moving the tubing into different buffers. Recording and current-passing electrodes were constructed from thin wall capillary tubing pulled to a resistance of 1-3 Mohms and filled with 3 M KCl. Oocytes were impaled (in the animal pole) with both electrodes under microscopic observation and connected to an Axon Instruments Axoclamp 2A voltage-clamp amplifier which was used to set the holding potential (-70 to -80 mV) and to measure the currents that were passed to maintain the holding potential. Currents were recorded directly onto a strip chart recorder.

For mRNA preparation, tissue was obtained from calves or patients with secondary HPT undergoing surgical removal of the parathyroid glands. Purified cells need not be prepared; whole pieces of gland were used to prepare mRNA that directs the expression of the Ca²⁺ receptor in Xenopus oocytes. Total cellular RNA was prepared by acid guanidinium thiocyanate/phenol extraction of homogenized glands. Oligo-dT cellulose chromatography was used to select poly(A)⁺ mRNA by standard procedures. For size fractionation of mRNA, centrifugation through glycerol gradients was used. The mRNA was denatured with 20 mM methylmercuric hydroxide and loaded (50-100 µg at a concentration of 1 mg/ml) onto a linear 15-30% glycerol gradient prepared in Beckman TLS55 tubes. Following centrifugation at 34,000 rpm for 16 hrs, 0.3 ml gradient fractions were collected and diluted in an equal volume of water containing 5mM beta-mercaptoethanol. mRNA was then recovered by two cycles of ethanol precipitation. If desired, the mRNA (50-100 µg of poly(A)⁺) may be separated on a 1.2% agarose/6.0 M urea preparative gel, along with a range of RNA size markers. Following visualization of the mRNA by ethidium bromide staining, gel slices containing RNA in approximately 1 kb to 2kb size steps are excised. mRNA is recovered from the agarose gel slices using RNAid binding matrix (according to the supplier's standard protocol; Stratagene, Inc.) and recovered mRNA fractions eluted into DEPC-treated water.

Amounts of recovered mRNA were quantified by UV absorbance measurement. The size range of mRNA contained within each fraction of the glycerol gradient was determined by formaldehyde/agarose gel electrophoresis using a small quantity (0.5 µg) of each sample. The integrity of the mRNA was assessed by in vitro translation of each sample. Reticulocyte lysates (commercially available kits; BRL) were used to translate 0.05-0.5 µg of each mRNA fraction. The resulting ³⁵S-labelled proteins were analyzed by SDS-PAGE. The intact mRNA was capable of directing the synthesis of proteins of a complete size range, corresponding roughly to the sizes of the individual mRNA fractions.

A cDNA library was constructed in the vector λ ZAPII, following modifications of the technique of Gubler and Hoffman. RNA from the fraction(s) giving the best response in the oocyte assay was used as starting material. First-strand cDNA syntheses was primed with an oligo-dT/NotI primer-linker. Second-strand synthesis was by the RNase H/DNA Polymerase I self-priming method. Double-stranded cDNA was blunted with T4 DNA polymerase and EcoRI adaptors blunt-end ligated to the cDNA with T4 ligase. Following NotI digestion to cleave the linker, full-length cDNA was size-selected by exclusion chromatography on Sephacryl 500 HA. First-strand cDNA was radiolabeled with α-³²P-dATP, and all synthesis and recovery steps monitored by following the incorporation of radioactivity. Full-length cDNA recovered from the sizing column was ligated to EcoRI/NotI digested λ ZAPII arms. The ligation mix was test packaged with commercially available high-efficiency packaging extract (Stratagene, Inc.) and plated on the appropriate host strain (XL1-blue). The percentage of recombinant phage was determined by the ratio of white-to-blue plaques when the library is plated on IPTG and X-gal.

The average insert size was determined from ten randomly selected clones. Phage DNA "mini-preps" were digested with EcoRI and NotI to release the insert, and the size determined by agarose gel electrophoresis. The library consisted of >90% recombinant phage, and the insert size ranged from 1.5 to 4.2 kb. The recombinant ligation was packaged in large scale to generate 800,000 primary clones. The packaging mix was titered and plated at 50,000 plaques per. 15 cm plate. Each pool of 50,000 clones was eluted in SM buffer and stored individually.

Plate lysate stocks of each of the clone pools were used for small-scale phage DNA preparation. Phage particles are concentrated by polyethylene glycol precipitation, and phage DNA purified by proteinase K digestion followed by phenol:chloroform extraction. Twenty micrograms of DNA are digested with NotI, and used as template for in vitro transcription of sense-strand RNA. In vitro transcription is according to standard protocols, utilizing T7 RNA polymerase and 5' cap analog m⁷ GpppG in a 50 µl total reaction volume. Following Dnase I/Proteinase K digestion and phenol/chloroform extraction, the RNA is concentrated by ethanol precipitation and used for oocyte injection.

Oocytes were injected with synthetic mRNA (cRNA) from each of the 16 library subpools constituting 50,000 independent clones each. After incubation for 3 to 4 days, oocytes were assayed for the ability of 10 mM neomycin to elicit a Ca²⁺-dependent Cl-current. A pool designated "pool 6" gave a positive signal and thus contains a cDNA clone encoding a functional calcium receptor. In order to decrease the complexity of pool 6 and thus proceed towards the purification of the calcium receptor clone contained within this pool, pool 6 phage were replated at µ20,000 plaques per plate and 12 plates harvested. DNA was prepared from each of these subpools and cRNA synthesized. Again, oocytes were injected with cRNA and assayed 3-4 days later for the ability of 10 mM neomycin to elicit a Ca²⁺⁻ dependent Cl-current. A subpool pool 6-3 was positive and this pool was subjected to a further round of plating, reducing the complexity of pools to around 5,000 clones per pool. Pools were again assayed by preparation of cRNA and injection in oocytes. A subpool pool 6-3.4 was positive. In order to expedite further purification of the positive clone in pool 6-3.4, phage DNA from this pool was rescued as plasmid DNA by superinfection with the helper phage, ExAssist (Stratagene). Transfection of rescued plasmids into bacterial strain DH5alphaF' resulted in transformed bacterial colonies on ampicillin plates. These were harvested in pool of 900 clones each. Plasmid DNA was then prepared from each subpool and cRNA synthesized and assayed in the usual manner. Subpool 6-3.4.4 was positive. Bacteria containing the plasmid subpool 6-3.4.4 were subsequently plated in subpools of µ50 clones each. Continuation of this process is expected to result in a single clone encoding a functional calcium receptor.

Alternative assays are available for detecting expression of a calcium receptor in oocytes. For example, oocytes can be loaded with ⁴⁵Ca⁺⁺ and then treated with a calcimimetic. Mobilization of ⁴⁵Ca⁺⁺ from intracellular stores results in a net increase in ⁴⁵Ca⁺⁺ efflux which is readily determined. Fluorescent Ca⁺⁺ indicators may also be injected into oocytes. In this case, oocytes expressing a calcium receptor will exhibit increased fluorescence upon activation with a calcimimetic. These assays may be employed for cloning calcium receptors in place of the electrophysiological assay for calcium induced Cl⁻ current described in the above example. In addition, the calcimimetic ligand used in the cloning procedure need not be neomycin as indicated above but could instead be, for example, Gd⁺⁺⁺, Ca⁺⁺, Mg⁺⁺ or other calcimimetic compound.

Initial experiments used Xenopus oocytes injected with water or poly(A)⁺-enriched mRNA (50 ng) from bovine parathyroid cells. After three days, the oocytes were examined for their ability to increase intracellular Ca²⁺ in response to increases in the concentration of extracellular di- and trivalent cations. The oocytes were impaled with recording and current-passing electrodes and [Ca²⁺]ᵢ was assessed indirectly by measuring currents through the endogenous Ca²⁺-activated Cl⁻ channel. In oocytes injected with poly(A)⁺-enriched mRNA from bovine (or human, Fig. 26) parathyroid cells, increasing the concentration of extracellular Ca²⁺ from 0.7 to 3, 5 or 10 mM caused a rapid and transient increase in the Cl⁻ current which then oscillated around a higher basal current. Increasing the concentration of extracellular Mg²⁺ from 1 to 10 mM likewise evoked oscillatory increases in Cl⁻ current. The Cl⁻ current response to extracellular Mg²⁺ persisted when the extracellular Ca²⁺ concentration was reduced to < 1 µM (Fig. 25).

The impermeant trivalent cation Gd³⁺ (600 µM) also caused oscillatory increases in the Cl⁻ current (Fig. 25). Such increases in the Cl⁻ current which oscillate and persist in the nominal absence of extracellular Ca²⁺ are noted when oocytes have been allowed to express other Ca²⁺-mobilizing receptors and are stimulated with the appropriate ligand (e.g., substance K, Fig. 25). In these instances, the increase in Cl⁻ current reflects the mobilization of intracellular Ca²⁺. These initial studies likewise show that extracellular polycations mobilize intracellular Ca²⁺ in parathyroid cell mRNA-injected oocytes.

Oocytes injected with water did not show any change in the Cl⁻ current when exposed to extracellular Ca²⁺ (10 mM) or Mg²⁺ (20 or 30 mM). In one series of experiments, oocytes were injected with the mRNA encoding the substance K receptor. In these oocytes, extracellular Mg²⁺ (20 mM) did not evoke any current but the cells responded vigorously to the addition of substance K (Fig. 25). These experiments indicate that there is no endogenous sensitivity of the oocyte to extracellular Ca²⁺ or Mg²⁺.

Similar experiments were performed using oocytes injected with poly(A)⁺-enriched mRNA prepared from human parathyroid glands (hyperplastic tissue from a case of secondary HPT). In these oocytes, increasing the concentration of extracellular Ca²⁺ caused a reversible increase in the Cl⁻ current which oscillated (Fig. 26). The addition of 300 µM La³⁺ likewise caused oscillatory increases in the Cl⁻ current. Increasing the concentration of extracellular Mg²⁺ from 1 to 10 mM evoked increases in the Cl⁻ current that persisted in the absence of extracellular Ca²⁺. Additional experiments suggest that the response to extracellular Ca²⁺ is concentration dependent. Thus, in three mRNA-injected oocytes, Cl⁻. current increased to a maximum of 111 ± 22 nA at 3 mM and 233 ± 101 nA at 10 mM extracellular Ca²⁺.

The results obtained in Xenopus oocytes demonstrate the presence of a mRNA(s) in parathyroid cells encoding a protein(s) which can impart, in normally unresponsive cells, sensitivity to extracellular Ca²⁺. Moreover, the ability of extracellular Mg²⁺ to evoke oscillatory increases in Cl⁻ current in the absence of extracellular Ca²⁺ demonstrates that the Cl⁻ current depends on the mobilization of intracellular Ca²⁺ rather than influx of extracellular Ca²⁺. The results obtained with La³⁺ likewise show that the expressed protein(s) is linked to the mobilization of intracellular Ca²⁺. Together, these data show that the expressed protein(s) acts as a cell surface receptor rather than a channel. These studies provide compelling evidence for the existence of a Ca²⁺ receptor protein on the surface of parathyroid cells and demonstrate the feasibility of using the Xenopus oocyte system to achieve the molecular cloning of the Ca²⁺ receptor cDNA.

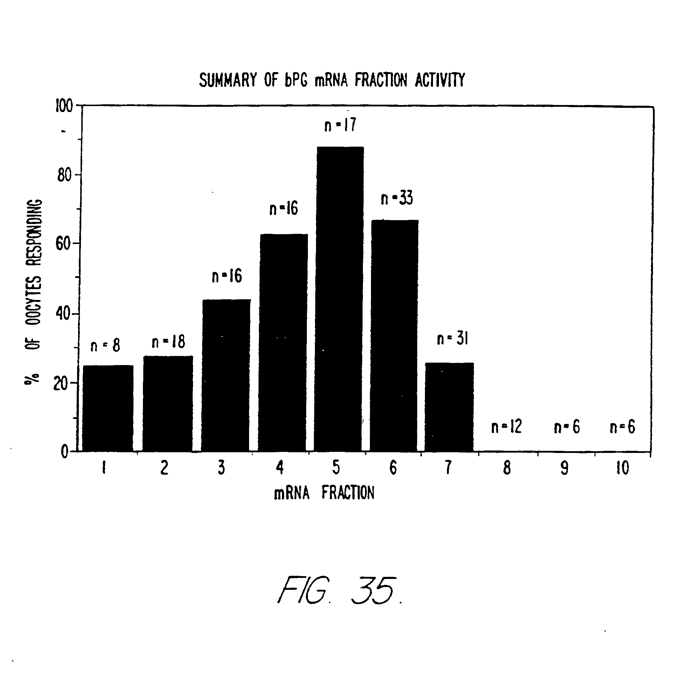

In another series of experiments, parathyroid cell mRNA was size fractionated by centrifugation through a glycerol gradient. Ten fractions were collected. Each group was injected into Xenopus oocytes and after a three day incubation period the oocytes were assayed for expression of the Ca²⁺ receptor. Those oocytes injected with fractions 4-6 showed the largest and most consistent increases in Cl⁻ current in response to extracellular Ca²⁺ (Fig. 35). These results indicate that the Ca²⁺ receptor is encoded by mRNA in a size range of 2.5-3.5 kb. This indicates that a strategy using direct expression of RNA synthesized from a transcription vector cDNA library is feasible. Size-fractionation experiments of this sort were conducted and in each of three different fractionation experiments similar results were obtained.

The mRNA fractions obtained and characterized in the preceding experiments can be assayed by injection into oocytes. For each mRNA fraction, 10-20 oocytes are injected with 50 ng of RNA at a concentration of 1 ng/nl in water. Injected oocytes are maintained at 18° C for 48-72 h after which they are assessed for expression of the Ca²⁺ receptor using measurements of Cl⁻ current. For each group of injected oocytes the number positive for expression of the receptor, as well as the magnitude of the Ca²⁺-dependent Cl⁻ current measured, is determined. As negative controls, oocytes are injected with rat liver poly(A)⁺-enriched mRNA, yeast RNA, or water.

It is expected that an mRNA in the range of 2.5 - 3.5 kb will encode the receptor. mRNA of a larger size may necessitate a cloning approach based on hybrid depletion of parathyroid mRNA prior to oocyte injection. This strategy is not dependent upon the generation of full-length cDNA clones for success. If receptor expression is not obtained with a single size fraction of mRNA, oocytes are injected with mixed size fractions to determine a combination that does give rise to a functional receptor. If it does appear that multiple subunits are necessary for the formation of a functional receptor, the hybrid depletion expression cloning strategy is used. In this approach, clones are selected on the basis of their ability to deplete a specific mRNA species from the total mRNA population. A clone encoding a single subunit is identified by its ability to prevent the formation of the active multi-subunit complex. By exhaustive screening it is possible to identify clones encoding all of the necessary subunits.

This approach permits the isolation of clones encoding individual subunits required to form a functional receptor complex. Synthetic RNA from pools of clones are assayed for their ability to induce expression of the Ca²⁺ receptor in Xenopus oocytes by the same techniques used to analyze the original mRNA fractions. Originally, 10 pools representing 100,000 primary clones each are examined. Pools of clones showing a positive response are screened at lower (typically 4-to-10 fold) complexity, and again positive pools further subdivided and screened. This process of library sub-fractionation is followed until individual positive clones are identified. As a negative control for the oocyte expression assay, anti-sense transcripts are generated from those DNA templates that induce a positive response. Anti-sense transcripts are unable to give rise to an authentic receptor, and this will control any non-specific positive signal arising from injection of synthetic RNA. Another concern is the fact that synthetic RNA can occasionally "poison" translation in injected oocytes, by an undefined mechanism. To control for this possibility, synthetic RNAs giving a negative response are co-injected at various dilutions with parathyroid cell mRNA, to determine if they are non-specifically interfering with the expression of the Ca²⁺ receptor.

When an individual clone encoding the Ca²⁺ receptor is identified, the cDNA insert will be excised from the vector and used for large'scale production of synthetic RNA. Oocyte injection of this single RNA species allows rigorous assessment of the characteristics of the expressed receptor.

If the size of the mRNA encoding the Ca²⁺ receptor is too large for cloning by direct transcription and expression, or if multiple subunits are involved, a hybrid-depletion technique of screening pools of clones is used. cDNA insert DNA will be prepared from pools of clones from the size-selected parathyroid cell cDNA library. This DNA is hybridized to parathyroid cell mRNA under conditions that permit the formation of DNA/RNA duplexes. The unannealed, hybrid-depleted RNA is recovered and used for oocyte injections. Other methods for hybrid-depletion or hybrid-arrest may be used alternatively and are well known by those skilled in the art. DNA from pools of clones containing sequences representing Ca²⁺-receptor mRNA is depleted from this mRNA from the total parathyroid cell mRNA population, and expression of the receptor is reduced or absent upon oocyte injection. A process of sub-fractionation is followed on pools of clones of decreasing complexity, at each step assaying for cloned DNA that depletes Ca²⁺ receptor-encoding mRNA from the total parathyroid cell mRNA population. The use of an internal control during the hybrid depletion assays ensures that the hybrid-depleted RNA is intact and capable of being translated in the oocyte.

Human parathyroid cells express a beta-adrenergic receptor coupled to adenylate cyclase. This receptor can be expressed in oocytes, where it is capable of agonist-induced activation of the endogenous adenylate cyclase. During the hybrid-depletion screening for Ca²⁺ receptor clones, oocytes injected with hybrid depleted mRNA are assayed for isoproterenol-induced adenylate cyclase activation. A positive response in this assay serves to indicate that any observed inhibition of Ca²⁺ receptor response is specific, and not due to a general inhibition of the total mRNA population.

The hybrid-depletion screening strategy can result in the isolation of clones that do not contain a complete protein coding region. Positive clones isolated by this screening strategy are sequenced to determine their protein coding capacity. Northern blot analysis of human parathyroid gland RNA permits the determination of the size of the complete mRNA corresponding to specific clones. If positive clones do not appear to be full length, the cloned cDNA will be used as a hybridization probe to screen a parathyroid gland cDNA library for complete cDNAs.

A variety of cell lines are capable of coupling exogenously expressed receptors to endogenous functional responses. A number of these cell lines (e.g., NIH-3T3, HeLa, NG115, CHO, HEK 293 and COS7) can be tested to confirm that they lack an endogenous Ca²⁺ receptor. Those lines lacking a response to external Ca²⁺ can be used to establish stably transfected cell lines expressing the cloned Ca²⁺ receptor.

Production of these stable tansfectants is accomplished by transfection of an appropriate cell line with a eukaryotic expression vector, such as pMSG, in which the coding sequence for the Ca²⁺ receptor cDNA has been cloned into the multiple cloning site. These expression vectors contain a promoter region, such as the mouse mammary tumor virus promoter (MMTV), that drive high-level transcription of cDNAs in a variety of mammalian cells. In addition, these vectors contain genes for the selection of cells that stably express the cDNA of interest. The selectable marker in the pMSG vector encodes an enzyme, xanthine-guanine phosphoribosyl transferase (XGPRT), that confers resistance to a metabolic inhibitor that is added to the culture to kill the nontransfected cells. A variety of expression vectors and selection schemes are usually assessed to determine the optimal conditions for the production of Ca²⁺ receptor expressing cell lines for use in high throughput screening assays.

The most effective method for transfection of eukaryotic cell lines with plasmid DNA varies with the given cell type. The Ca²⁺ receptor expression construct will be introduced into cultured cells by the appropriate technique, either Ca²⁺ phosphate precipitation, DEAE-dextran transfection, lipofection or electroporation.

Cells that have stably incorporated the transfected DNA will be identified by their resistance to selection media, as described above, and clonal cell lines will be produced by expansion of resistant colonies. The expression of the Ca²⁺ receptor cDNA by these cell lines will be assessed by solution hybridization and Northern blot analysis. Functional expression of the receptor protein will be determined by measuring the mobilization of intracellular Ca²⁺ in response to externally applied Ca²⁺ receptor agonists.

Cloning the Ca²⁺ receptor enables both structural and functional studies of this novel receptor. Recombinantly produced receptor may be crystallized for structural studies. Stably transfected cell lines expressing the receptor can be used for high-throughput screening of natural product or other compound libraries. Molecules of the requisite potency and specificity can be labeled (radioactively or fluorescently). The ability of test molecules/extracts to displace such a labeled molecule will form the basis of a high-throughput assay for screening.

Another strategy for cloning an inorganic-ion receptor in oocytes is as follows.

The general procedures for isolation, defolliculation and injection of frog oocytes are described below. In brief, Xenopus laevis frogs are anesthetized by immersion in 0.17% Tricaine and a lobe of the ovary removed and minced into small pieces. Individual stage V-VI oocytes are isolated using an L-shaped capillary tube after incubating the ovarian tissue in a calcium-free buffer at room temperature for 90 min. The separated oocytes [usually 200-300 per experiment] are then incubated in collagenase solution [Sigma, Type II; 2 mg/ml] for an additional 90 min following which the follicular layer can be removed with fine forceps. The defolliculated oocytes are incubated overnight in frog ringer [ND96] at 17°C and degenerating oocytes removed [usually <2-3% of the total]. Surviving oocytes are then injected with 50 nl H₂O [control] or poly(A)⁺RNA[15-50 ng/oocyte; in 50 nl H₂O] and then , incubated for 2-4 days at 17°C. (The frog ringer, ND96, contains (mM): NaCl (96), RCl (2), CaCl₂ (0.5), MgCl₂ (0.5), HEPES (5), pyruvate (2.5)].

Total RNA is extracted from the tissue [kidney, osteoclasts, etc.] with guanidinium thiocyanate and separated on a CsCl cushion. Poly(A)⁺RNA is then isolated by oligo(dT) cellulose chromatography [two passes through the column]. The integrity of the poly(A)⁺RNA is assessed by agarose gel electrophoresis. The ability of 25-50 ng of poly(A)⁺RNA to give rise to Ca²⁺-dependent Cl⁻ channel activity upon exposure to extracellular Gd³⁺ is assessed [i.e., as evidence for Ca²⁺ receptor activity]. The next step is to localize the mRNA expressing Ca²⁺ receptor activity to a small size range (∼1 kb). For this purpose, ∼100 µg of poly(A)⁺RNA is separated by sucrose gradient and 40 size fractions collected from the gradient. Alternatively, ~200-300 µg of poly(A)⁺RNA can be fractionated by preparative, continuous flow agarose gel electrophoresis [Hediger, M.A.: Anal. Biochem. 159:280-86, (1986) and 70-90 fractions collected. X. laevis oocytes are injected with pools of fractions [3-5 fractions/pool] of poly(A)+RNA [0.2-0.5 ng/oocyte] fractions from the pool(s) and the ability of these pools to give rise to Ca²⁺ receptor activity assessed. Individual fractions from the pool(s) giving the highest Ca²⁺ receptor activity are injected into oocytes to define the fraction(s) within this pool of poly(A)+RNA that give the highest expression of the Ca²⁺ receptor in oocytes.

A directional cDNA library is constructed from this poly(A)+RNA pool using the Superscript Plasmid system [pSPORT1; BRL]. cDNA is generated using the Superscript, MuMLV-RT, reverse transcripts [many of which will be full-length for the coding region]. cDNA is sized by gel electrophoresis and the appropriate cDNA size region [based on the poly(A)+RNA size range] is extracted using GENECLEAN II [Bio 101]. This size selected cDNA is then directionally ligated into the pSP4RT1 plasmid vector using a Not I primer at the 3' end and an adapter for Sal I at the 5' end. The resulting plasmids are introduced into ELECTROMAX DH10B cells [BRL] by electroporation. The transformed bacteria are grown on nitrocellulose filters [500-800 colonies/filter] and master filters stored at 4°C [short-term] or -70°C [long-term]. Replicate filters are grown and plasmid DNA is isolated from the filters, linearized by restriction cutting, and then used as a template for sense cRNA synthesis by in vitro transcription [T7 promoter in the presence of Cap-analog]. The pools of cRNA are individually injected into oocytes which are assayed for expression of Gd³⁺-induced [1-100µM] activation of Cl⁻ currents. A standard sib selection procedure is utilized to identify a clone that expresses Ca²⁺ receptor activity in oocytes.

A restriction map of the Ca²⁺ receptor cDNA is generated and suitable restriction fragments subcloned into pSPORT1. Subcloned cDNAs are bidrectionally sequenced using standard methods [Sequenase Polymerase 2® 2, USB]. Sequencing primers (18-mer) are used where necessary to sequence regions within or between subclones or to resolve compressions or ambiguous regions of sequence. The coding region for the Ca²⁺ receptor protein is determined from the longest open reading frame that has a start site preferably homologous with the Kozak consensus sequence. Hydropathy and other protein algorithms are used to generate a topology for the Ca²⁺ receptor protein. The nucleotide sequence of the cDNA, the amino acid sequence of the Ca²⁺ receptor protein, and the protein topology are compared with that for the other Ca²⁺ receptors and other known cDNAs and proteins present in the database. Homologous regions might represent domains involved in cation binding or regulation.

The presently preferred method for isolating inorganic-ion receptor nucleic acid is based upon hybridization screening.

Region specific primers or probes derived from BoPCaR 1 can be used to prime DNA synthesis and PCR amplification, as well as to identify colonies containing cloned DNA encoding a member of the inorganic ion receptor family using known methods (Innis et al., PCR Protocols, Academic Press, San Diego, CA (1990)).

When using primers that are derived from nucleic acid encoding an inorganic-ion receptor, one skilled in the art will recognize that by employing high stringency conditions, annealing at 50-60°C, sequences which are greater than about 76% homologous to the primer will be amplified. By employing lower stringency conditions, annealing at 35-37°C, sequences which are greater than about 40-50% homologous to the primer will be amplified.

When using DNA probes derived from inorganic ion receptors for colony/plaque hybridization, one skilled in the art will recognize that by employing high stringency condition, hybridization at 50-65°C, 5X SSPC, 50% formamide, wash at 50-65°C, 0.5X SSPC, sequences having regions which are greater than about 90% homologous to the probe can be obtained, and by employing lower stringency conditions, hybridization at 35-37°C, 5X SSPC, 40-45% formamide, wash at 42°C SSPC, sequences having regions which are greater than 35-45% homologous to the probe will be obtained.

Any tissue can be used as the source for the genomic DNA encoding members of the inorganic-ion receptor family. However, with respect to RNA, the most preferred source is tissues which express elevated levels of the desired inorganic-ion receptor family member. In the present invention, oocyte injection and two-electrode whole oocyte voltage clamping was used to identify expression from such a tissue source. However, using the sequences disclosed herein, it is now possible to identify such cells using the inorganic-ion receptor sequence as a probe in northern blot or in situ hybridization procedures, thus eliminating the necessity of the procedures employed to clone the first member of this family and eliminating the need to obtain RNA from a tissue which expresses elevated levels of inorganic-ion receptor.

The present invention further provides methods of identifying cells or tissues which express a member of the inorganic-ion receptor family. A probe comprising the DNA sequence, for example, of BoPCaR 1, a fragment thereof, or a DNA sequence encoding another member of the inorganic-ion receptor family of proteins can be used as a probe or amplification primer to detect cells which express a message homologous to the probe or primer. One skilled in the art can readily adapt currently available nucleic acid amplification or detection techniques so that it employs probes or primers based on the sequences encoding a member of the inorganic-ion receptor family.

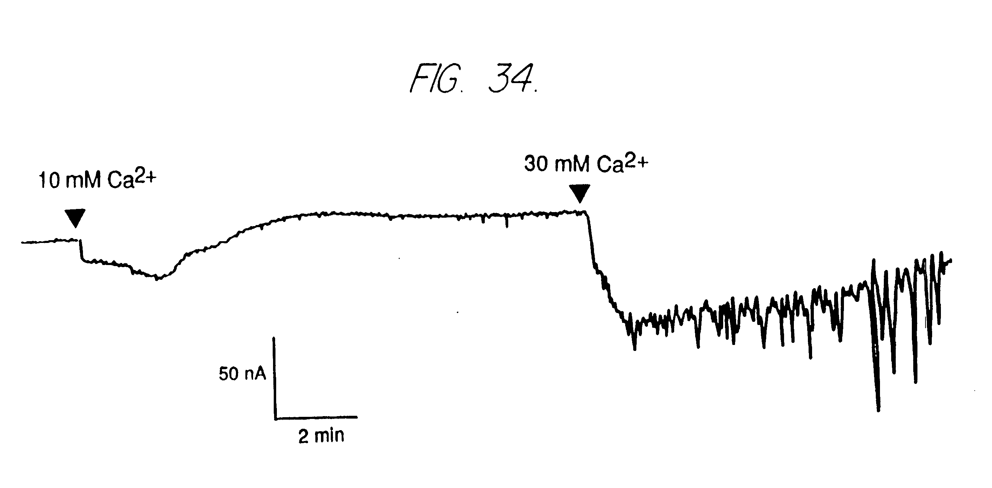

Given the appropriate cells or tissues expressing other receptors, these receptors may be cloned in a manner analogous to that described above for the parathyroid cell calcium receptor. For example, mRNA from human osteoclastoma tissue encodes the osteoclast calcium receptor (Figure 34). Thus, to isolate a clone for the human osteoclast receptor, one need only isolate mRNA from osteoclastoma tissue, prepared a cDNA library and assay/fractionate subpools as described above.. Furthermore, the preferred receptors for drug screening are of human origin. A clone encoding a receptor from one species may be used to obtain the corresponding human cDNA clone by cross-hybridization as is well known by those skilled in the art. In addition, the clone of the parathyroid cell or other cell receptor allows isolation of genes encoding similar inorganic-ion sensing proteins in other cells, and expression of those proteins. This is achieved by a variety of approaches. Southern blot analysis of human genomic DNA, utilizing the Ca²⁺ receptor cDNA as a hybridization probe, will give an indication of the number of related sequences encoded within the genome; hybridization at varying stringencies will give an indication of the degree of divergence among the related sequences. This will provide information about the potential number of genes encoding related receptor proteins. Northern blot analysis with Ca²⁺ receptor cDNA as probe will determine if the same or related transcripts are present in various tissues. If related transcripts homologous to the parathyroid cell Ca²⁺ receptor are detected, it is a relatively simple matter to obtain clones of these mRNAs, either by screening the appropriate cDNA libraries or by polymerase chain reaction (PCR) techniques.

Targeted gene walking (TGW) is a modification of a standard polymerase chain reaction (PCR) that allows amplification of unknown DNA sequences adjacent to short segments of known sequence. Parker et al., Nucl. Acids Res., 19:3055, (1991). Unlike conventional PCR techniques that amplify DNA sequences between two known primer sites, TGW can amplify DNA adjacent to one such site. Thus, TGW can serve as a replacement for conventional cloning and library screening methods for isolating sequences upstream or downstream from known sequences. The procedure can be used to isolate genes from any starting DNA template for which a limited amount of sequence information is known.

First, several standard PCR reactions are run in parallel using one "targeted primer" and different "walking primers." The targeted primer is a sequence-specific primer exactly complementary to a known sequence on the DNA molecule of interest, and is directed towards unknown adjacent sequences. The walking primers are non-specific sequences not complementary to DNA near the target primer. The walking primers can be any oligonucleotides unrelated to the target primer sequence. In the first series of PCRs, products are produced only when a walking primer anneals to a DNA strand contiguous with and complementary to the strand to which the targeted primer has hybridized. The PCR products of interest are preferably within the 5 kilobase size range. Amplification products are produced with as many as 60% mismatched nucleotides within the walking primer relative to DNA template. Perfect base-pairing is required only for the first two 3' nucleotides of the walking primer, but partial homology is tolerated otherwise. Annealing temperature is a key variable in determining the number of PCR products, as identified by agarose gel electrophoresis.

Second, an oligomer extension assay is performed using an "internal detection primer." This primer represents known sequences between the previous two primers, contiguous with the targeted primer. The internal detection primer is kinased with ³²P-gamma-ATP, then used in a single PCR cycle with DNA from the first PCR as template. This extension identifies products in the first PCR contiguous with the targeted primer. These new products are identified by agarose gel electrophoresis and autoradiography. Any products that do no hybridize to the internal detection primer represent non-contiguous amplification products produced by any subset of the primers.

Last, bands identified in the oligomer extension assay are excised from the gel, and reamplified by standard PCR using target primer and the walking primer that produced the band initially. This new PCR band is then sequenced directly to provide previously unknown sequence information.

To extend information in the opposite direction, complements are made of the targeted and internal detection primers, and their order is reversed in the protocol.

In performing hybridization screening and cloning, the following should be considered. Since there is degeneracy in the genetic code, more than one codon exists for almost all the amino acids (except tryptophan and methionine). Moreover, the frequency of usage of any particular codon is different in non-humans as compared to humans. Taking into account codon degeneracy and human-preferred codons, oligonucleotides are synthesized. Moreover, oligonucleotides with various permutations of possible codons are also synthesized. To avoid an excessive number, not all possible sequences resulting from the degeneracy of the code need be synthesized. Rather, a subset of codons are chosen which have the highest frequency of occurrence.

Novel receptor clones so obtained can be assessed functionally by expression, either in oocytes or in transfected cell lines. Transfected cell lines expressing a cell-specific inorganic-ion receptor can then provide a means of high-throughput screening for molecules that act specifically on the ion-sensing mechanism of, for example, osteoclasts or juxtaglomerular cells.

In an alternative method, the calcium receptor can be cloned by expression in eukaryotic cells. For example, a cDNA library can be prepared from parathyroid mRNA and cloned into a eukaryotic expression vector such as, pCDNA1. Subpools from this library can be transfected into eukaryotic cells such as COS7 or HEK293 cells resulting in relatively high-level transient expression of encoded cDNA sequences. Cells transfected with a function calcium receptor clone will express the calcium receptor which can then be activated by calcium, neomycin or other calcimimetic compounds. If cells are first loaded with a fluorometric indicator for [Ca²⁺]ᵢ, activation of the calcium receptor results in increased fluorescence. Thus library subpools containing the calcium receptor are identified by their ability, upon transfection into eukaryotic cells, to induce a calcium or calcimimetic-specific increase in fluorescence. This fluorescence can be detected using either a fluorimeter or a fluorescence-activated cell sorter (FACS).

We have also developed a "calcium trapping assay" for the detection of COS 7 cells expressing G-protein coupled receptors. In this assay COS 7 cell monolayers are transfected with cDNA clones from a bovine parathyroid cDNA library (e.g., subtractions or pools from a library prepared in pCDNA1) and are assayed for their ability to trap radioactive ⁴⁵Ca⁺⁺ in response to treatment with an agonist for the Ca²⁺ receptor. The monolayers undergo emulsion autoradiography and cells that have trapped ⁴⁵Ca⁺⁺ are identified by the presence of photographic grain clusters under dark-field microscopy. Library pools that produce a positive signal are then sequentially subdivided until a single cDNA that produces the signal is identified.

The calcium receptors appear to be functionally related to a class of receptors which utilize so-called "G" proteins to couple ligand binding to intracellular signals. Such "G-coupled" receptors may elicit increases in intracellular cyclic AMP due to the stimulation of adenylyl cyclase by a receptor activated "Gₛ" protein, or else may elicit a decrease in cyclic AMP due to inhibition of adenylyl cyclase by a receptor activated "Gᵢ" protein. Other receptor activated G proteins elicit changes in inositol trisphosphate levels resulting in release of Ca⁺⁺ from intracellular stores. This latter mechanism is particularly pertinent to calcium receptors. All known G-coupled receptors are structurally related, having seven conserved transmembrane domains. A number of such receptors have been cloned based on sequence homology to previously cloned receptors. One particularly useful approach is to employ degenerate primers homologous to the conserved transmembrane domain coding regions and to amplify DNA regions encoding these sequences using polymerase chain reaction (PCR). Thus, such oligonucleotide primers are mixed with genomic DNA or cDNA to RNA isolated from the tissue of choice and PCR carried out. Some experimentation may be required to specifically amplify novel G-coupled receptor sequences from the tissue of choice since these are not necessarily identical to already known G-coupled receptors but, this is well understood by those skilled in the art [see for example, Buck, L. and Axel, R. (1991) Cell, 65:175-187].

Calcium receptors may also be cloned by such a PCR approach. Two examples of degenerate oligonucleotide primer pairs which can be used to PCR amplify sequences encoding calcium receptors are given below. The first pair is based on the cross-homology exhibited by the majority of G-coupled receptors in the sequences encoding transmembrane domains II and VII respectively. The second primer pair is based on the cross-homology exhibited by a more divergent subgroup of G-coupled receptors which includes the calcitonin, secretin, PTH and GLP receptors. This pair corresponds to conserved sequences encoding transmembrane domains III and VII. When one or both of these two primer pairs are mixed with cDNA from parathyroid or osteoclast tissues, for example, PCR amplification results in the generation of amplified DNAs of about 500 to 800 base pairs. These DNAs can be isolated and analyzed for the presence of calcium receptor sequences.
For example, each amplified sequence can be used as a probe to isolate a full-length cDNA clone which can then be assessed in one or more of the assays indicated above to determine if an inorganic-ion receptor is encoded.

In an alternative method, the inorganic-ion receptor can be cloned by use of a monoclonal antibody generated against the receptor. Monoclonal antibodies provide powerful tools for the immunoaffinity purification of specific proteins. Once purified, limited amino acid sequence data can be obtained from the protein of interest, and used to design oligonucleotide sequence probes to screen for clones of the complete cDNA sequence.

An example involving a calcium receptor will be described. For production of hybridomas, whole bovine parathyroid gland cells are used as the immunogen.
Purified, dispersed cells are obtained, and live or fixed cell preparations are injected intraperitoneally into the appropriate mouse strain, according to established procedures. Standard protocols are followed for immunization schedules and for the production of hybridomas. A two-step screening procedure is used to identify hybridomas secreting monoclonal antibodies that recognize the Ca²⁺ receptor. The initial screen will identify those monoclonals that recognize parathyroid cell surface antigens. Immunohistochemical techniques are then used to screen hybridoma supernatants for the presence of mouse antibodies that bind to the surface of parathyroid cells. This screen can be performed on fixed sections of parathyroid gland tissue, or on dispersed cells in primary culture. The techniques for this assay are well established in the literature.

This screen will identify hybridomas producing monoclonal antibodies to a variety of cell-surface determinants, and monoclonals specific for the Ca²⁺ receptor would be expected to comprise only a small subset of these. To identify monoclonal antibodies that bind to the Ca²⁺ receptor, hybridoma supernatants that test positive in the initial screen are assayed for their ability to block the response of cultured parathyroid cells to Ca²⁺ receptor agonists. Some antibodies that bind to the extracellular domain of the receptor are expected to inhibit or activate ligand binding or to otherwise interfere with or affect receptor activation.

Monoclonal antibodies positive in both screens are characterized through Western blotting, immunoprecipitation and immunohistochemistry. This permits the determination of the size of the antigen that is recognized and its tissue distribution. The appropriate monoclonal antibody is then used for purification of the Ca²⁺ receptor protein by immunoaffinity chromatography, following standard techniques. Sufficient quantities of protein are obtained to allow limited amino acid sequence determination. Degenerate oligonucleotide probes are then designed on the basis of the peptide sequence information. These probes are then used to screen parathyroid gland cDNA libraries for full-length clones of the Ca²⁺ receptor. Clones obtained are characterized by DNA sequencing and by functional expression in the oocyte system and in cultured mammalian cell lines.

Alternatively, the antibodies can be used to screen expression libraries, e.g., cDNA libraries in λgt11 or its equivalent, to determine those clones expressing antigenically reactive protein. Such clones can then be sequenced to determine whether they encode a protein that might be a Ca²⁺ receptor.

It will also be appreciated by those skilled in the art that phage display libraries can be used to clone and analyze calcium receptors in place of monoclonal antibodies. In these libraries, antibody-variable regions or random peptides are shotgun cloned into phage expression vectors such that the antibody regions or peptides are displayed on the surface of the phage particle. Phage(s) which display antibody regions or peptides capable of high specific binding to calcium receptors will bind to cells which display these receptors (e.g., parathyroid cells, C-cells, osteoclasts, etc.). Hundreds of millions of such phage can be panned against these cell types preferentially selecting those phage which can bind to these cells (which includes those phage binding to calcium receptors). In this manner, the complexity of the library can be vastly reduced. Iterative repetition of this process results in a pool of phage which bind to the cell type used. Subsequently, the screens described above for monoclonal antibodies can be used to isolate phage which display calcium receptor-binding antibody or peptide regions, and these phage can be used to isolate the calcium receptor for purposes of structural identification and cloning. Kits to prepare such phage- display libraries are commercially available (e.g., Stratacyte, or Cambridge Antibody Technology Limited). Recombinant phage endowed with such calcium receptor-binding properties can also be used in lieu of monoclonal antibodies in the various analyses of calcium receptors. Such phage can also be used in high throughput binding-competition screens to identify organic compounds capable of functional binding to calcium receptors which can serve as structural leads for the development of human therapeutics acting at the calcium receptor.

In another alternative, affinity cross-linking of radioligands to their receptors can be used to isolate the receptor protein as described by Pilch & Czech, 1 Receptor Biochem. Methodol. 161, 1984. Covalent attachment of a radioligand allows extensive washing to remove non-specific binding. For example, a high- affinity molecule, e.g., a random copolymer of arginine and tyrosine (MW = 22K; argtyr ratio = 4:1) which mobilizes intracellular Ca²⁺ with an EC₅₀ of about 100 nM or less, is iodinated with ¹²⁵I, and cross-linked. Protamines, because of their much smaller size, may be preferable in cross-linking studies and can be reductively alkylated as described by Dottavio-Martin & Ravel, 87 Analyt. Biochem. 562, 1978.

Nonspecific labelling is kept to a minimum by cross-linking in the presence of unlabeled polycations and di- and trivalent cations. At high concentrations of these molecules nonspecific interactions of the label with the cell surface might be reduced.

The invention provides isolated nucleic acid sequences encoding inorganic-ion receptors and the isolated receptors themselves. It also provides unique fragments of the foregoing. The term "isolated" refers to a nucleic acid sequence: (i) amplified in vitro by, for example, polymerase chain reaction (PCR); (ii) synthesized by, for example, chemical synthesis; (iii) recombinantly produced by cloning; or (iv) purified, as by cleavage and gel separation. The term "isolated" when used in descriptions of polypeptide or amino acid sequences refers to polypeptides resulting from an expression system using the isolated nucleic acid sequences of the invention, as well as polypeptides synthesized by, for example, chemical synthetic methods, and polypeptides separated from native biological materials, and then substantially purified using conventional protein analytical procedures.

Also included within the scope of the invention are unique fragments of inorganic-ion receptors. The term unique fragments refers to portions of the receptor that find no counterpart in known sequences as of the date of this invention. These polypeptide fragments can be readily identified as unique by scanning protein databases known as of the time of the invention for identical peptides. In addition to generating fragments of receptors from expression of cloned partial sequences of receptor DNA, fragments can be generated directly from the intact protein. Proteins are specifically cleaved by proteolytic enzymes, including, but not limited to, trypsin, chymotrypsin or pepsin. Each of these enzymes is specific for the type of peptide bond it attacks. Trypsin catalyzes the hydrolysis of peptide bonds whose carbonyl group is from a basic amino acid, usually arginine or lysine. Pepsin and chymotrypsin catalyze the hydrolysis of peptide bonds from aromatic amino acids, particularly tryptophan, tyrosine and phenylalanine. Alternate sets of cleaved polypeptide fragments are generated by preventing cleavage at a site which is susceptible to a proteolytic enzyme. For example, reaction of the ε-amino groups of lysine with ethyltrifluorothioacetate in mildly basic solution yields a blocked amino acid residue whose adjacent peptide bond is no longer susceptible to hydrolysis by trypsin. Goldberger et al. Biochem., 1:401 (1962). Treatment of such a polypeptide with trypsin thus cleaves only at the arginyl residues. Polypeptides also can be modified to create peptide linkages that are susceptible to proteolytic enzyme catalyzed hydrolysis. For example, alkylation of cysteine residues with β-haloethylamines yields peptide linkages that are hydrolyzed by trypsin. Lindley, Nature, 178: 647 (1956).
In addition, chemical reagents that cleave polypeptide chains at specific residues can be used. Witcop, Adv. Protein Chem. 16: 221 (1961). For example, cyanogen bromide cleaves polypeptides at methionine residues. Gross & Witkip, J. Am Chem Soc., 83: 1510 (1961). Thus, by treating an inorganic ion receptor, such as, for example, a human calcium receptor or fragments thereof, with various combinations of modifiers, proteolytic enzymes and/or chemical reagents, numerous discrete overlapping peptides of varying sizes are generated. These peptide fragments can be isolated and purified from such digests by chromatographic methods.

Alternatively, fragments can be synthesized using an appropriate solid state synthetic procedure. Steward and Young, Solid Phase Peptide Synthesis, Freemantle, San Fansisco, CA (1968).

The fragments may be used in assays, as ion-binding agents, in the production of antibodies and the like. The fragments also may be selected to have desirable biological activities. For example, the fragment may include just the binding site, or a site which binds to agonists or antagonists (e.g. of calcium), as described herein. Such fragments are readily identified by those of ordinary skill in the art using routine methods to detect specific binding to the fragment. For example, in the case of a calcium receptor, a fragment to be tested can be expressed, using recombinant DNA methodology, from a fragment of the gene encoding the recombinant receptor. This fragment is then contacted with calcium or another chemical under appropriate association conditions to determine whether the calcium binds to the fragment. These fragments are useful in screening assays for agonists and antagonists of calcium, and for therapeutic effect where it is useful to remove calcium from serum, or other bodily tissues.

Other useful fragments include those that have only the external portion, membrane-spanning portion, or intracellular portion of the receptor. These portions are readily identified by comparison of the amino acid sequence of the receptor with those of known receptors, or by other standard methodology. These fragments are useful for forming chimeric receptors with fragments of other receptors so that a cell lacking a receptor may be formed with an intracellular portion which performs a desired function within that cell, and an extracellular portion which causes that cell to respond to the presence of ions, or those agonists or antagonists described herein. Such chimeric receptor genes when appropriately formulated are useful genetic therapies for a variety of diseases involving dysfunction of receptors or where modulation of receptor function provides a desirable effect in the patient. In addition, chimeric receptors can be constructed such that the intracellular domain is coupled to a desired enzymatic process which can be readily detected by colorometric, radiometric luminometric, spectrophotometric or fluorometric assays and is activated by interaction of the extracellular portion with its native ligand (e.g. calcium) or agonists and or antagonists of the invention. Cells expressing such chimeric receptors are the basis for facilitated screens for the discovery of new agonists and/or antagonists of the invention.

The invention also features muteins or analogs and other derivatives of isolated receptors, as described herein. Thus, the invention embraces not only naturally occurring proteins, but also such derivatives. Such derivatives have the desired receptor activity described herein and are generally identified by methods described herein. While examples of such proteins, and genes encoding them, are provided, these examples are not limiting in the invention but demonstrate only the variation that can be associated with such genes and proteins. Generally, the gene will have the amino acid sequence of the native receptor, but may be altered at one or more amino acid positions which do not significantly affect the receptor activity of the produced receptor protein. Thus, for example, in non-conserved regions of the calcium receptor protein (i.e., those regions not necessary for receptor activity) amino acids may be deleted, added or substituted. In more conserved regions, which are required for receptor activity, amino acids may be more conservatively substituted. For example, one or more amino acid residues within the sequence can be substituted by another amino acid of a similar polarity which acts as a functional equivalent. Substitutes for an amino acid within the sequence may be selected from other members of the class to which the amino acid belongs. The non-polar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include asparatic acid and glutamic acid.

Those of ordinary skill in the art will recognize that standard procedures can be used to determine the conserved and non-conserved regions of the protein using in vitro mutagenesis techniques or deletion analyses, and that all such derivatives are equivalent of those described below.

Also included within the scope of the invention are receptor proteins or unique fragments or derivatives thereof which are differentially modified during or after translation, e.g., by phosphorylation, glycosylation, crosslinking, acylation, proteolytic cleavage, linkage to an antibody molecule, membrane molecule or other ligand, (Ferguson et al., 1988, Ann. Rev. Biochem. 57:285-320).

In addition, recombinant nucleic acid sequences of the invention may be engineered so as to modify processing or expression of receptor sequences. For example, and not by way of limitation, the coding sequence may be combined with a promoter sequence and/or a ribosome binding site using well characterized methods, and thereby facilitate improve expression and bioavailability.

Additionally, a given recombinant coding sequence can be mutated in vitro or in vivo, to create variations in coding regions and/or form new restriction endonuclease sites or destroy preexisting ones, to facilitate further in vitro modification. Any technique for mutagenesis known in the art can be used including, but not limited to, in vitro site-directed mutagenesis (Hutchinson, et al., 1978, J. Biol. Chem. 253:6551), use of TAB® linkers (Pharmacia), PCR-directed mutagenesis, and the like.

In addition, codons may be modified such that while they encode an identical amino acid, that codon may be a preferred codon in the chosen expression system.

The invention also provides unique fragments of nucleic acid encoding an inorganic-ion receptor. The term "unique fragments" refers to portions of the nucleic acid that find no identical counterpart in known sequences as of the date of this invention. These fragments can be identified easily by an analysis of nucleic acid data bases existing as of the date of the invention to detect counterparts. Unique fragments are useful inter alia in cloning procedures and assays.

The invention further provides receptor binding agents including antibodies and/or fragments thereof which can be conjugated to a toxin moiety, or expressed along with a toxin moiety as a recombinant fusion protein. The toxin moiety will bind to and enter a target cell using interaction of the binding agent and the corresponding target cell surface receptor. The toxic moiety to which the agent, antibody and/or fragment is conjugated can be a protein such as, for example, pokeweed anti-viral protein, ricin, gelonin, abrin, diphtheria exotoxin, or Pseudomonas exotoxin. The toxin moiety can also be a high energy-emitting radionuclide such as cobalt-60. The chemical structure of the toxin moiety is not intended to limit the scope of the invention in any way. Those of ordinary skill in the art will recognize that a large variety of possible moieties can be linked to the binding agents. See, for example, "Conjugate Vaccines", Contributions to Microbiology and Immunology, J.M. Cruse and R.E. Lewis, Jr (eds). Carger Press, New York, (1989). It further is art recognized that many toxin moieties, including those specifically listed above, are considered pharmaceutically acceptable.

The conjugation of the binding agent to another moiety (e.g. bacterial toxin) can be accomplished by any chemical reaction that will bind the two molecules so long as both molecules retain their respective activity. This linkage can include many chemical mechanisms, for instance covalent binding, affinity binding, intercalation, coordinate binding and complexation. The preferred binding is, however, covalent binding. The covalent binding can be achieved either by direct condensation of existing side chains or by the incorporation of external bridging molecules. Many bivalent or polyvalent linking agents are useful in coupling protein molecules, such as an antibody, to other molecules. For example, representative coupling agents can include organic compounds such as thioesters, carbodiimides, succinimide esters, diisocyanates, glutaraldehydes, diazobenzenes and hexamethylene diamines. This listing is not intended to be exhaustive of the various classes of coupling agents known in the art but, rather, is exemplary of the more common coupling agents. (See Killen and Lindstrom 1984, "Specific killing of lymphocytes that cause experimental Autoimmune Myasthenia Gravis by toxin-acetylcholine receptor conjugates." Jour. Immun. 133:1335-2549; Jansen, F.K., H.E. Blythman, D. Carriere, P. Casella, O. Gros, P. Gros, J.C. Laurent, F. Paolucci, B. Pau, P. Poncelet, G. Richer, H. Vidal, and G.A. Voisin. 1982. "Immunotoxins: Hybrid molecules combining high specificity and potent cytotoxicity". Immunoloctical Reviews 62:185-216; and Vitetta et al., supra).

Using recombinant DNA techniques, the present invention provides for target cells including mammalian target cells which are engineered to express inorganic-ion receptors. For example, the genes for such receptors, cloned according to the methods set forth above, may be inserted into cells which naturally express the receptors such that the recombinant gene is expressed at much higher levels.

The present invention also allows to provide for experimental model systems for studying the physiological role of the receptors. In these model systems, the inorganic-ion receptors, fragments, or derivatives thereof, may be either supplied to the system or produced within the system. Such model systems could be used to study the effects or cell function of receptor excess or depletion. The experimental model systems may be used to study the effects in cell or tissue cultures, in whole animals, or in particular cells or tissues within whole animals or tissue culture systems, or over specified time intervals (including during embryogenesis). A preferred embodiment is assays involving the cloned receptors or parts thereof, including particularly those useful in high through-put drug screening assays.

A recombinant gene of the invention may be used to inactivate the endogenous gene by homologous recombination, and thereby create an inorganic-ion receptor deficient cell, tissue, or animal. For example, and not by way of limitation, a recombinant gene may be engineered to contain an insertional mutation (e.g.. the neo gene) which, when inserted into the genome of a recipient cell, tissue or animal, inactivates transcription of the receptor. Such a construct may be introduced into a cell, such as an embryonic stem cell, by a technique such as transfection, transduction, injection, etc. Stem cells lacking an intact receptor sequence may generate transgenic animals deficient in the receptor.

A "transgenic animal" is an animal having cells that contain DNA which has been artificially inserted into a cell, which DNA becomes part of the genome of the animal which develops from that cell. Preferred transgenic animals are primates, mice, rats, cows, pigs, horses, goats, sheep, dogs and cats. The transgenic DNA may encode for human inorganic-ion receptors. In a further embodiment of the invention, native expression in an animal may be reduced by providing an amount of anti-sense RNA or DNA effective to reduce expression of the receptor.

A variety of methods are available for the production of transgenic animals associated with this invention. DNA can be injected into the pronucleus of a fertilized egg before fusion of the male and female pronuclei, or injected into the nucleus of an embryonic cell (e.g., the nucleus of a two-cell embryo) following the initiation of cell division (Brinster et al., Proc. Nat. Acad. Sci. USA, 82: 4438-4442 (1985)). Embryos can be infected with viruses, especially retroviruses, modified to carry inorganic-ion receptor nucleotide sequences of the invention.

Pluripotent stem cells derived from the inner cell mass of the embryo and stabilized in culture can be manipulated in culture to incorporate nucleotide sequences of the invention. A transgenic animal can be produced from such cells through implantation into a blastocyst that is implanted into a foster mother and allowed to come to term.

Animals suitable for transgenic experiments can be obtained from standard commercial sources such as Charles River (Wilmington, MA), Taconic (Germantown, NY), Harlan Sprague Dawley (Indianapolis, IN), etc.

The procedures for manipulation of the rodent embryo and for microinjection of DNA into the pronucleus of the zygote are well known to those of ordinary skill in the art (Hogan et al., supra). Microinjection procedures for fish, amphibian eggs and birds are detailed in Houdebine and Chourrout, Experientia, 47: 897-905 (1991). Other procedures for introduction of DNA into tissues of animals are described in U.S. Patent No., 4,945,050 (Sandford et al., July 30, 1990).

By way of example only, to prepare a transgenic mouse, female mice are induced to superovulate. Females are placed with males, and the mated females are sacrificed by CO₂ asphyxiation or cervical dislocation and embryos are recovered from excised oviducts. Surrounding cumulus cells are removed. Pronuclear embryos are then washed and stored until the time of injection. Randomly cycling adult female mice are paired with vasectomized males. Recipient females are mated at the same time as donor females. Embryos then are transferred surgically.

The procedure for generating transgenic rats is similar to that of mice. See Hammer et al., Cell, 63:1099-1112 (1990).

Methods for the culturing of embryonic stem (ES) cells and the subsequent production of transgenic animals by the introduction of DNA into ES cells using methods such as electroporation, calcium phosphate/DNA precipitation and direct injection also are well known to those of ordinary skill in the art. See, for example, Teratocarcinomas and Embryonic Stem Cells, A Practical Approach, E.J. Robertson, ed., IRL Press (1987).

In cases involving random gene integration, a clone containing the sequence(s) of the invention is co-transfected with a gene encoding resistance. Alternatively, the gene encoding neomycin resistance is physically linked to the sequence(s) of the invention. Transfection and isolation of desired clones are carried out by any one of several methods well known to those of ordinary skill in the art (E.J. Robertson, supra).

DNA molecules introduced into ES cells can also be integrated into the chromosome through the process of homologous recombination. Capecchi, Science, 244: 1288-1292 (1989). Methods for positive selection of the recombination event (i.e., neo resistance) and dual positive-negative selection (i.e., neo resistance and gancyclovir resistance) and the subsequent identification of the desired clones by PCR have been described by Capecchi, supra and Joyner et al., Nature, 338: 153-156 (1989), the teachings of which are incorporated herein. The final phase of the procedure is to inject targeted ES cells into blastocysts and to transfer the blastocysts into pseudopregnant females. The resulting chimeric animals are bred and the offspring are analyzed by Southern blotting to identify individuals that carry the transgene.

Procedures for the production of non-rodent mammals and other animals have been discussed by others. See Houdebine and Chourrout, supra; Pursel et al., Science 244: 1281-1288 (1989); and Simms et al., Bio/Technology, 6: 179-183 (1988).

### Uses

Primary hyperparathyroidism (HPT) is characterized by hypercalcemia and elevated levels of circulating PTH. One of the major defects in HPT appears to be a diminished sensitivity of parathyroid cells to negative feedback regulation by extracellular Ca²⁺. Thus, in tissue from patients with primary HPT, the "set-point" for extracellular Ca²⁺ is shifted to the right so that higher than normal concentrations of extracellular Ca²⁺ are required to depress PTH secretion. Moreover, in primary HPT, even high concentrations of extracellular Ca²⁺ often depress PTH secretion only partially. In secondary (uremic) HPT, a similar increase in the set-point for extracellular Ca²⁺ is observed even though the degree to which Ca²⁺ suppresses PTH secretion is normal. The changes in PTH secretion are paralleled by changes in [Ca²⁺]ᵢ: the set-point for extracellular Ca²⁺-induced increases in [Ca²⁺]ᵢ is shifted to the right and the magnitude of such increases is reduced. Moreover, staining of tissue with a monoclonal antibody that appears to recognize the Ca²⁺ receptor is diminished in adenomatous and hyperplastic parathyroid cells.

The Ca²⁺ receptor constitutes a discrete molecular entity for pharmacological intervention. Molecules that mimic or antagonize the action of extracellular Ca²⁺ are beneficial in the long-term management of both primary and secondary HPT. Such molecules provide the added impetus required to suppress PTH secretion which the hypercalcemic condition alone cannot achieve. Such molecules with greater efficacy than extracellular Ca²⁺ may overcome the apparent nonsuppressible component of PTH secretion which is particularly troublesome in adenomatous tissue. Alternatively or additionally, such molecules can depress synthesis of PTH, as prolonged hypercalcemia has been shown to depress the levels of preproPTH mRNA in bovine and human adenomatous parathyroid tissue. Prolonged hypercalcemia also depresses parathyroid cell proliferation in vitro, so calcimimetics can also be effective in limiting the parathyroid cell hyperplasia characteristic of secondary HPT.

Other cells in the body can respond directly to physiological changes in the concentration of extracellular Ca²⁺. Calcitonin secretion from parafollicular cells in the thyroid (C-cells) is regulated by changes in the concentration of extracellular Ca²⁺. Renin secretion from juxtaglomerular cells in the kidney, like PTH secretion, is depressed by increased concentrations of extracellular Ca²⁺. Extracellular Ca²⁺ causes the mobilization of intracellular Ca²⁺ in these cells. Other kidney cells respond to calcium as follows: elevated Ca²⁺ inhibits formation of 1.25(ON)₂ Vitamin D by proximal tubular cells, stimulates production of calcium-binding protein in distal tubular cells, and inhibits tubular reabsorption of Ca²⁺ and Mg²⁺ and the action of vasopressin on the medullary thick ascending limb of Henle's loop (MTAL), reduces vasopressin action in the cortical collecting duct cells, and affects vascular smooth muscle cells in blood vessels of the renal glomerulus. Calcium promotes the differentiation of intestinal goblet cells, mammary cells, and skin cells. It also inhibits atrial natriuretic peptide secretion from cardiac atria, reduces cAMP accumulation in platelets, alters gastrin and glucagon secretion, and acts on vascular smooth muscle cells to modify cell secretion of vasoactive factors. Isolated osteoclasts respond to increases in the concentration of extracellular Ca²⁺ with corresponding increases in [Ca²⁺]ᵢ that arise partly from the mobilization of intracellular Ca²⁺. Increases in [Ca²⁺]ᵢ in osteoclasts are associated with an inhibition of functional responses (bone resorption) analogous to PTH secretion in parathyroid cells. Release of alkaline phosphatase from bone-forming osteoblasts is directly stimulated by calcium. Thus, there are sufficient indications to suggest that Ca²⁺, in addition to its ubiquitous role as an intracellular signal, also functions as an extracellular signal to regulate the responses of certain specialized cells. Molecules of this invention can be used in the treatment of diseases associated with disrupted Ca²⁺ responses in these cells.

Cloning the Ca²⁺ receptor on parathyroid cells and other cells will allow the presence of homologous proteins in other cells to be directly assessed. A family of structurally homologous Ca²⁺ receptor proteins can thus be obtained. Such receptors will allow understanding of how these cells detect extracellular Ca²⁺ and enable evaluation of the mechanism(s) as a site of action for the therapeutics described herein effective in the treatment of HPT, osteoporosis, and hypertension, and novel therapies for other bone and mineral-related diseases.

Other uses are discussed above. For example, recombinant Ca²⁺ receptor proteins may be used in therapy, and introduced by standard methods, e.g., by transfection of nucleic acid encoding that protein. In addition, such protein is useful in assays for calcimimetic molecules of this invention.

The following examples illustrate the invention but do not limit its scope.

Methods for the culturing of embryonic stem (ES) cells and the subsequent production of transgenic animals by the introduction of DNA into ES cells using methods such as electroporation, calcium phosphate/DNA precipitation and direct injection also are well known to those of ordinary skill in the art. See, for example, Teratocarcinomas and Embryonic Stem Cells, A Practical Approach, E.J. Robertson, ed., IRL Press (1987).

In cases involving random gene integration, a clone containing the sequence(s) of the invention is co-transfected with a gene encoding resistance. Alternatively, the gene encoding neomycin resistance is physically linked to the sequence(s) of the invention. Transfection and isolation of desired clones are carried out by any one of several methods well known to those of ordinary skill in the art (E.J. Robertson, supra).

DNA molecules introduced into ES cells can also be integrated into the chromosome through the process of homologous recombination. Capecchi, Science, 244: 1288-1292 (1989). Methods for positive selection of the recombination event (i.e., neo resistance) and dual positive-negative selection (i.e., neo resistance and gancyclovir resistance) and the subsequent identification of the desired clones by PCR have been described by Capecchi, supra and Joyner et al., Nature, 338: 153-156 (1989), the teachings of which are incorporated herein. The final phase of the procedure is to inject targeted ES cells into blastocysts and to transfer the blastocysts into pseudopregnant females. The resulting chimeric animals are bred and the offspring are analyzed by Southern blotting to identify individuals that carry the transgene.

Procedures for the production of non-rodent mammals and other animals have been discussed by others. See Houdebine and Chourrout, supra; Pursel et al., Science 244: 1281-1288 (1989); and Simms et al., Bio/Technology, 6: 179-183 (1988).

### Uses

Primary hyperparathyroidism (HPT) is characterized by hypercalcemia and elevated levels of circulating PTH. One of the major defects in HPT appears to be a diminished sensitivity of parathyroid cells to negative feedback regulation by extracellular Ca²⁺. Thus, in tissue from patients with primary HPT, the "set-point" for extracellular Ca²⁺ is shifted to the right so that higher than normal concentrations of extracellular Ca²⁺ are required to depress PTH secretion. Moreover, in primary HPT, even high concentrations of extracellular Ca²⁺ often depress PTH secretion only partially. In secondary (uremic) HPT, a similar increase in the set-point for extracellular Ca²⁺ is observed even though the degree to which Ca²⁺ suppresses PTH secretion is normal. The changes in PTH secretion are paralleled by changes in [Ca²⁺]ᵢ: the set-point for extracellular Ca²⁺-induced increases in [Ca²⁺]ᵢ is shifted to the right and the magnitude of such increases is reduced. Moreover, staining of tissue with a monoclonal antibody that appears to recognize the Ca²⁺ receptor is diminished in adenomatous and hyperplastic parathyroid cells.

The Ca²⁺ receptor constitutes a discrete molecular entity for pharmacological intervention. Molecules that mimic or antagonize the action of extracellular Ca²⁺ are beneficial in the long-term management of both primary and secondary HPT. Such molecules provide the added impetus required to suppress PTH secretion which the hypercalcemic condition alone cannot achieve. Such molecules with greater efficacy than extracellular Ca²⁺ may overcome the apparent nonsuppressible component of PTH secretion which is particularly troublesome in adenomatous tissue. Alternatively or additionally, such molecules can depress synthesis of PTH, as prolonged hypercalcemia has been shown to depress the levels of preproPTH mRNA in bovine and human adenomatous parathyroid tissue. Prolonged hypercalcemia also depresses parathyroid cell proliferation in vitro, so calcimimetics can also be effective in limiting the parathyroid cell hyperplasia characteristic of secondary HPT.

Other cells in the body can respond directly to physiological changes in the concentration of extracellular Ca²⁺. Calcitonin secretion from parafollicular cells in the thyroid (C-cells) is regulated by changes in the concentration of extracellular Ca²⁺. Renin secretion from juxtaglomerular cells in the kidney, like PTH secretion, is depressed by increased concentrations of extracellular Ca²⁺. Extracellular Ca²⁺ causes the mobilization of intracellular Ca²⁺ in these cells. Other kidney cells respond to calcium as follows: elevated Ca²⁺ inhibits formation of 1,25(OH)₂ Vitamin D by proximal tubular cells, stimulates production of calcium-binding protein in distal tubular cells, and inhibits tubular reabsorption of Ca²⁺ and Mg²⁺ and the action of vasopressin on the medullary thick ascending limb of Henle's loop (MTAL), reduces vasopressin action in the cortical collecting duct cells, and affects vascular smooth muscle cells in blood vessels of the renal glomerulus. Calcium promotes the differentiation of intestinal goblet cells, mammary cells, and skin cells. It also inhibits atrial natriuretic peptide secretion from cardiac atria, reduces CAMP accumulation in platelets, alters gastrin and glucagon secretion, and acts on vascular smooth muscle cells to modify cell secretion of vasoactive factors. Isolated osteoclasts respond to increases in the concentration of extracellular Ca²⁺ with corresponding increases in [Ca²⁺]ᵢ that arise partly from the mobilization of intracellular Ca²⁺. Increases in [Ca²⁺]ᵢ in osteoclasts are associated with an inhibition of functional responses (bone resorption) analogous to PTH secretion in parathyroid cells. Release of alkaline phosphatase from bone-forming osteoblasts is directly stimulated by calcium. Thus, there are sufficient indications to suggest that Ca²⁺, in addition to its ubiquitous role as an intracellular signal, also functions as an extracellular signal to regulate the responses of certain specialized cells. Molecules of this invention can be used in the treatment of diseases associated with disrupted Ca²⁺ responses in these cells.

Cloning the Ca²⁺ receptor on parathyroid cells and other cells will allow the presence of homologous proteins in other cells to be directly assessed. A family of structurally homologous Ca²⁺ receptor proteins can thus be obtained. Such receptors will allow understanding of how these cells detect extracellular Ca²⁺ and enable evaluation of the mechanism(s) as a site of action for the therapeutics described herein effective in the treatment of HPT, osteoporosis, and hypertension, and novel therapies for other bone and mineral-related diseases.

Other uses are discussed above. For example, recombinant Ca²⁺ receptor proteins may be used in therapy, and introduced by standard methods, e.g., by transfection of nucleic acid encoding that protein. In addition, such protein is useful in assays for calcimimetic molecules of this invention.

The following examples illustrate the invention but do not limit its scope.

### Examples

In the studies described herein, a variety of organic molecules were found to mobilize intracellular Ca²⁺ and depress PTH secretion in parathyroid cells.
These molecules are structurally diverse but most have a net positive charge at physiological pH. The cationic nature of the organic molecules plays an important role but is not the sole factor determining activity.

### Example 1: Screening Calcimimetic Molecules on Bovine Parathyroid cells

Dissociated bovine parathyroid cells were purified on gradients of Percoll and cultured overnight in serum- free medium. The cells were subsequently loaded with fura-2 and the concentration of free intracellular Ca²⁺ measured fluorimetricly. Changes in [Ca²⁺]ᵢ were used to screen for molecules active at the Ca²⁺ receptor. To be considered a calcimimetic, a molecule was required to show the normal effects caused by increasing extracellular Ca²⁺ and triggered by the activation of the Ca²⁺ receptor. That is,
1) The molecule must elicit an increase in [Ca²⁺]ᵢ; the increase in [Ca²⁺]ᵢ may persist in the absence of extracellular Ca²⁺ and/or the molecule may potentiate increases in [Ca²⁺]ᵢ elicited by extracellular Ca²⁺.
2) The molecule must cause a decrease in isoproterenol-stimulated cyclic AMP formation which is blocked by pertussis toxin;
3) The molecule must inhibit PTH secretion over the same range of concentrations that cause the increase in [Ca²⁺]ᵢ; and
4) The concentration-response curves for increases in [Ca²⁺]ᵢ and PTH secretion by the molecule must be shifted to the right by a PKC activator, such as phorbol myristate acetate (PMA).

Several structurally different classes of molecules were tested: polyamines, aminoglycoside antibiotics, protamine, and polymers of lysine or arginine. The structures of these molecules are depicted in Figure 1. Included in Figure 1 are the net positive charge of the molecules and their EC₅₀'s for evoking the mobilization of intracellular Ca²⁺ in bovine parathyroid cells.

In general, the greater the net positive charge on the molecule, the greater its potency in causing the mobilization of intracellular Ca²⁺. However, some striking exceptions to this apparent rule have been found as discussed below.

As can be seen from the figures, spermine, neomycin B, and protamine evoked rapid and transient increases in [Ca²⁺]ᵢ in fura-2-loaded bovine parathyroid cells (Figs. 6, 7, 11). They did not, however, cause sustained, steady-state increases in [Ca²⁺]ᵢ in bovine parathyroid cells (Fig. 6, 11), although they did in human parathyroid cells (Fig. 19). In this respect, they resembled the cytosolic Ca²⁺ response elicited by extracellular Mg²⁺, which causes the mobilization of intracellular Ca²⁺ unaccompanied by an influx of extracellular Ca²⁺ in bovine cells (Fig. 11b). Transient increases in [Ca²⁺]ᵢ elicited by spermine, neomycin B, or protamine were not blocked by low concentrations (1 µM) of La³⁺ or Gd³⁺ (Fig. 11f,g). Cytosolic Ca²⁺ transients elicited by the molecular polycations persisted in the absence of extracellular Ca²⁺ but were blocked when cellular stores of Ca²⁺ were depleted by pretreatment with ionomycin (Figs. 7; 11h,i). All these molecules therefore cause the mobilization of intracellular Ca²⁺ in parathyroid cells.

It was additionally shown that the molecular polycations mobilized the same pool of intracellular Ca²⁺ as that used by extracellular Ca²⁺. Thus, increasing the concentration of extracellular Ca²⁺ progressively inhibited the transient increases in [Ca²⁺]ᵢ evoked by spermine (Fig. 6). Conversely, a maximally effective concentration of spermine or neomycin B (Fig. 12) blocked transient, but not steady-state increases in [Ca²⁺]ᵢ evoked by extracellular Ca²⁺.

Significantly, spermine, neomycin B, and protamine inhibited PTH secretion to the same extent as extracellular Ca²⁺. These inhibitory effects on secretion were obtained at concentrations that caused the mobilization of intracellular Ca²⁺ (Figs. 8, 13). These findings are relevant to understanding the mechanisms contributing to the regulation of PTH secretion by extracellular Ca²⁺. Because a variety of inorganic polycations all inhibit secretion, yet only extracellular Ca²⁺ causes sustained, steady-state increases in [Ca²⁺]ᵢ, such increases in [Ca²⁺]ᵢ cannot be importantly involved in the regulation of secretion. Mobilization of intracellular Ca²⁺, rather than the influx of extracellular Ca²⁺, is the essential mechanism associated with the inhibition of PTH secretion. This is important because it defines the sufficient mechanism to be affected if a molecule is to affect PTH secretion; molecules stimulating selectively the influx of extracellular Ca²⁺ will be relatively ineffective in suppressing PTH secretion. In contrast, molecules causing solely the mobilization of intracellular Ca²⁺ should be just as efficacious as extracellular Ca²⁺ in suppressing PTH secretion.

Like the mobilization of intracellular Ca²⁺ elicited by extracellular Ca²⁺, that elicited by molecular polycations was depressed by PMA. A representative experiment showing the preferential inhibitory effects of PMA on cytosolic Ca²⁺ transients elicited by spermine is shown in Fig. 14. Cytosolic Ca²⁺ transients evoked by ATP were unaffected, even when a submaximal concentration of ATP was used. The effect of PMA on cytosolic Ca²⁺ transients elicited by the molecular polycations paralleled its effect on responses to extracellular Ca²⁺; in both cases there was a shift to the right in the concentration-response curve (Fig. 15). The depressive effects of PMA on [Ca²⁺]ᵢ were accompanied by potentiating effects on secretion which were overcome at higher concentrations of the organic polycations (Fig. 16).

The mobilization of intracellular Ca²⁺ elicited by molecular polycations was associated with increases in the formation of inositol phosphates. For example, protamine caused a rapid (within 30 s) increase in the formation of IP₃ which was accompanied by a rise in levels of IP₁. Both these effects were dependent on the concentration.of extracellular protamine (Fig. 17). Moreover, pretreatment with PMA blunted the formation of inositol phosphates elicited by molecular polycations. Representative results obtained with spermine are presented in Fig. 18.

Spermine, neomycin B, and protamine depressed isoproterenol-induced increases in cyclic AMP. Like the inhibitory effects of extracellular Ca²⁺ on cyclic AMP formation, those caused by molecular polycations were blocked by pretreatment with pertussis toxin (Table 2).

**Table 2**

| | cyclic AMP (% of control) | |
|---|---|---|
| | control | +PTx |
| 0.5 mM Ca²⁺ | 100 | 106 ± 8 |
| 2.0 mM Ca²⁺ | 19 ± 4 | 94 ± 2 |
| 0.5 mM Ca²⁺, 200 µM Spermine | 23 ± 5 | 93 ± 6 |
| 0.5 mM Ca²⁺, 30 µM Neomycin B | 28 ± 8 | 87 ± 6 |
| 0.5 mM Ca²⁺, 2 µg/ml Protamine | 20 ± 4 | 89 ± 9 |

Pertussis toxin (PTx) blocks the inhibitory effects of extracellular Ca²⁺ and molecular polycations on cyclic AMP formation. Bovine parathyroid cells were cultured for 16 h with or without 100 ng/ml pertussis toxin. The cells were subsequently washed and incubated for 15 min with 10 µM isoproterenol with or without the indicated concentrations of extracellular Ca²⁺ or molecular polycations. Total cyclic AMP (cells + supernatant) was determined by RIA and the results are expressed as a percentage of the levels obtained in 0.5 mM Ca²⁺ (112 ± 17 pmole/10⁶ cells). Each value is the mean ± SEM of three experiments.

In human parathyroid cells, extracellular Mg²⁺ elicited a sustained, steady-state increase in [Ca²⁺]ᵢ in addition to a rapid transient increase (Fig. 10). As in bovine parathyroid cells responding to extracellular Ca²⁺, the steady-state increase in [Ca²⁺]ᵢ evoked by Mg²⁺ in human parathyroid cells results from Ca²⁺ influx through voltage-insensitive channels (Fig. 10a). This effect of Mg²⁺ on steady-state [Ca²⁺]ᵢ in human parathyroid cells is seen in both adenomatous and hyperplastic tissue.

Neomycin B and spermine were tested for effects on [Ca²⁺]ᵢ in human parathyroid cells prepared from adenomatous tissue. Representative results with neomycin B are shown in Fig. 19. Neomycin B caused not only a transient but additionally a steady-state increase in [Ca²⁺]ᵢ in human parathyroid cells (Fig. 19a). Thus, in human cells, the pattern of change in [Ca²⁺]ᵢ evoked by extracellular Ca²⁺, Mg²⁺ or neomycin B is very similar.

Cytosolic Ca²⁺ transients elicited by neomycin B persisted in the presence of La³⁺ (1µM) and absence of extracellular Ca²⁺. Neomycin B therefore causes the mobilization of intracellular Ca²⁺ in human parathyroid cells. Neomycin B inhibited PTH secretion from human parathyroid cells at concentrations that caused the mobilization of intracellular Ca²⁺ (Fig. 13). There were, however, some differences in the responses of human and bovine parathyroid cells to neomycin B. The EC₅₀ of neomycin B for the mobilization of intracellular Ca²⁺ was 40 µM in bovine and 20 µM in human parathyroid cells (cf. Figs. 13 and 15), whereas the potency of spermine was similar in bovine and human parathyroid cells (EC₅₀ = 150 µM). Thus, although bovine cells can be used for initial studies to screen test molecules for activity, it is important to perform follow-up studies using human parathyroid cells.

To assess the effects of molecular polycations on C-cells, a neoplastic cell line, derived from a rat medullary thyroid carcinoma (rMTC 6-23 cells) was used. Both spermine (10 mM) and neomycin B (5 mM) were without effect on basal [Ca²⁺]ᵢ in these cells. Nor did either molecule affect the response to the subsequent addition of extracellular Ca²⁺. Representative results documenting the lack of effect of neomycin B are shown in Fig 21. Neomycin B (1 mM) or spermine (1 or 5 mM) failed to evoke any increase in [Ca²⁺]ᵢ in osteoclasts (Fig. 23). In the trace shown, there appeared to be some potentiation of the response to a subsequent increase in the concentration of extracellular Ca²⁺, although this was not a consistent finding. In two other cells, spermine (5 mM) was again without effect on basal [Ca²⁺]ᵢ and caused a small inhibition (about 15%) of the extracellular Ca²⁺-induced increase in [Ca²⁺]ᵢ. In a third cell, neomycin B (5 mM) was without effect on basal [Ca²⁺]ᵢ and did not affect increases in [Ca²⁺]ᵢ elicited by extracellular Ca²⁺. The overall picture that develops from these studies is that spermine and neo mycin B are without effect on basal or stimulated levels of cytosolic Ca²⁺ in osteoclasts.

The failure of the molecular polycations to affect the Ca²⁺-sensing mechanisms of C-cells or osteoclasts demonstrates the ability to discover or design novel lead molecules that act specifically on the parathyroid cell Ca²⁺ receptor or otherwise modulate one or more functions of the parathyroid cell's normal response to [Ca²⁺].

### Example 2: Molecule Screening on Human Parathyroid Cells

Spermine and neomycin were tested for effects on [Ca²⁺]ᵢ in human parathyroid cells obtained from glands removed by surgery and prepared as in Example 1. In human parathyroid cells, spermine was found to cause only a small increase in [Ca²⁺]ᵢ when tested at a concentration of 300 µM.

Neomycin, on the other hand, evoked a large increase in [Ca²⁺]ᵢ in human parathyroid cells when tested at a concentration of 20 pM. The magnitude of the response elicited by neomycin was equal to that evoked by a maximally effective concentration of extracellular Ca²⁺.

### Example 3: Molecule Screening on Xenopus Oocytes

Oocytes injected with mRNA from human parathyroid cells express the Ca²⁺ receptor and mobilize intracellular Ca²⁺ in response to a variety of extracellular inorganic di- and trivalent cations. Using this screen allows one to test for an action directly on the Ca²⁺ receptor. Oocytes expressing the Ca²⁺ receptor also responded to several molecules active on intact parathyroid cells when screened as follows. Hexacyclen caused the mobilization of intracellular Ca²⁺ at a concentration of 135 µM. Neomycin (100 µM) and NPS 382 (5 mM) were also effective. This offers rather compelling evidence showing that these molecules act on the Ca²⁺ receptor or on some other protein intimately associated with its function.

For example, we have been able to detect Ca²⁺ receptor expression in oocytes by measuring ⁴⁵Ca²⁺ mobilization. In these experiments, oocytes were injected with bovine parathyroid mRNA or water and, after 72 hours exposed to serum or 10 mM neomycin. Prior to being stimulated, oocytes were loaded with ⁴⁵Ca²⁺. Stimulation with serum for 20 min resulted in intracellular ⁴⁵Ca²⁺ release representing a 45% increase compared to mock challenge with buffer. Challenge with 10 mM neomycin for 20 min. resulted in a 76% increase in ⁴⁵Ca²⁺ release. The assay is sensitive enough for use in cloning the Ca²⁺ receptor, and has the advantage of a higher throughout than the electrophysiological measurement of Ca²⁺ activated Cl⁻ current.

In another example, human osteoclastoma tissue was obtained from bone biopsy tissue. Oocytes injected with mRNA isolated from this tissue were challenged with 30 mM Ca²⁺. Controls did not respond while 8 of 12 oocytes injected with osteoclastoma mRNA responded appropriately (Fig. 34). These experiments provide the first evidence that the Ca²⁺ response of osteoclasts to extracellular Ca²⁺ is in fact genetically encoded. The results also indicate that the osteoclast Ca²⁺ receptor may be cloned by expression in Xenopus oocytes.

### Example 4: Molecule Screening on Rat Osteoclasts

The different sensitivities of parathyroid cells and rat osteoclasts to extracellular Ca²⁺ suggest that their Ca²⁺ receptors are different. While parathyroid cells respond to extracellular Ca²⁺ concentrations between 0.5 and 3 mM, osteoclasts respond only when the level of extracellular Ca²⁺ increases beyond 5 mM. This rather high concentration of Ca²⁺ is nonetheless physiological for osteoclasts; as they resorb bone, the local concentration of extracellular Ca²⁺ may reach levels as high as 30 mM.

Molecule screening with rat osteoclasts was performed as follows. Osteoclasts were obtained from the long bones of neonatal rats. [Ca²⁺]ᵢ was measured in single cells using the fluorimetric indicator indo-1. Spermine, spermidine, neomycin, and verapamil were tested, and none of these caused any large increase in [Ca²⁺]ᵢ in osteoclasts (although small responses were detected).

At a concentration of 1 mM, spermidine caused a small increase in [Ca²⁺]ᵢ (about 10% of that evoked by a maximal concentration of extracellular Ca²⁺). Neither neomycin (10 mM) nor spermine (10 or 20 mM) caused increases in [Ca²⁺]ᵢ in rat osteoclasts. Neomycin (10 mM) did not block the increase in [Ca²⁺]ᵢ elicited by the subsequent addition of 25 mM extracellular Ca²⁺. Pretreatment with spermine (20 mM), however, did depress the response to extracellular Ca²⁺. Verapamil (100 µM) caused no detectable increase in [Ca²⁺]ᵢ but it did block the response to extracellular Ca²⁺.

Comparisons between osteoclasts and parathyroid cells show that molecules active on the latter are relatively ineffective in osteoclasts. This demonstrates that drugs that target a specific Ca²⁺ receptor without affecting those receptor types present on other Ca²⁺-sensing cells are readily developed. Similarly, drugs active at two or more such Ca²⁺ receptors may also be developed.

### Screening for Calcimimetic and Calcilytic Activity on the Osteoclast Calcium Receptor

Compounds possessing activity on the osteoclast calcium receptor can be discovered by measuring [Ca²⁺]ᵢ in single rat osteoclasts as described above. An improved assay enables moderate-to-high levels of compound throughout. This new method is based on the use of rabbit osteoclasts which can be obtained in high yield (10⁵ per animal) and purity (95% of the cells are osteoclasts). The purity of the rabbit osteoclast preparation allows measurements of [Ca²⁺]ᵢ to be performed on populations of cells. Because the recorded fluorescence signal is an averaged population response, intercellular variability is minimized and the precision of the assay is greatly increased. This, in turn, enables more compounds to be screened for activity.

Rabbit osteoclasts are prepared from 6-day old bunnies. The animals are sacrificed by decapitation and the long bones removed and placed into osteoclast medium (OC medium: alpha-minimum essential medium containing 5% fetal bovine serum and penicillin/streptomycin). The bones are cut into sections with a scalpel and placed in 2 ml of OC media in a 50 ml conical centrifuge tube. The bone sections are minced with scissors until a fairly homogeneous suspension of bone particles is obtained. The suspension is then diluted with 25 ml of OC media and the preparation swirled gently ("vortexed") for 30 seconds. The bone particles are allowed to settle for 2 minutes after which the supernatant is removed and added to a 50 ml centrifuge tube. The bone particles are resuspended in OC media, swirled, sedimented and harvested as just described. The supernatants from the two harvests are combined and centrifuged and the resulting cellular pellet resuspended in Percoll. The suspension is then centrifuged and the white viscous band just below the meniscus is removed and washed with OC media. The Percoll centrifugation step results in a significant improvement in purity and allows osteoclasts to be plated at high densities, suitable for measuring [Ca²⁺]ᵢ in populations of cells. The cells are plated onto glass cover slips appropriate for measuring [Ca²⁺]ᵢ according to one of the methods described below. If necessary, the purity of the preparation can be improved. In this case, the cells are cultured overnight and then rinsed with Ca²⁺- and Mg²⁺-free buffer. The cell monolayer is then immersed in Ca²⁺-and Mg²⁺-free buffer containing 0.02% EDTA and 0.001% pronase for 5 min. This buffer is then removed and replaced with OC media and the cells allowed to recover for 1 to 2 hours before loading the cells with fluorimetric indicator and measuring [Ca²⁺]ᵢ as described below.

In one embodiment, this technique allows the measurement of [Ca²⁺]ᵢ in populations of osteoclasts using fluorescence microscopy. The purified osteoclasts are allowed to attach to 25 mm diameter glass cover slips and then loaded with indo-1. The cover slips are secured into a superfusion chamber and placed onto the stage of a fluorescence microscope. The use of a low power objective (x4) allows a field containing 10 to 15 osteoclasts to be visualized. In one variation, the fluorescence of each cell in the field can be recorded simultaneously and stored separately for later analysis. Changes in [Ca²⁺]ᵢ of each cell can be estimated and the average response of all cells in the field calculated. In another variation, the fluorescence from the entire field of cells can be recorded and processed immediately. In either variation, the final data is in the form of an average response from the cells present in the microscopic field. Because of this, intercellular variability is minimized and precision of the assay greatly increased. This method enables 10-20 compounds per week to be screened for activity on the osteoclast calcium receptor.

In a more preferred embodiment, this technique allows the measurement of [Ca²⁺]ᵢ in populations of osteoclasts using a conventional fluorimeter. The purified osteoclasts are allowed to attach to rectangular glass cover slips. In one variation, a standard quartz cuvette (1cm²) is used and the glass coverslips are 2 x 1.35 cm. In another variation, a microcuvette is used (0.5 cm²) and the glass coverslips are 1 x 0.75 cm. In either case the cells are loaded with fura-2 or some other suitable fluorimetric indicator for measuring [Ca²⁺]ᵢ. The fluorescence of indicator-loaded cells is recorded as described above for bovine parathyroid cells. This method allows a higher throughput than fluorescence microscopy and enables 20-50 compounds per week to be evaluated for activity on the osteoclast calcium receptor.

In a most preferred embodiment, the technique can be used to measure [Ca²⁺]ᵢ in osteoclasts in a 96-well plate. The purified osteoclasts are plated at high density into each well of a 96-well plate and subsequently loaded with a suitable fluorimetric indicator. The fluorescence of each well is recorded using a fluorimetric plate reader. This method has the potential of becoming fully automated using robotics a would enable high throughput screening in which 50 to 100 compounds per week could be screened.

### Other Ca²⁺ Receptor Examples

The following examples demonstrate that, just as there are subtypes of receptors for molecular ligands, so too do there appear to be subtypes of Ca²⁺ receptors that can be differentially affected by drugs. The parathyroid cell Ca²⁺ receptor senses levels of extracellular Ca²⁺ around 1.5 mM whereas the Ca²⁺ receptor on the osteoclast responds to levels around 10 mM (Fig. 22). Neomycin or spermine, which activate the parathyroid cell Ca²⁺ receptor, fail to affect the Ca²⁺ receptors on C-cells or osteoclasts (Figs. 21 and 23). These data constitute the first evidence for pharmacologically distinct subtypes of Ca²⁺ receptors and these data are being used to design and develop drugs that act selectively on a particular type of Ca²⁺ receptor. Indeed, testing of lead molecules demonstrate such cell-specific effects. For example, NPS 449, which elicits increases in [Ca²⁺]ᵢ in osteoclasts is without effect on [Ca²⁺]ᵢ in parathyroid cells. Conversely, NPS 447, which activates the parathyroid cell Ca²⁺ receptor, is effective in activating the osteoclast Ca²⁺ receptor only at concentrations 10-fold higher. Finally, agatoxin 489, although not very potent in activating the C-cell Ca²⁺ receptor (EC₅₀ = 150 µM), is a quite potent activator of the parathyroid cell Ca²⁺ receptor (EC₅₀ = 3 µM). The lead molecules presently under development will affect selectively the activity of a specific type of Ca²⁺-sensing cell in vivo.

Drugs with less specificity might not necessarily be therapeutically undesirable. Thus, depressing osteoclast activity and stimulating calcitonin secretion are two different approaches to inhibiting bone resorption. Drugs that target the Ca²⁺ receptors on both of these cells might be very effective therapies for osteoporosis. Because PTH is also involved in regulating bone metabolism, drugs acting on the parathyroid cell Ca²⁺ receptor may also be useful in the treatment and/or prevention of osteoporosis.

Results of some test molecules are shown below. In Table 6, the comparative activity of calcimimetic molecules is shown. Bovine parathyroid cells and C-cells (rMTC 6-23 cells) were loaded with fura-2, and rat osteoclasts with indo-1 and the potency of the indicated molecules to mobilize intracellular Ca²⁺ determined by constructing cumulative concentration-response curves. Molecules listed as "inactive" did not alter [Ca²⁺]ᵢ when tested at a concentration of 1 mM.

**Table 6**

| | EC₅₀(µM) | | |
|---|---|---|---|
| COMPOUND | PARATHYROID | OSTEOCLAST | C-CELL |
| NPS R-568 | 0.60 | 200 | >300 |
| NPS S-568 | 30 | --- | --- |
| NPS R-467 | 2 | >100 | --- |
| NPS S-467 | >30 | --- | --- |
| NPS 017 | 6 | inactive | 150 |
| NPS 447 | 9 | 150 | --- |
| NPS 456* | 15 | 200 | >100 |
| NPS 015 | 22 | --- | inactive |
| NPS 109 | 40 | >300 | 5 |
| NPS 449 | inactive | 150 | --- |
| NPS 468* | 30 | 250 | --- |
| spermine | 150 | inactive | inactive |
| neomycin | 40 | inactive | inactive |

| | | | |
|---|---|---|---|
| *racemic mixture; "inactive" is defined as causing no increase in cytosolic Ca²⁺ at a concentration of 1-5 mM. | | | |

### Example 5: Lead Molecules for Parathyroid Ca²⁺ Receptor

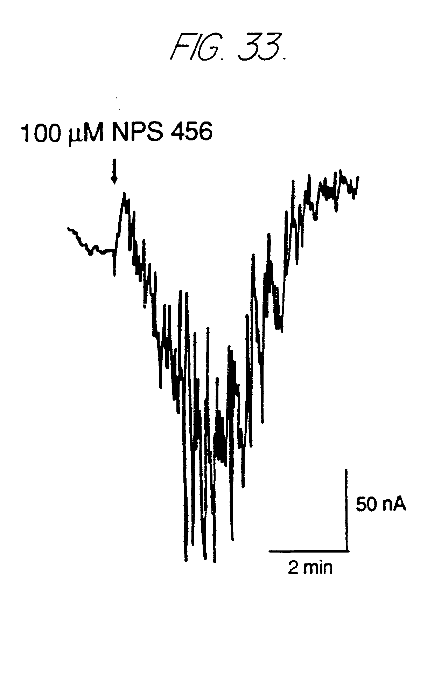

Structure-activity studies using polyamines and arylalkylamines led to the testing of molecules structurally akin to NPS 456. NPS 456 is a potent activator of the parathyroid cell Ca²⁺ receptor. This molecule is notable because it possess only one positive charge yet is much more potent than many polybasic molecules. Brief (2 min) pretreatment with PMA shifts the concentration-response curve for NPS 456 to the right. This indicates that NPS 456 acts through the same mechanism used by extracellular Ca²⁺. NPS 456 evokes the mobilization of intracellular Ca²⁺ in Xenopus oocytes expressing the parathyroid cell Ca²⁺ receptor, which demonstrates a direct action on the Ca²⁺ receptor (Fig. 33). Moreover, NPS 456 contains a chiral carbon, and therefore exists in two isomeric forms. Both isomers have been synthesized and examined for activity. The R-isomer, NPS 447, is 12 times more potent than the S-isomer, NPS 448 (Fig. 28). This is the first demonstration that a Ca²⁺ receptor can recognize an organic molecule in a stereospecific manner.

Because NPS 447 is a structurally simple molecule with selective and potent effects on the parathyroid cell Ca²⁺ receptor, structure-activity studies around this lead molecule are simple. The aim of these studies is to generate an array of related molecules with various characteristics from which the final development candidate can be selected. This effort has already revealed some of the structural domains of NPS 447 that contribute to activity and potency. For example, the novel compound NPS 459 is an analog of NPS 447 that is smaller (MW < 240) yet nearly as potent as the parent molecule, whereas several other analogs are relatively inactive. The most interesting molecules from this analog project can be put into in vivo testing for effects on PTH secretion and serum Ca²⁺ levels (see Examples 15, 16, 17, 18 and 23).

The novel compound NPS 467 is an even smaller molecule than NPS 447 yet the former is about 3-fold more potent than the latter in causing increases in [Ca²⁺]ᵢ in parathyroid cells. Like NPS 456, NPS 467 is a racemic mixture. Resolution of NPS 467 into its enantiomers provides an isomer of even greater potency than the racemic mixture (see Example 10). Further structure-activity studies on molecules related to NPS 447, NPS 467 and NPS 568 are expected to yield pure isomers with greater potency than these molecules in their racemic forms.

Results obtained with NPS 456 (Fig. 33) show that it elicits oscillatory increases in Cl⁻ current at concentrations of 100 µM. NPS 456 is the most potent molecule activate on Xenopus oocytes expressing the parathyroid cell Ca²⁺ receptor. The results obtained in this expression system with neomycin and NPS 456 demonstrate that these molecules act directly on the Ca²⁺ receptor.

### Example 6: Osteoclast Ca²⁺ Receptor Lead Molecules

The strategy used for elucidating the mechanism of action of extracellular Ca²⁺ on the osteoclast was similar to that proven effective in parathyroid cells. The first experiments examined the effects of La³⁺ on [Ca²⁺]ᵢ in single rat osteoclasts loaded with the fluorimetric indicator indo-1. As described above, trivalent cations like La³⁺ are impermeant and block Ca²⁺ influx. Low micromolar concentrations of La³⁺ partially depressed extracellular Ca²⁺-induced increases in [Ca²⁺]ᵢ (Fig. 29). The demonstration of a La³⁺-resistant increase in [Ca²⁺]ᵢ provides evidence for the mobilization of intracellular Ca²⁺. The results of these experiments parallel those obtained in parathyroid cells and suggest that similar mechanisms are used by extracellular Ca²⁺ to regulate [Ca²⁺]ᵢ in both cell types.

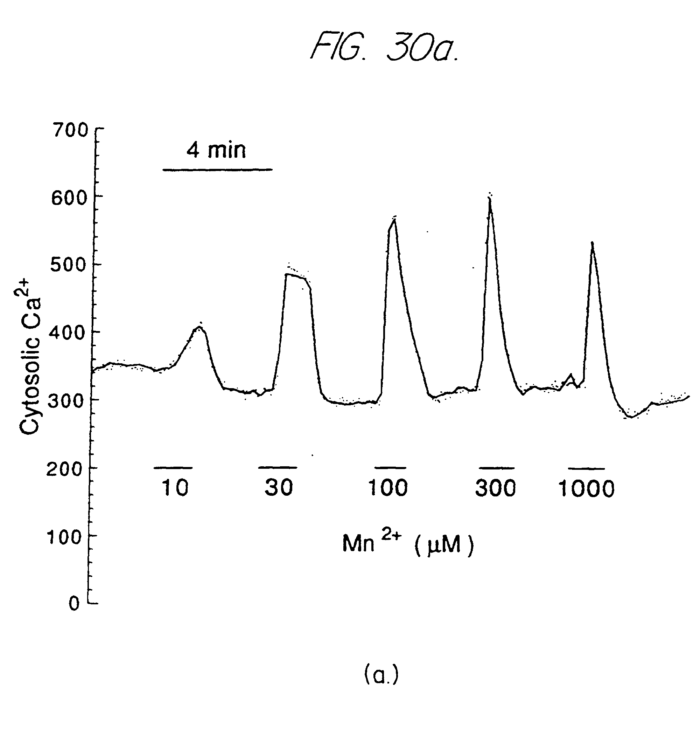

Another series of experiments showed that extracellular Mn²⁺ evoked transient increases in [Ca²⁺]ᵢ (Fig. 30(a)) that persisted in the absence of extracellular Ca²⁺ (Fig. 30B). These results are likewise indicative of the mobilization of intracellular Ca²⁺. Although Mn²⁺ can enter some cells, it is unlikely to do so in the osteoclast because Mn²⁺ quenches the fluorescence of indo-1. Thus, if Mn²⁺ penetrated intracellularly, a decrease, not an increase in the fluorescent signal would be observed.

The results obtained with a variety of di- and trivalent cations are all consistent with the presence of a Ca²⁺ receptor on the surface of the osteoclast that is coupled to the mobilization of intracellular Ca²⁺ and influx of extracellular Ca²⁺ through voltage-insensitive channels. Results show evidence for genetic material in human osteoclasts that encodes a Ca²⁺ receptor protein (see below). Transient increases in [Ca²⁺]ᵢ resulting from the mobilization of intracellular Ca²⁺, are sufficient to inhibit osteoclastic bone resorption in vitro. Thus, as with the parathyroid cell, activation of the Ca²⁺ receptor appears to be a viable means of inhibiting the activity of osteoclasts.

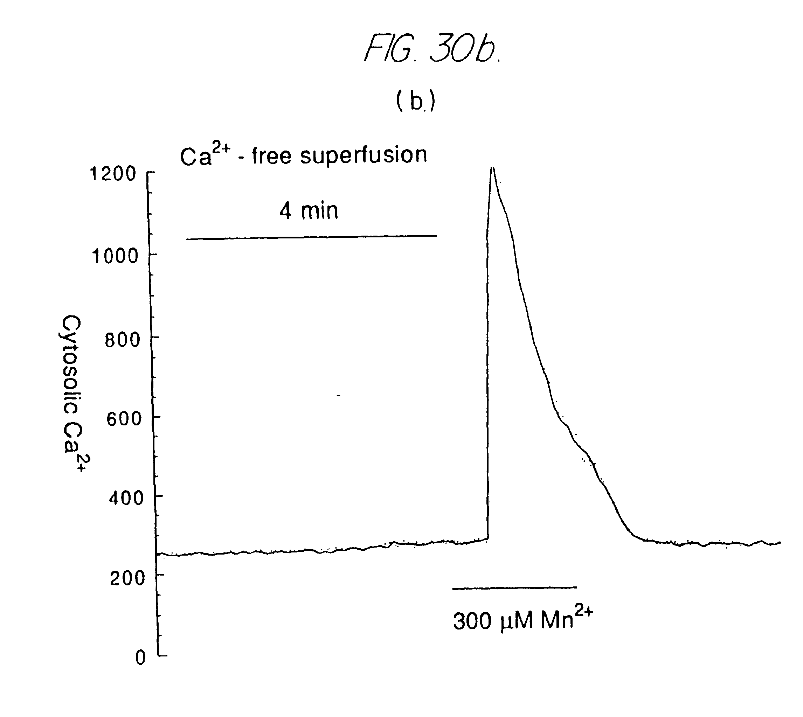

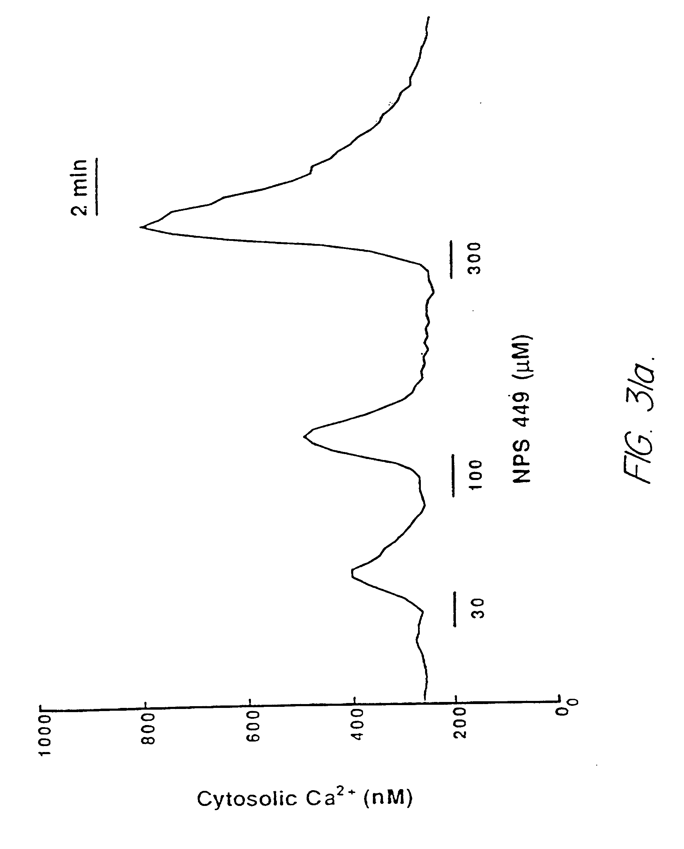

NPS 449 is presently the lead molecule for calcimimetic drugs on this receptor. It is a small molecule (MW < 425) and it mobilizes intracellular Ca²⁺ in rat osteoclasts with a EC₅₀ of about 150 µM (Figs. 31A and 31B). Although the potency of NPS 449 is relatively low, it has a simple structure with only one positive charge and is expected to have desirable pharmacodynamic and pharmacokinetic properties.

NPS 449 was examined for its ability to inhibit bone resorption in vitro. This was done by morphometric analysis of pit formation on thin slices of bovine cortical bone using scanning electron microscopy. Rat osteoclasts were incubated for 24 hours in slices of bone in the presence or absence of various concentrations of NPS 449. NPS 449 caused a concentration-dependent inhibition of bone resorption with an IC₅₀ of 10 µM. The anticipated results provide the first demonstration that molecules acting at this novel site can inhibit osteoclastic bone resorption. More potent analogs of NPS 449 will be generated using synthetic chemistry and will be tested and assayed using the methods described herein.

### Example 7: C-Cell Ca²⁺ Receptor Lead Molecules

Activation of the C-cell Ca²⁺ receptor stimulates the secretion of calcitonin which then acts on osteoclasts to inhibit bone resorption. Calcimimetic drugs selectively affecting C-cells are useful in the treatment of osteoporosis.

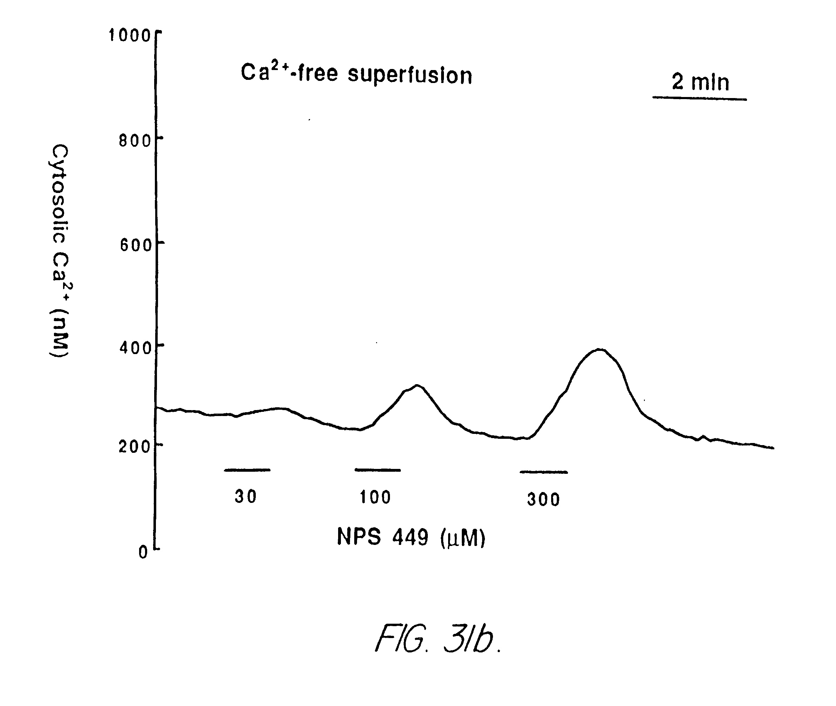

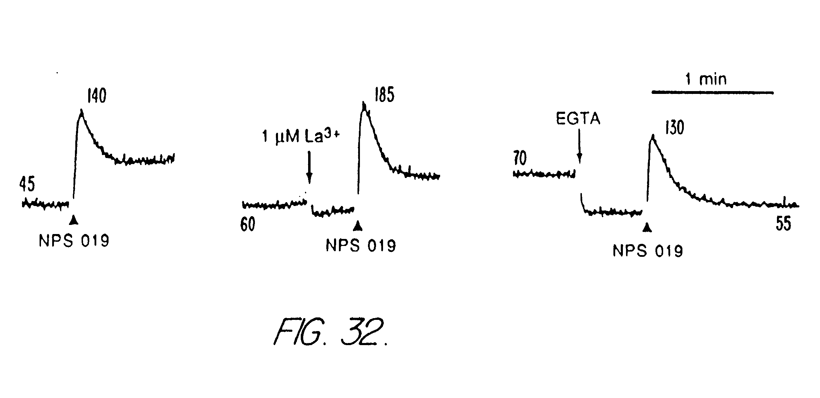

The mobilization of intracellular Ca²⁺ is used as a functional index of Ca²⁺ receptor activity. The screening effort in C-cells is facilitated by the availability of cultured cell lines expressing the C-cell phenotype (e.g., rat medullary thyroid carcinoma cells; rMTC 6-23 cells). Selected for initial study were three arylalkylamine molecules. Two are naturally occurring (agatoxin 489 and agatoxin 505) and the other (NPS 019) is a synthetic agatoxin analog. Agatoxin 505 was found to block extracellular Ca²⁺-induced increases in [Ca²⁺]ᵢ, with an IC₅₀ of 3 µM. The inhibitory effect resulted from a block of the L-type voltage-sensitive Ca²⁺ channel present in these cells. In contrast, agatoxin 489 was found to mobilize intracellular Ca²⁺ in rMTC cells with an EC₅₀ of 150 µM. This was the first organic molecule discovered that was found to activate the C-cell Ca²⁺ receptor. The synthetic analog, NPS 019, was even more potent and mobilized intracellular Ca²⁺ with an EC₅₀ of 5 µM (Fig. 32). It is significant that the only structural difference between NPS 019 and agatoxin 489 is the presence or absence of an hydroxyl group. The fact that such subtle differences in structure affect profoundly the potency of molecules indicates a structurally specific binding site on the Ca²⁺ receptor. This, in turn, encourages the view that very potent and selective activators of Ca²⁺ receptors can be developed.

NPS 019, which is a small molecule (MW < 500), is a lead molecule for development of calcimimetics of the C-cell Ca²⁺ receptor and can be tested for its ability to stimulate calcitonin secretion in vitro. Subsequent in vivo testing will then determine the ability of this molecule to stimulate calcitonin secretion and inhibit bone resorption. These in vivo studies will be performed in rats. The results obtained in these studies, which are anticipated to be positive, will provide the first evidence showing that a small organic molecule acting on a novel receptor can stimulate calcitonin secretion and depress bone resorption.

### Example 8: Calcilytic Activity of NPS 021 on Parathyroid Cells

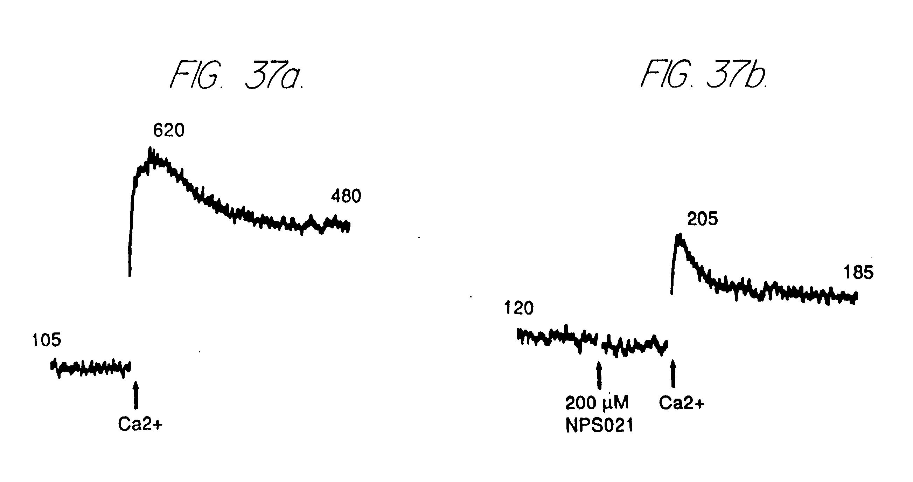

For a compound to be considered a calcilytic, it must block the effects of extracellular Ca²⁺ or a calcimimetic compound on an extracellular Ca²⁺-sensing cell. An example of a calcilytic compound is NPS 021, the structure of which is provided in Fig. 1. In bovine parathyroid cells loaded with fura-2, NPS 021 blocks increases in [Ca²⁺]ᵢ elicited by extracellular Ca²⁺. The IC₅₀ of NPS 021 for blocking this response is about 200 àM and, at concentrations around 500 àM, the increase in [Ca²⁺]ᵢ evoked by extracellular Ca²⁺ is abolished. Significantly, NPS 021 does not by itself cause any change in [Ca²⁺]ᵢ when tested at low [Ca²⁺] (0.5 mM; Fig. 37). Ga³⁺ is also calcilytic to Xenopus oocytes expressing the cloned Ca²⁺ receptor: Ga³⁺ by itself has no effect on the Cl⁻ currents activated by Gd³⁺, a calcimimetic, but pretreatment with Ga³⁺ blocks the action of Gd³⁺.

### Example 9: NPS 467 Lowers Serum Ionized Calcium

Compounds shown to activate the bovine parathyroid cell Ca²⁺ receptor in vitro were tested for hypocalcemic activity in vivo. Male Sprague-Dawley rates (200 g) were maintained on a low calcium diet for one week prior to receiving test substance or vehicle as control. Blood was collected from the tail vein three hours after the intra-peritoneal administration of NPS 467. Ionized Ca²⁺ in whole blood or serum was measured with a Ciba- Corning 634 Analyzer according to the instructions provided with the instrument. Serum total calcium, albumin and phosphate were measured by techniques well known in the art.

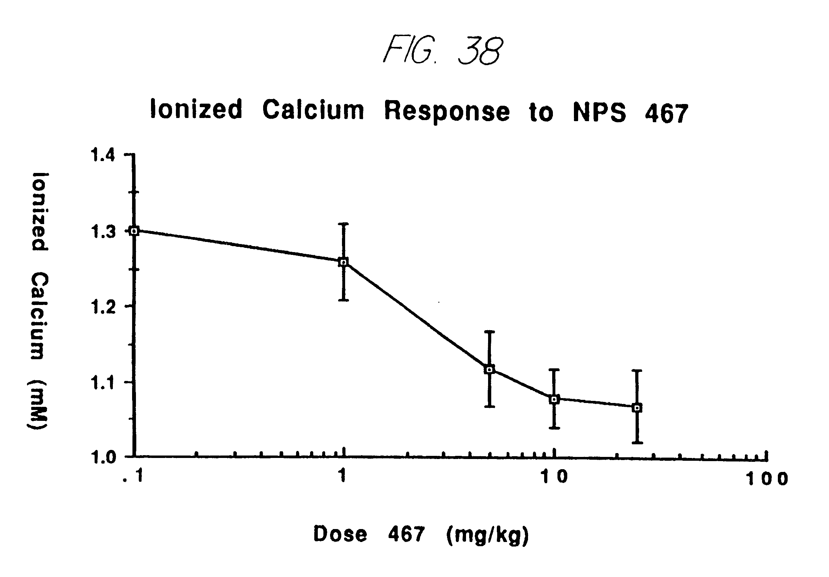

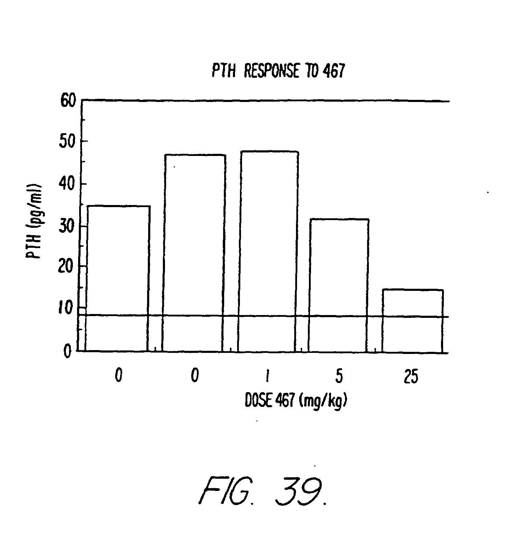

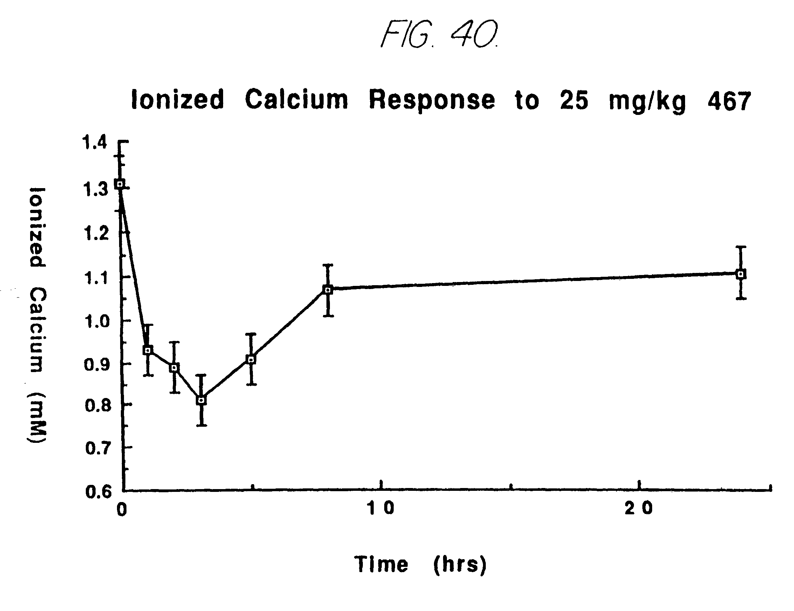

NPS 467 caused a dose-dependent reduction in serum or whole blood Ca²⁺ (Fig. 38). The fall in blood Ca²⁺ at this time was paralleled by a proportional fall in the levels of blood total calcium. There was no change in serum albumin or phosphate levels at any of the doses examined. In preliminary studies, NPS 467, at doses effective in lowering blood Ca²⁺, caused a dose-dependent reduction in circulating levels of PTH (Fig. 39). The hypocalcemic effect of NPS 467 was maximal within three hours and returned toward control levels after 24 hours (Fig. 40).

NPS R-467 (see Example 16) was also effective in lowering serum ionized Ca²⁺ in rats maintained on a normal, calcium-replete diet. A single dose of NPS R-467 (10 mg/kg i.p.) caused a rapid fall in serum levels of ionized Ca²⁺ which were maximal by 1 hr (22% decrease from the control level) and remained depressed at or near this level for up to 6 hours.

### Example 10: NPS 467 Lowers Serum Ionized Calcium in a Stereospecific Manner

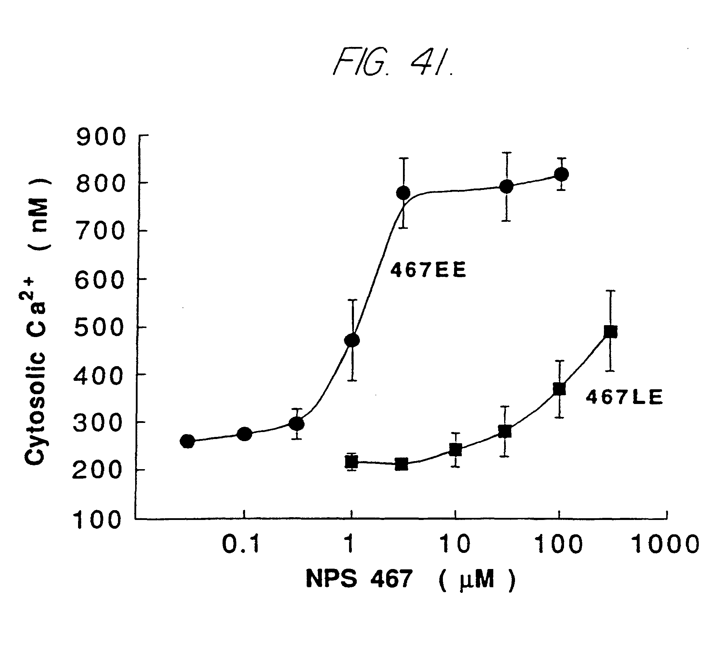

NPS 467 is a racemic mixture. Resolution of NPS 467 into its two enantiomers was achieved by separation on a chiral column. The R-isomer was about 100-fold more potent than the S-isomer in activating the bovine parathyroid cell Ca²⁺ receptor in vitro as assessed by the ability of the enantiomers to evoke increases in [Ca²⁺]ᵢ in parathyroid cells (Fig. 41). Likewise, similar resolution of the novel compound NPS 568 into its enantiomers showed that the R-isomer was 40-fold more potent than the S-isomer in causing the mobilization of intracellular Ca²⁺ in bovine parathyroid cells (see Table 6, supra).

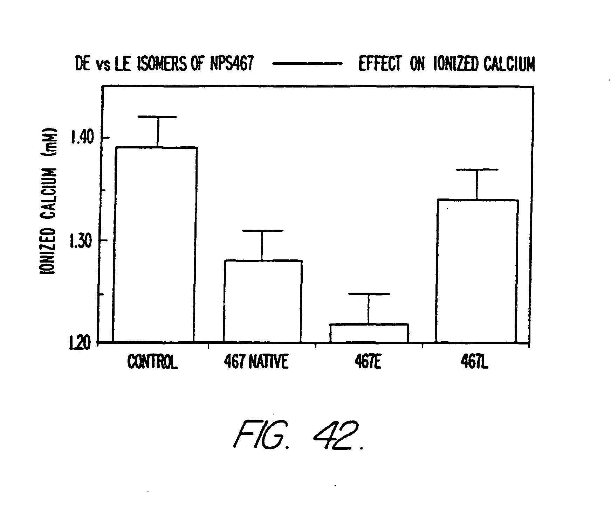

The isomers of NPS 467 were examined for effects on serum Ca²⁺ as in Example 15. Consistent with the in vitro results, the R-isomer of NPS 467 proved to be more potent than the S-isomer in lowering serum Ca²⁺ in vivo (Fig. 42; each compound was tested at a concentration of 5 mg/kg body weight).

### Example 11: NPS 467 Lowers Serum Ionized Calcium in an in vivo Model of Secondary Hyperparathyroidism

An accepted and widely used animal model of secondary hyperparathyroidism arising from chronic renal failure is the 5/6 nephrectomized rat. Animals receiving such surgery become initially hypocalcemic and, to maintain serum Ca²⁺ levels, there is a compensatory hyperplasia of the parathyroid glands and elevated levels of circulating PTH. Male Sprague-Dawley rats (250 g) received a 5/6 nephrectomy and were allowed to recover for 2 weeks. At this time they were normocalcemic (due to elevated levels of serum PTH). The administration of NPS R-467 (10 mg/kg i.p.) caused a rapid (within 2 hours) fall in serum ionized Ca²⁺ levels to 83% of controls in an animal model of secondary hyperparathyroidism. This suggests that compounds of this sort will effectively depress PTH secretion in patients with secondary hyperparathyroidism and hyperplastic parathyroid glands.

### Example 12: NPS 467 Fails to Lower Serum Ionized Calcium Levels in Parathyroidectomized Animals

To determine the primary target tissue upon which NPS 467 acts to cause a hypocalcemic response, the para thyroid glands in rats were surgically removed. Animals receiving a total parathyroidectomy become hypocalcemic and are largely dependent upon dietary calcium to maintain serum Ca²⁺ homeostasis. Parathyroidectomized animals had serum ionized Ca²⁺ levels of 0.92 mM which fell gradually to 0.76 mM after 6 hours of fasting. The administration of a single dose of NPS R-467 (10 mg/kg i.p.) did not cause any change in serum ionized Ca²⁺ levels over a period of 6 hours. These results demonstrate that intact parathyroid glands are required for the hypocalcemic effects of NPS R-467. The data additionally demonstrate that NPS R-467 can target the parathyroid glands in vivo. The results are consistent with the view that NPS R-467 acts on the parathyroid cell Ca²⁺ receptor in vivo to depress secretion of PTH and thereby cause serum levels of ionized Ca²⁺ to fall.

### Example 13: NPS 467 Increases Intracellular Calcium in Human Parathyroid Glands

Dissociated parathyroid cells were prepared from a parathyroid adenoma obtained by surgery from a patient with primary hyperparathyroidism. The cells were loaded with fura-2 and [Ca²⁺]ᵢ measured as described above. Both NPS R-467 and NPS R-568 caused concentration- dependent increase in [Ca²⁺]ᵢ. The EC₅₀'s for NPS R-467 and NPS R-568 were 20 and 3 µM, respectively. Both these compounds are thus able to increase [Ca²⁺]ᵢ in pathological human tissue and would thus be expected to decrease serum levels of PTH and Ca²⁺ in patients with primary hyperparathyroidism.

### Example 14: Mechanism of Action of NPS 467 at the Para thyroid Cell Calcium Receptor

Dissociated bovine parathyroid cells were used to further explore the mechanism of action of NPS 467 at the receptor level. In the presence of 0.5 mM extracellular Ca²⁺, NPS R-467 caused a rapid and transient increase in [Ca²⁺]ᵢ which persisted in the presence of 1 µM La³⁺ and was partially depressed by pretreatment with PMA (100 nM for 2 min.). All these results are consistent with an action of NPS R-467 on the Ca²⁺ receptor. However, the cytosolic Ca²⁺ response to NPS R-467 was abolished when parathyroid cells were suspended in Ca²⁺-free buffer. This suggests that NPS R- 467 cannot, by itself, cause the mobilization of intracellular Ca²⁺. It does, however, elicit responses in parathyroid cells and in oocytes when a small amount of extracellular Ca²⁺ is present. This suggests that partial occupancy of the Ca²⁺-binding site is required for NPS R-467 to elicit a response. To test this hypothesis, parathyroid cells were suspended in Ca²⁺-free buffer and exposed to a submaximal concentration of neomycin. Neomycin was used because it mimics, in nearly all respects, the effects of extracellular Ca²⁺ on parathyroid cells and on Xenopus oocytes expressing the parathyroid cell Ca²⁺ receptor. The addition of 10 àM neomycin did not by itself cause an increase in [Ca²⁺]ᵢ under these conditions. However the subsequent addition of NPS R-467 (30 µM) now elicited a transient increase in [Ca²⁺]ᵢ which, because there was no extracellular Ca²⁺ present, must have come from the mobilization of intracellular Ca²⁺. When cells bathed in Ca²⁺-free buffer were exposed to 30 àM NPS R-467 there was no increase in [Ca²⁺]ᵢ. This concentration of NPS R-467 is maximally effective in increasing [Ca²⁺]ᵢ when extracellular Ca²⁺ (0.5 mM) is present. However, the subsequent addition of 10 µM neomycin now evoked a transient increase in [Ca²⁺]ᵢ. Presumably, neomycin binds to the same site as extracellular Ca²⁺ and can functionally substitute for it. Using a submaximal concentration, which by itself causes no response, achieves partial occupancy of the Ca²⁺-binding site and allows activation of the Ca²⁺ receptor by NPS R-467.

Additional studies to further define the mechanism of action of NPS R-467 were performed. The cells were once again suspended in Ca²⁺-free buffer to insure that any observed increase in [Ca²⁺]ᵢ resulted from the mobilization of intracellular Ca²⁺. In these experiments, however, a maximally effective concentration (100 µM) of neomycin was used. In the absence of extracellular Ca²⁺, 100 µM neomycin evoked a rapid and transient increase in [Ca²⁺]ᵢ. The subsequent addition of 30 µM NPS R-467 did not cause an increase in [Ca²⁺]ᵢ. In the converse experiment, 30 µM NPS R-467 was added before 100 µM neomycin. As expected, NPS R-467 did not cause any increase in [Ca²⁺]ᵢ. It did not, however, affect the increase in [Ca²⁺]ᵢ evoked by the subsequent addition of 100 µM neomycin. These results, obtained with maximally effective concentrations of NPS R-467 and neomycin, suggest that these two compounds do not act at the same site. Rather, the results can be sufficiently explained by postulating two separate sites on the Ca²⁺ receptor, one to which extracellular Ca²⁺ and neomycin bind, and another to which NPS R-467 and structurally related compounds (such as NPS R-568) bind. Ligand binding to the former site can result in full activation of the Ca²⁺ receptor whereas ligand binding to the latter site can only occur and/or be functionally relevant when the extracellular Ca²⁺-binding site is occupied to some as yet undefined degree. It is possible that ligand binding to the extracellular Ca²⁺- binding site exposes a previously occluded binding site for NPS R-467. It appears that the NPS R-467-binding site is an allosteric site that augments receptor activation in response to ligand binding at the extracellular Ca²⁺-binding site.

The data demonstrate that the parathyroid cell Ca²⁺ receptor possesses at least two distinct sites for organic ligands. One site binds the physiological ligand, extracellular Ca²⁺, and certain organic polycations like neomycin. Binding to this site result in full activation of the Ca²⁺ receptor, an increase in [Ca²⁺]ᵢ, and the inhibition of PTH secretion. NPS R-467 and NPS R-568 define a previously unrecognized binding site on the Ca²⁺ receptor. Binding to this site can only occur and/or results in full activation of the Ca²⁺ receptor when the extracellular Ca²⁺-binding site is partially occupied. Ligands acting at either site are effective in suppressing serum Ca²⁺ levels in vivo.

### Allosteric Site on Parathyroid Cell Calcium Receptor

Calcimimetic compounds that activate the bovine parathyroid cell calcium receptor, such as NPS R-467 and NPS R-568, do not cause the mobilization of intracellular Ca²⁺ in the absence of extracellular Ca²⁺. Rather, they increase the sensitivity of the Ca²⁺ receptor to activation by extracellular Ca²⁺, thus causing a shift to the left in the concentration-response curve for extracellular Ca²⁺. Because of this, it is unlikely that they act at the same site on the receptor as does extracellular Ca²⁺. In contrast, organic and inorganic polycations do cause the mobilization of intracellular Ca²⁺ in the absence of extracellular Ca²⁺ and therefore probably act at the same site as does extracellular Ca²⁺. Compounds like NPS R-568, presumably act in an allosteric manner and their activity is dependent on some minimal level of extracellular Ca²⁺. This suggests that partial occupancy of the extracellular Ca²⁺-binding site on the receptor is required for compounds like NPS R-568 to be effective. This model is consistent with the observations described in Example 20.

Other details of the mechanism of action of NPS R- 568 on the parathyroid cell Ca²⁺ receptor, however, are more accurately investigated by binding studies in which the specific binding of radiolabeled (using ³H for example) NPS R-568 is assessed. There are several molecular mechanisms that could explain the activity of NPS R-568 on the parathyroid cell Ca²⁺ receptor. In one mechanism (model 1), NPS R-568 could bind to the Ca²⁺ receptor at a site that, when occupied, is not sufficient to activate the receptor functionally. Activation only occurs when some level of occupancy of the extracellular Ca²⁺-binding site(s) is achieved. In an alterative mechanism (model 2), the occupation of the extracellular Ca²⁺-binding site could unmask latent binding sites for compounds such as NPS R-568. Occupancy of this latent site by NPS R-568 then increases the affinity and/or efficacy of binding at the extracellular Ca²⁺ site. Either mechanism involves a form of allosteric activation of the Ca²⁺ receptor by compounds such as NPS R-568. These are not the only possible mechanisms that could explain the effect of compounds like NPS R-568 on the parathyroid cell Ca²⁺ receptor. Other mechanisms of action may be suggested by the results of the binding studies described below.

To further investigate the mechanism of action of compounds like NPS R-568 on the parathyroid cell Ca²⁺ receptor, binding studies using ³H-NPS R-568 can be performed. The specific binding of ³H-NPS R-568 to intact parathyroid cells or to membranes prepared from parathyroid cells is initially investigated by techniques well-known in the art. The kinetic parameters of binding will then be measured as a function of extracellular Ca²⁺ concentrations. Specifically, Scatchard analysis of the data will reveal the number of binding sites and the apparent affinity of the receptor site for ³H-NPS R-568. These parameters will then be investigated as a function of changes in the level of extracellular Ca²⁺ in the buffer used for the assay. If model 1 is correct, then a significant level of specific binding should occur in the absence of extracellular Ca²⁺. Large changes in the kinetic parameters of binding as a function of the level of extracellular Ca²⁺ would favor model 2. It is expected that various other inorganic and organic polycations described above in other examples will cause similar changes in the binding parameters of ³H-NPS R-568 as does extracellular Ca²⁺. This would support the view that these polycations act at the extracellular Ca²⁺-binding site, which is distinct from that to which compound like NPS R-568 bind.

### Example 15:

### BOPCAR1 Cloning Method 1

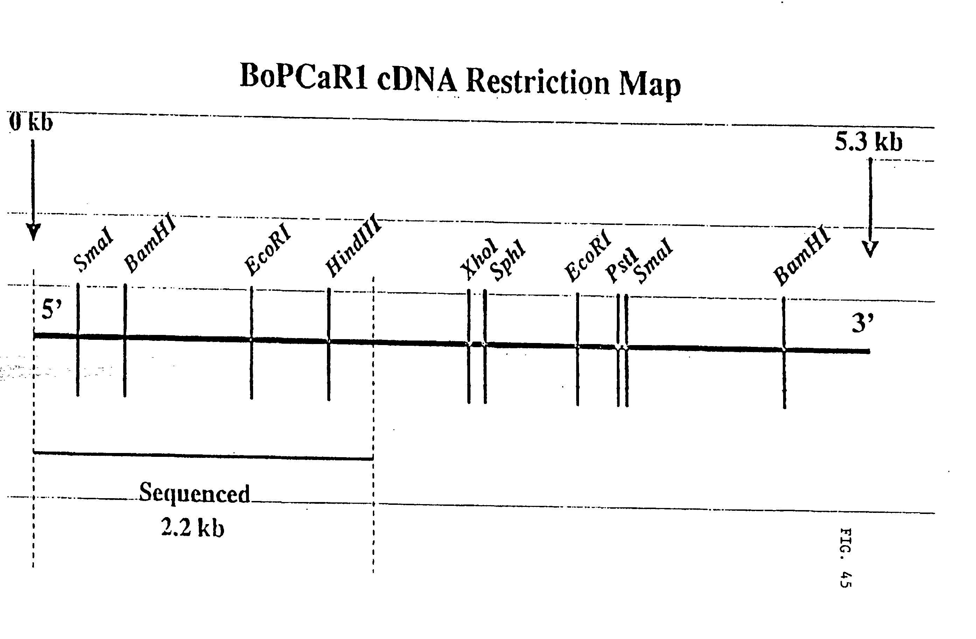

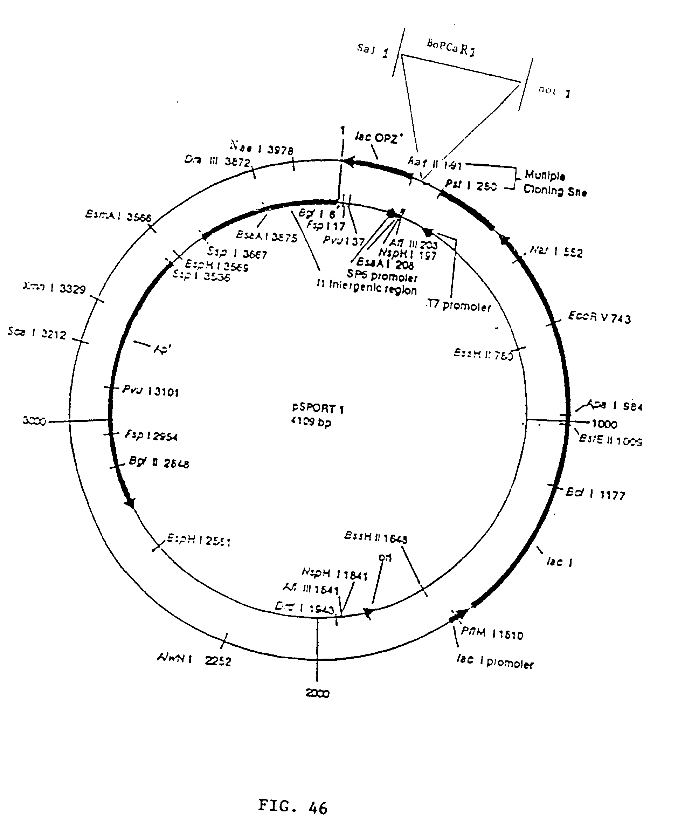

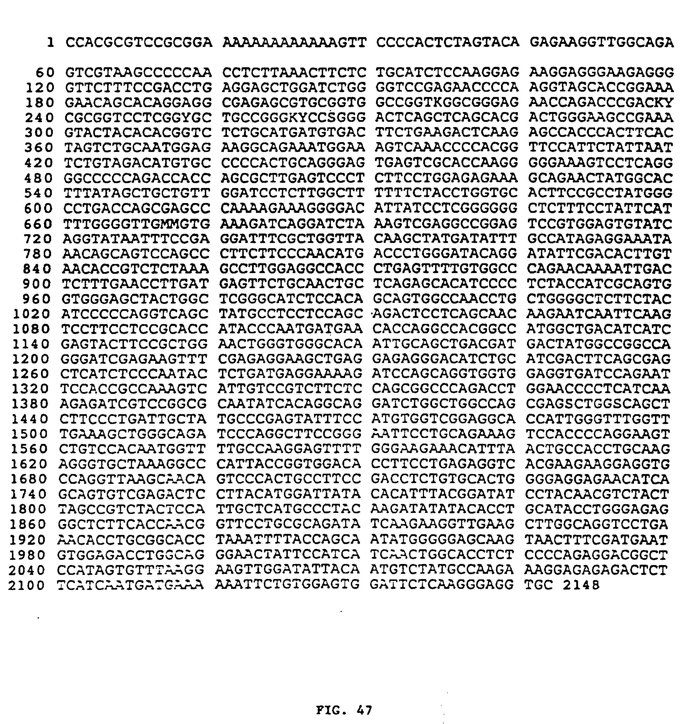

An expression cloning strategy using Xenopus laevis oocytes was used to identify the cDNA encoding the bovine parathyroid calcium receptor. These techniques are described only briefly here, and a more complete description preceded this one in the sections describing techniques which may be used to clone additional forms of the Ca²⁺-receptor from other cell types. Poly(A⁺)-enriched RNA was initially prepared from bovine parathyroid glands using extraction with guanidinium thiocyanate, centrifugation through CsCl and oligo(dT) cellulose chromatography. Injection of the resultant poly(A⁺)-enriched RNA into oocytes (25-50 ng/oocyte) conferred sensitivity to elevated extracellular concentrations of Ca²⁺ and the trivalent cation (1-100µM) Gd³⁺ as described herein, such that the two cations elicited calcium activated chloride currents. No such currents were elicited in control eggs injected with water. X. laevis frogs were selected on the basis of their harvested oocytes exhibiting: (i) a high level of maturity (i.e., Stage V, VI); (ii) a high activity of Cl⁻ currents activated by Ca²⁺ ionophores like A23187; (iii) a high level of functional expression of Gd³⁺-induced Cl⁻current when injected with 25 ng/oocyte of total poly(A)⁺RNA isolated from bovine parathyroid. The mRNA was then subjected to size fractionation, utilizing preparative, continuous flow agarose gel electrophoresis (Hediger, M.A., Anal. Biochem. 159:280-286 (1986)) to obtain fractions of poly(A⁺)-RNA further enriched in transcripts coding for the Ca²⁺-receptor. A peak of activity (e.g., oocytes injected with mRNA from these fractions showed enhanced expression of Gd³⁺-activated Cl⁻currents obtained with a size range of 4-5.5 kb. This RNA was used to prepare a size-selected, directional cDNA library in the plasmid pSPORT1 that was enriched in full length transcripts. Sense complementary RNA (cRNA) was then synthesized from the DNA inserts pooled from 350-500 independent clones from this library and injected into oocytes, Gd³⁺-activated Cl⁻currents were observed following injection of RNA from a single filter containing 350 colonies. Preparation and injection of cRNA from successively smaller pools of clones led to isolation of a single clone (BoPCaR 1) with a cDNA insert of 5.3 kb which expressed greatly enhanced Ca²⁺-receptor activity following injection of its cRNA into oocytes. A plasmid containing the BoPCaR 1 cRNA (See plasmid, Figure 45; restriction map, Figure 46; and partial nucleotide sequence, Figure 47) has been deposited in the ATCC under deposit number ATCC 75416.

The BoPCaR 1 cDNA is outside the size range of the size-selected RNA found to express neomycin elicited Cl⁻ channel activity in Xenopus oocytes. This is consistent with the possibility that different isoforms or multiple genes may conceal other numbers of the Ca²⁺ receptor gene family.

Several pharmacological and biochemical criteria were used to identify this clone as encoding a bona fide bovine parathyroid Ca²⁺-receptor. Oocytes expressing the cloned receptor, but not water-injected oocytes, responded to increasing concentrations of extracellular Ca²⁺ (1.5-5 mM) or Gd³⁺ (20-600µM) with large increases in Cl⁻ currents (up to at least 1.8 microamperes) that were several fold larger than those observed in poly(A⁺)-injected oocytes. These responses increased markedly over a period of one (1) to four (4) days after injection of the eggs with cRNA prepared from the BoPCaR 1 cDNA. Furthermore, the ranges of the concentrations of the two cations eliciting this response were very similar to those shown previously to act on bovine parathyroid cells in vitroN eomycin (20-100 µM), which is known to mimic closely the effects of Ca²⁺ on parathyroid cells, produced changes in Cl⁻ current in oocytes essentially identical to those produced by Ca²⁺ or Gd³⁺, and these occurred over the same range of concentrations over which this antibiotic moculates parathyroid function in vitro. Finally, in vitro translation of RNA prepared from the clone resulted in a single major protein on polyacrylamide gels with a molecular weight of about 100 kd, whose synthesis was enhanced by inclusion of dog pancreatic microsomes, concomitant with an increase in apparent molecular weight of 10-15%. The latter suggests that the cloned receptor interacts strongly with membranes, as might be expected of an integral membrane protein receptor, and is glycosylated in its native form. Studies with the lectin concanavalin A indicate that the Ca²⁺-receptor is likely a glycoprotein. Thus, the pharmacological properties of the cloned receptor, which is expressed at high levels in oocytes, as well as the biochemical studies carried out to date are completely consistent with its identity with the bovine parathyroid Ca²⁺ dynamics (as exemplified by changes in Ca²⁺-activated Cl⁻ currents in oocytes).

## Claims

1. A nucleic acid molecule encoding a calcium receptor polypeptide selected from the group consisting of:
(a) nucleic acid molecules comprising the nucleotide sequence as depicted in SEQ ID NO. 1;
(b) nucleic acid molecules comprising the nucleic acid sequence of the cDNA insert contained in ATCC 75416; and
(c) nucleic acid molecules which are able to hybridize to a nucleic acid molecule complementary to a molecule of (a) or (b) and which encode a functional calcium receptor.

2. The nucleic acid molecule of claim 1 which is genomic DNA.

3. The nucleic acid molecule of claim 1 which is cDNA.

4. A cell produced by introducing the nucleic acid molecule of any one of claims 1 to 3.

5. The cell of claim 4 expressing said calcium receptor polypeptide from an exogenously added nucleic acid molecule.

6. A method for producing a calcium receptor polypeptide comprising culturing the cell of claim 5 under conditions allowing expression of the polypeptide and recovering the polypeptide from the cell.

7. A calcium receptor polypeptide encoded by the nucleic acid molecule of any one of claims 1 to 3 or obtainable by the method of claim 6.

8. An antibody or a fragment thereof which specifically binds to the calcium receptor polypeptide of claim 7.

9. The antibody of claim 8 which is optionally coupled to a toxin.

10. A method for identifying a nucleic acid molecule encoding an inorganic ion receptor comprising the steps of contacting a library of nucleic acid molecules with the nucleic acid molecule of any one of claims 1 to 3 or the complement thereof, and detecting hybridization under low stringency hybridization conditions.

11. A method for identifying a nucleic acid molecule encoding an inorganic ion receptor comprising the steps of contacting a library of nucleic acid molecules with the nucleic acid molecule of any one of claims 1 to 3 and the complement thereof, and detecting hybridization under high stringency hybridization conditions.

## Patentansprüche

1. Nucleinsäuremolekül, das ein Calciumrezeptorpolypeptid codiert, ausgewählt aus der Gruppe bestehend aus:
(a) Nucleinsäuremolekülen, umfassend die Nucleotidsequenz wie in SEQ ID NO.1 dargestellt;
(b) Nucleinsäuremolekülen, die die Nucleinsäuresequenz des cDNA-Inserts umfassen, das in ATCC 75416 enthalten ist; und
(c) Nucleinsäuremolekülen, die an ein Nucleinsäuremolekül hybridisieren können, das zu einem Molekül nach (a) oder (b) komplementär ist, und einen funktionellen Calciumrezeptor codieren.

2. Nucleinsäuremolekül nach Anspruch 1, das eine genomische DNA ist.

3. Nucleinsäuremolekül nach Anspruch 1, das eine cDNA ist.

4. Zelle, hergestellt durch Einfügen des Nucleinsäuremoleküls nach einem der Ansprüche 1 bis 3.

5. Zelle nach Anspruch 4, die das Calciumrezeptorpolypeptid aus einem exogen hinzugefügten Nucleinsäuremolekül exprimiert.

6. Verfahren zur Herstellung eines Calciumrezeptorpolypeptids, umfassend das Züchten der Zelle nach Anspruch 5 unter Bedingungen, die die Expression des Polypeptids erlauben, und Gewinnen des Polypeptids aus der Zelle.

7. Calciumrezeptorpolypeptid, das von dem Nucleinsäuremolekül nach einem der Ansprüche 1 bis 3 codiert wird oder durch das Verfahren nach Anspruch 6 erhältlich ist.

8. Antikörper oder Fragment davon, der/das spezifisch an das Calciumrezeptorpolypeptid nach Anspruch 7 bindet.

9. Antikörper nach Anspruch 8, der gegebenenfalls an ein Toxin gekoppelt ist.

10. Verfahren zur Identifizierung eines Nucleinsäuremoleküls, das einen Rezeptor für anorganische Ionen codiert, umfassend die Schritte des Inkontaktbringens einer Bibliothek aus Nucleinsäuremolekülen mit dem Nucleinsäuremolekül nach einem der Ansprüche 1 bis 3 oder dem Komplement davon und des Nachweises der Hybridisierung unter niedrig-stringenten Hybridisierungsbedingungen.

11. Verfahren zur Identifizierung eines Nucleinsäuremoleküls, das einen Rezeptor für anorganische Ionen codiert, umfassend die Schritte des Inkontaktbringens einer Bibliothek aus Nucleinsäuremolekülen mit dem Nucleinsäuremolekül nach einem der Ansprüche 1 bis 3 und dem Komplement davon und des Nachweises der Hybridisierung unter hoch-stringenten Hybridisierungsbedingungen.

## Revendications

1. Molécule d'acide nucléique codant pour un polypeptide récepteur de calcium choisie parmi le groupe constitué de :
(a) molécules d'acide nucléique comprenant la séquence nucléotidique telle que décrite dans SEQ ID NO.1 ;
(b) molécules d'acide nucléique comprenant la séquence d'acide nucléique de l'insert d'ADNc contenu dans ATCC 75416 ; et
(c) molécules d'acide nucléique qui sont capables de s'hybrider à une molécule d'acide nucléique complémentaire d'une molécule selon (a) ou (b) et qui codent pour un récepteur de calcium fonctionnel.

2. Molécule d'acide nucléique selon la revendication 1 qui est un ADN génomique.

3. Molécule d'acide nucléique selon la revendication 1 qui est un ADNc.

4. Cellule produite par l'introduction d'une molécule d'acide nucléique selon l'une quelconque des revendications 1 à 3.

5. Cellule selon la revendication 4 exprimant ledit polypeptide récepteur de calcium à partir d'une molécule d'acide nucléique ajoutée de manière exogène.

6. Méthode pour produire un polypeptide récepteur de calcium comprenant la culture de la cellule selon la revendication 5 dans' des conditions permettant l'expression du polypeptide et la récupération du polypeptide à partir de la cellule.

7. Polypeptide récepteur de calcium codé par la molécule d'acide nucléique selon l'une quelconque des revendications 1 à 3 ou susceptible d'être obtenu par la méthode selon la revendication 6.

8. Anticorps ou fragment de celui-ci qui lie spécifiquement le polypeptide récepteur de calcium selon la revendication 7.

9. Anticorps selon la revendication 8 qui est éventuellement couplé à une toxine.

10. Méthode pour identifier une molécule d'acide nucléique codant pour un récepteur d'ion inorganique comprenant les étapes de mise en contact d'une banque de molécules d'acide nucléique avec la molécule d'acide nucléique selon l'une quelconque des revendications 1 à 3 ou le complément de celles-ci, et détection de l'hybridation dans des conditions d'hybridation de faible stringence.

11. Méthode pour identifier une molécule d'acide nucléique codant pour un récepteur d'ion inorganique comprenant les étapes de mise en contact d'une banque de molécules d'acide nucléique avec la molécule d'acide nucléique selon l'une quelconque des revendications 1 à 3 ou le complément de celles-ci, et détection de l'hybridation dans des conditions d'hybridation de stringence élevée.


### SEQUENCE LISTING

(1) GENERAL INFORMATION:
   (i) APPLICANT: Nemeth, Edward F.
   (ii) TITLE OF INVENTION: CALCIUM RECEPTOR-ACTIVE MOLECULES
   (iii) NUMBER OF SEQUENCES: 1
   (iv) CORRESPONDENCE ADDRESS:
      (A) ADDRESSEE: Wolf, Greenfield & Sacks, P.C.
      (B) STREET: 600 Atlantic Avenue
      (C) CITY: Boston
      (D) STATE: Massachusetts
      (E) COUNTRY: United States of America
      (F) ZIP: 02210-2204
   (v) COMPUTER READABLE FORM:
      (A) MEDIUM TYPE: Diskette, 5.25 inch
      (B) COMPUTER: IBM-compatible
      (C) OPERATING SYSTEM: MS:DOS Version 3.3
      (D) SOFTWARE: WordPerfect 5.1
   (vi) CURRENT APPLICATION DATA:
      (A) APPLICATION NUMBER: Not Available
      (B) FILING DATE: Filed Herewith
      (C) CLASSIFICATION: Not Available
   (viii) ATTORNEY/AGENT INFORMATION:
      (A) NAME: Gates, Edward R.
      (B) REGISTRATION NUMBER: 31,616
      (C) REFERENCE/DOCKET NUMBER: B0801/7012
   (ix) TELECOMMUNICATION INFORMATION:
      (A) TELEPHONE: (617) 720-3500
      (B) TELEFAX: (617) 720-2441
      (C) TELEX: EZEKIEL
(2) INFORMATION FOR SEQ ID NO: 1:
   (i) SEQUENCE CHARACTERISTICS:
      (A) LENGTH: 2148 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: No
   (iv) ANTI-SENSE: No
   (xi) SEQUENCE DESCRIPTION: SEQ ID NO: 1:
   CCACGCGTCC GCGGAAAAAA AAAAAAAGTT CCCCACTCTA 40
   GTACAGAGAA GGTTGGCAGA GTCGTAAGCC CCCAACCTCT 80
   TAAACTTCTC TGCATCTCCA AGGAGAAGGA GGGAAGAGGG 120
   GTTCTTTCCG ACCTGAGGAG CTGGATCTGG GGTCCGAGAA 160
   CCCCAAGGTA GCACCGGAAA GAACAGCACA GGAGGCGAGA 200
   GCGTGCGGTG GCCGGTKGGC GGGAGAACCA GACCCGACKY 240
   CGCGGTCCTC GGYGCTGCCG GGKYCCSGGG ACTCAGCTCA 280
   GCACGACTGG GAAGCCGAAA GTACTACACA CGGTCTCTGC 320
   ATGATGTGAC TTCTGAAGAC TCAAGAGCCA CCCACTTCAC 360
   TAGTCTGCAA TGGAGAAGGC AGAAATGGAA AGTCAAACCC 400
   CACGGTTCCA TTCTATTAAT TCTGTAGACA TGTGCCCCCA 440
   CTGCAGGGAG TGAGTCGCAC CAAGGGGGAA AGTCCTCAGG 480
   GGCCCCCAGA CCACCAGCGC TTGAGTCCCT CTTCCTGGAG 520
   AGAAAGCAGA ACTATGGCAC TTTATAGCTG CTGTTGGATC 560
   CTCTTGGCTT TTTTCTACCT GGTGCACTTC CGCCTATGGG 600
   CCTGACCAGC GAGCCCAAAA GAAAGGGGAC ATTATCCTCG 640
   GGGGGCTCTT TCCTATTCAT TTTGGGGTTG MMGTGAAAGA 680
   TCAGGATCTA AAGTCGAGGC CGGAGTCCGT GGAGTGTATC 720
   AGGTATAATT TCCGAGGATT TCGCTGGTTA CAAGCTATGA 760
   TATTTGCCAT AGAGGAAATA AACAGCAGTC CAGCCCTTCT 800
   TCCCAACATG ACCCTGGGAT ACAGGATATT CGACACTTGT 840
   AACACCGTCT CTAAAGCCTT GGAGGCCACC CTGAGTTTTG 880
   TGGCCCAGAA CAAAATTGAC TCTTTGAACC TTGATGAGTT 920
   CTGCAACTGC TCAGAGCACA TCCCCTCTAC CATCGCAGTG 960
   GTGGGAGCTA CTGGCTCGGG CATCTCCACA GCAGTGGCCA 1000
   ACCTGCTGGG GCTCTTCTAC ATCCCCCAGG TCAGCTATGC 1040
   CTCCTCCAGC AGACTCCTCA GCAACAAGAA TCAATTCAAG 1080
   TCCTTCCTCC GCACCATACC CAATGATGAA CACCAGGCCA 1120
   CGGCCATGGC TGACATCATC GAGTACTTCC GCTGGAACTG 1160
   GGTGGGCACA ATTGCAGCTG ACGATGACTA TGGCCGGCCA 1200
   GGGATCGAGA AGTTTCGAGA GGAAGCTGAG GAGAGGGACA 1240
   TCTGCATCGA CTTCAGCGAG CTCATCTCCC AATACTCTGA 1280
   TGAGGAAAAG ATCCAGCAGG TGGTGGAGGT GATCCAGAAT 1320
   TCCACCGCCA AAGTCATTGT CCGTCTTCTC CAGCGGCCCA 1360
   GACCTGGAAC CCCTCATCAA AGAGATCGTC CGGCGCAATA 1400
   TCACAGGCAG GATCTGGCTG GCCAGCGAGS CTGGSCAGCT   1440
   CTTCCCTGAT TGCTATGCCC GAGTATTTCC ATGTGGTCGG   1480
   AGGCACCATT GGGTTTGGTT TGAAAGCTGG GCAGATCCCA   1520
   GGCTTCCGGG AATTCCTGCA GAAAGTCCAC CCCAGGAAGT   1560
   CTGTCCACAA TGGTTTTGCC AAGGAGTTTT GGGAAGAAAC   1600
   ATTTAACTGC CACCTGCAAG AGGGTGCTAA AGGCCCATTA   1640
   CCGGTGGACA CCTTCCTGAG AGGTCACGAA GAAGGAGGTG   1680
   CCAGGTTAAG CAACAGTCCC ACTGCCTTCC GACCTCTGTG   1720
   CACTGGGGAG GAGAACATCA GCAGTGTCGA GACTCCTTAC   1760
   ATGGATTATA CACATTTACG GATATCCTAC AACGTCTACT   1800
   TAGCCGTCTA CTCCATTGCT CATGCCCTAC AAGATATATA   1840
   CACCTGCATA CCTGGGAGAG GGCTCTTCAC CAACGGTTCC   1880
   TGCGCAGATA TCAAGAAGGT TGAAGCTTGG CAGGTCCTGA   1920
   AACACCTGCG GCACCTAAAT TTTACCAGCA ATATGGGGGA   1960
   GCAAGTAACT TTCGATGAAT GTGGAGACCT GGCAGGGAAC   2000
   TATTCCATCA TCAACTGGCA CCTCTCCCCA GAGGACGGCT   2040
   CCATAGTGTT TAAGGAAGTT GGATATTACA ATGTCTATGC   2080
   CAAGAAAGGA GAGAGACTCT TCATCAATGA TGAAAAAATT   2120
   CTGTGGAGTG GATTCTCAAG GGAGGTGC                2148